# EUROPEAN PATENT SPECIFICATION

(11) **EP 4 025 592 B1**
(45) Date of publication and mention of the grant of the patent: **29.10.2025**
(21) Application number: 20860676.4
(22) Date of filing: 03.09.2020
(51) Int. Cl.: C07K 14/575, A61K 31/00, A61K 38/22

(54) **CONJUGATED HEPCIDIN MIMETICS FOR USE IN TREATING OF POLYCYTHEMIA VERA**
KONJUGIERTE HEPCIDIN-MIMETIKA ZUR VERWENDUNG BEI DER BEHANDLUNG VON POLYCYTHEMIA VERA
MIMETIQUES D'HEPCIDINE CONJUGÉS POUR LE TRAITEMENT DE LA POLYCYTHÉMIE VERA

(30) Priority: 03.09.2019 US 201962895201 P; 28.02.2020 US 202062983515 P; 06.05.2020 US 202063020945 P; 31.07.2020 US 202063059747 P
(43) Date of publication of application: 13.07.2022
(73) Proprietor: Protagonist Therapeutics, Inc., Newark, CA 94560-1160 (US)
(72) Inventor: LIU, David Y., Newark California 94560-1160 (US); BOURNE, Gregory Thomas, Brisbane Queensland 4070 (AU); TARANATH, Roopa, Cupertino California 95014 (US); GUPTA, Suneel Kumar, Sunnyvale California 94087 (US); MODI, Nishit Bachulal, Sunnyvale California 94087 (US)
(74) Representative: Carpmaels & Ransford LLP
(86) International application number: PCT/US2020/049244
(87) International publication number: WO 2021/046246

(56) References cited:
- WO-A1-2015/157283
- WO-A1-2017/117411
- WO-A1-2018/128828
- WO-A1-2019/157268
- US-A1- 2017 313 754
- CASU CARLA ET AL: "Use of Minihepcidins As a "Medical Phlebotomy" in the Treatment of Polycythemia Vera", BLOOD, AMERICAN SOCIETY OF HEMATOLOGY, US, vol. 124, no. 21, 14 November 2014 (2014-11-14), pages 3231, XP086740262, ISSN: 0006-4971, [retrieved on 20210625], DOI: 10.1182/BLOOD.V124.21.3231.3231
- WANG CAIYI ET AL: "Reciprocal regulation between hepcidin and erythropoiesis and its therapeutic application in erythroid disorders", EXPERIMENTAL HEMATOLOGY, ELSEVIER INC, US, vol. 52, 10 May 2017 (2017-05-10), pages 24 - 31, XP085135639, ISSN: 0301-472X, DOI: 10.1016/J.EXPHEM.2017.05.002
- CLINICALTRIALS.GOV: "Hepcidin Mimetic in Patients With Polycythemia Vera (REVIVE)", CLINICALTRIALS.GOV, 14 August 2019 (2019-08-14), pages 1 - 8, XP093058438, Retrieved from the Internet <URL:https://classic.clinicaltrials.gov/ct2/show/NCT04057040> [retrieved on 20230627]
- CASU CARLA ET AL: "Hepcidin agonists as therapeutic tools", BLOOD, 19 April 2018 (2018-04-19), XP093058227
- CLINICALTRIALS.GOV: "Hepcidin Mimetic in Patients With Polycythemia Vera (REVIVE)", CLINICALTRIALS.GOV, 14 August 2019 (2019-08-14), pages 1 - 8, XP93058438, Retrieved from the Internet <URL:https://classic.clinicaltrials.gov/ct2/show/NCT04057040> [retrieved on 20230627]

## Description

### FIELD OF THE INVENTION

The present invention relates, inter alia, to certain hepcidin peptide analogues, including both peptide monomers and peptide dimers, and conjugates and derivatives thereof, and to the use of the peptide analogues in the treatment and/or prevention of Polycythemia vera (PV).

### BACKGROUND

Hepcidin (also referred to as LEAP-1), a peptide hormone produced by the liver, is a regulator of iron homeostasis in humans and other mammals. Hepcidin acts by binding to its receptor, the iron export channel ferroportin, causing its internalization and degradation. Human hepcidin is a 25-amino acid peptide (Hep25). See Krause et al. (2000) FEBS Lett 480:147-150, and Park et al. (2001) J Biol Chem 276:7806-7810. The structure of the bioactive 25-amino acid form of hepcidin is a simple hairpin with 8 cysteines that form 4 disulfide bonds as described by Jordan et al. J Biol Chem 284:24155-67. The N terminal region is required for iron-regulatory function, and deletion of 5 N-terminal amino acid residues results in a loss of iron-regulatory function. See Nemeth et al. (2006) Blood 107:328-33.

Abnormal hepcidin activity is associated with iron overload diseases, including hereditary hemochromatosis (HH) and iron-loading anemias. Hereditary hemochromatosis is a genetic iron overload disease that is mainly caused by hepcidin deficiency or in some cases by hepcidin resistance. This allows excessive absorption of iron from the diet and development of iron overload. Clinical manifestations of HH may include liver disease (e.g., hepatic cirrhosis and hepatocellular carcinoma), diabetes, and heart failure. Currently, the only treatment for HH is regular phlebotomy, which is very burdensome for the patients. Iron-loading anemias are hereditary anemias with ineffective erythropoiesis such as β-thalassemia, which are accompanied by severe iron overload. Complications from iron overload are the main cause of morbidity and mortality for these patients. Hepcidin deficiency is the main cause of iron overload in non-transfused patients and contributes to iron overload in transfused patients. The current treatment for iron overload in these patients is iron chelation which is very burdensome, sometimes ineffective, and accompanied by frequent side effects.Hepcidin has a number of limitations which restrict its use as a drug, including a difficult synthesis process due in part to aggregation and precipitation of the protein during folding, which in turn leads to high cost of goods.

US patents, US 9,822,157 and US 10,030,061, describe novel hepcidin analogs and their use to treat iron overload diseases, which include hereditary hemochromatosis and iron-loading anemias.

PCT application publication, WO15200916, describes additional novel hepcidin analogs and their use to treat iron overload diseases.

PCT application publication, WO17117411, describes additional novel hepcidin analogs with improved *in vivo* half-lives and their use to treat iron overload diseases.

PCT application publication, WO18048944, describes additional novel hepcidin analogs and their use to treat prevention of iron overload in a subject and/or reducing serum iron levels in a subject.

PCT application publication, WO18128828, describes additional novel hepcidin analogs and their use to treat hepcidin-associated disorders, including prophylaxis and treatment of iron overload diseases such as hemochromatosis, iron-loading anemias such as thalassemia, and diseases being associated with ineffective or augmented erythropoiesis.

PCT application publication, WO17068089, describes additional novel hepcidin analogs (ferroportin inhibitors) and their use to treat thalassemia, and hemochromatosis.

US Patent, US9315545, describe additional novel hepcidin analogs and their use to treat diseases of iron metabolism, beta thalassemia, hemochromatosis, iron-loading anemias, alcoholic liver disease, or chronic hepatitis C.

Polycythemia vera (PV) is a chronic, progressive trilineage clonal disorder signified by increased myeloid, erythroid, and megakaryocytic cell proliferation/accumulation and is characterized by the World Health Organization (WHO) as a myeloproliferative neoplasm (Arber et al., 2016,127(20):2391-405). Diagnosis is defined by two criteria; the first being increased red blood cell mass, bone marrow biopsy showing trilineage hypercellularity and presence of JAK2V617F or JAK2 exon 12 mutations and the second criteria incorporates polycythemia, bone marrow biopsy confirmation and subnormal serum erythropoietin levels (Arber et al., 2016,127(20):2391-405).

An estimated 148,000 people in the United States are living with PV with a median age at diagnosis of 61 years (Stein et al., J Clin Oncol. 2015 Nov 20;33(33):3953-60). Polycythemia symptoms, related to blood hyperviscosity include fatigue, bone pain, headaches, lightheadedness, visual disturbances, atypical chest pain, pruritus, erythromelalgia, and paresthesia (Tefferi et al., Blood Cancer J. 2018, 8(1):3). Clinical features include splenomegaly, thrombotic and bleeding complications, and risk of leukemic transformation.

As PV is a disease characterized by increased erythropoiesis, it has been shown in animal models that high doses of hepcidin mimetics can ameliorate this disease by diminishing erythropoiesis (Casu et al., Blood. 2016;128(2):265-276). In PV mice that express the orthologous JAK2 mutation causing human PV, administration of minihepcidin significantly reduces splenomegaly and normalizes hematocrit. These studies indicate that drug-like minihepcidins have a potential as future therapeutics for untransfused β-thalassemia and PV (Casu et al., Blood. 2016;128(2):265-276).

There is clearly a need for new therapeutic agents and methods for treating and preventing PV, including PV in high-risk patients or those who cannot tolerate phlebotomy. The present invention addresses such needs of treating PV.

### BRIEF SUMMARY OF THE INVENTION

The invention is defined by the appended claims.

The present invention provides a hepcidin analogue or a pharmaceutically acceptable salt or solvate thereof, for use in a method of treating polycythemia vera in a human subject in need thereof, wherein the hepcidin analogue is: 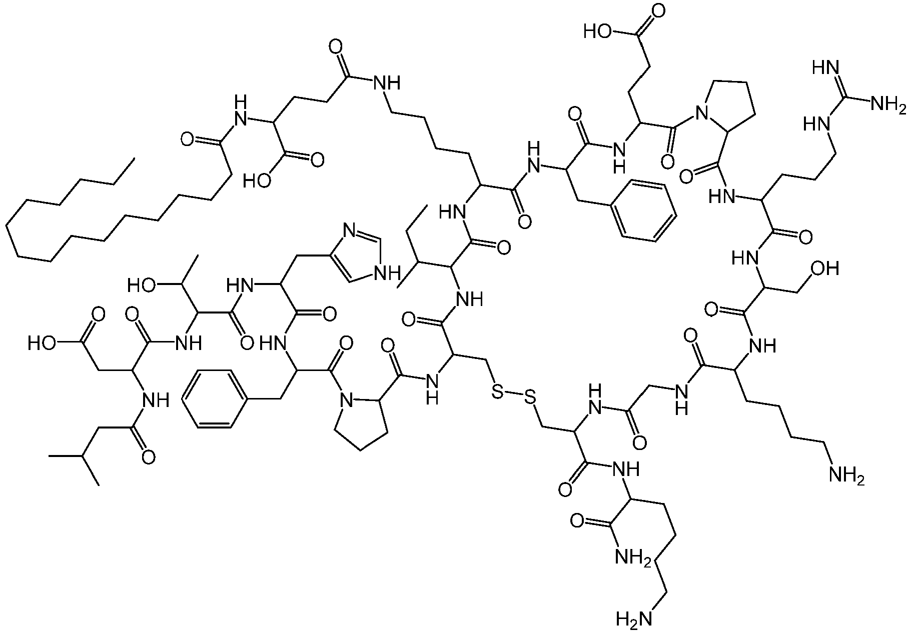 Isovaleric acid-DTHFPCI(K(isoGlu-Palm))FEPRSKGCK-NH2 (SEQ ID NO:45);
wherein the amino acids are L-amino acids; and wherein the hepcidin analogue or pharmaceutically acceptable salt thereof is administered via subcutaneous injection; the subject is administered from about 20 mg to about 100 mg of the hepcidin analogue or pharmaceutically acceptable salt thereof; and the hepcidin analogue or pharmaceutically acceptable salt thereof is provided to the subject about twice a week, about once a week, or about once every two weeks.

**In** some embodiments, the hepcidin analogue is administered to the subject in a pharmaceutical composition comprising one or more pharmaceutically acceptable carriers, excipients, or diluents.

**In** some embodiments, the hepcidin analogue or pharmaceutically acceptable salt thereof is provided to the subject at a dosage of about 20 mg to about 50 mg, about 20 mg to about 40 mg, about 80 mg, about 70 mg, about 60 mg, about 50 mg, about 40 mg, about 30 mg, about 25 mg, or about 20 mg. In some embodiments, the hepcidin analogue or pharmaceutically acceptable salt thereof is provided to the subject at a dosage of about 20 mg. In other embodiments, the hepcidin analogue or pharmaceutically acceptable salt thereof is provided to the subject at a dosage of about 40 mg.

In some embodiments, the hepcidin analogue or pharmaceutically acceptable salt thereof is provided to the subject about once a week. In other embodiments, the hepcidin analogue or pharmaceutically acceptable salt thereof is provided to the subject about twice a week.

In some embodiments, the hepcidin analogue is provided to the subject at a dosage of about 20 mg, about 25 mg, about 30 mg, or about 40 mg about once a week. In other embodiments, the hepcidin analogue is provided to the subject at a dosage of about 20 mg, about 25 mg, about 30 mg, or about 40 mg about twice a week.

In some embodiments, the polycythemia vera is phlebotomy-requiring polycythemia vera.

In some embodiments, the subject is diagnosed with polycythemia vera and has received at least three phlebotomies to goal hematocrit <45% in the 24 weeks prior to administration of the hepcidin analogue or pharmaceutically acceptable salt or solvate thereof to the subject.

In some embodiments, the method further comprises determining the subject's hematocrit at one or more time points following administration of the hepcidin analogue or pharmaceutically acceptable salt or solvate thereof, and maintaining or adjusting the amount of the hepcidin analogue or pharmaceutically acceptable salt or solvate thereof administered to the subject, wherein the amount is increased if the subject's determined hematocrit is greater than 45%, wherein the amount is decreased if the subject's determined hematocrit is less than either 37.5% or 40%, and maintaining the amount if the subject's determined hematocrit is between 37.5% and 45% or between 40% and 44%. In some embodiments, the hepcidin analogue or pharmaceutically acceptable salt or solvate thereof is initially administered about 20 mg of the hepcidin analogue or pharmaceutically acceptable salt or solvate thereof, and wherein the adjust amount is about 10 mg to about 100 mg of the hepcidin analogue or pharmaceutically acceptable salt or solvate thereof to achieve or maintain hematocrit <45%.

In some embodiments, the subject is treated by cytoreductive therapy, optionally wherein the cytoreductive therapy comprises hydroxyurea.

some embodiments, the method comprises multiple administrations of an effective amount of the hepcidin analogue or pharmaceutically acceptable salt thereof over a period of time, wherein the method further comprises determining the subject's hematocrit following one or more of the multiple administrations of the hepcidin analogue or pharmaceutically acceptable salt or solvate thereof, and maintaining or adjusting the amount of the hepcidin analogue or pharmaceutically acceptable salt or solvate thereof next administered to the subject, wherein the next administered amount is increased if the subject's determined hematocrit is above an acceptable range based on the subject's sex and pregnancy status, wherein the next administered amount is decreased if the subject's determined hematocrit is below the acceptable range, and wherein the next administered amount is the same as the previously administered amount if the subject's determined hematocrit is within the acceptable range.

Also described are methods of treating polycythemia vera in a subject in need thereof, comprising administering to the subject an effective amount of a pharmaceutical composition comprising a hepcidin analogue and a pharmaceutically acceptable carrier, diluent or excipient, wherein the hepcidin analogue comprises a peptide comprising Formula (I):

R1-X-Y-R2 (I) (SEQ ID NO: 1)

or a pharmaceutically acceptable salt or solvate thereof,
wherein
R1 is hydrogen, a C1-C6 alkyl, a C6-C12 aryl, a C1-C20 alkanoyl or pGlu;
R2 is NH₂ or OH;
X is a peptide sequence having the formula II

X1-X2-X3-X4-X5-X6-X7-X8-X9-X10 (II) (SEQ ID NO:2)

wherein
X1 is Asp, Ala, Ida, pGlu, bhAsp, Leu, D-Asp or absent;
X2 is Thr, Ala, or D-Thr;
X3 is His, Lys, or D-His;
X4 is Phe, Ala, Dpa or D-Phe;
X5 is Pro, Gly, Arg, Lys, Ala, D-Pro or bhPro;
X6 is Ile, Cys, Arg, Lys, D-Ile or D-Cys;
X7 is Cys, Ile, Leu, Val, Phe, D-Ile or D-Cys;
X8 is Ile, Arg, Phe, Gln, Lys, Glu, Val, Leu or D-Ile;
X9 is Phe or bhPhe; and
X10 is Lys, Phe or absent;
wherein if Y is absent, X7 is Ile; and
Y is a peptide sequence having the formula III

Y1-Y2-Y3-Y4-Y5-Y6-Y7-Y8-Y9-Y10-Y11-Y12-Y13-Y14-Y15 (III) (SEQ ID NO:3)

wherein
Y1 is Gly, Cys, Ala, Phe, Pro, Glu, Lys, D-Pro, Val, Ser or absent;
Y2 is Pro, Ala, Cys, Gly or absent;
Y3 is Arg, Lys, Pro, Gly, His, Ala, Trp or absent;
Y4 is Ser, Arg, Gly, Trp, Ala, His, Tyr or absent;
Y5 is Lys, Met, Arg, Ala or absent;
Y6 is Gly, Ser, Lys, Ile, Ala, Pro, Val or absent;
Y7 is Trp, Lys, Gly, Ala, Ile, Val or absent;
Y8 is Val, Thr, Gly, Cys, Met, Tyr, Ala, Glu, Lys, Asp, Arg or absent;
Y9 is Cys, Tyr or absent;
Y10 is Met, Lys, Arg, Tyr or absent;
Y11 is Arg, Met, Cys, Lys or absent;
Y12 is Arg, Lys, Ala or absent;
Y13 is Arg, Cys, Lys, Val or absent;
Y14 is Arg, Lys, Pro, Cys, Thr or absent; and
Y15 is Thr, Arg or absent;
wherein said peptide of formula I is optionally PEGylated on R1, X, or Y, and wherein a side chain of an amino acid of the peptide is optionally conjugated to a lipophilic substituent or polymeric moiety. Alternatively, formula II is shown as above, but X3 is D-Lys.

R1 may be hydrogen, isovaleric acid, isobutyric acid or acetyl.

The half-life extension moiety may be selected from C12 (Lauric acid), C14 (Mysteric acid), C16(Palmitic acid), C18 (Stearic acid), C20, C12 diacid, C14 diacid, C16 diacid, C18 diacid, C20 diacid, biotin, and isovaleric acid. The half-life extension moiety may beattached to a linker moiety that is attached to the peptideThe half-life extension moiety may increase the molecular weight of the hepcidin analogue by about 50 D to about 2 KD. The half-life extension moiety may increase serum half-life, enhances solubility, and/or improve bioavailability of the hepcidin analogue.

The peptide analogue may comprise an isovaleric acid moiety conjugated to an N-terminal Asp residue.

The peptide analogue may comprise an amidated C-terminal residue.

The peptide analogue may comprise the sequence: Asp-Thr-His-Phe-Pro-Cys-Ile-Lys-Phe-Glu-Pro-Arg-Ser-Lys-Gly-Cys-Lys (SEQ ID NO: 19), or comprise a sequence having at least 80%, at least 90%, or at least 94% identity to this sequence.

The peptide analogue may comprise the sequence: Asp-Thr-His-Phe-Pro-Cys-Ile-Lys-Phe-Pro-Arg-Ser-Lys-Gly-Cys-Lys (SEQ ID NO: 19), or comprise a sequence having at least 80%, at least 90%, or at least 94% identity to this sequence.

Also described herein ia a polynucleotide that encodes a peptide of a hepcidin analogue or dimer (or monomer subunit of a dimer).

Also described herein is a vector comprising the polynucleotide.

Also described herein is a pharmaceutical composition, comprising a hepcidin analogue, dimer, polynucleotide, or vector, and a pharmaceutically acceptable carrier, excipient or vehicle.

Also described herein is a method of binding a ferroportin or inducing ferroportin internalization and degradation, comprising contacting the ferroportin with at least one hepcidin analogue, dimer or composition as described above.

In certain embodiments, the method further comprises determining the subject's hematocrit level at one or more time points following administration of the hepcidin analogue, and maintaining or adjusting the amount of the hepcidin analogue or pharcaceutically acceptable salt thereof administered to the subject, wherein the amount is increased if the subject's determined hematocrit is greater than 44% or 45%, wherein the amount is descreased if the subject's determined hematocrit is less than either 37.5% or 40%, and maintaining the amount if the subject's determined hematocrit is between 37.5% and 45%, between 37.5% and 44%, between 40% and 45%, or between 40% and 44%.

Also described herein is a device comprising pharmaceutical composition of the present invention, for delivery of a hepcidin analogue of the invention to a subject.

Also described herein is a kit comprising a pharmaceutical composition of the invention, packaged with a reagent, a device, or an instructional material, or a combination thereof.

In particular embodiments, the Polycythemia vera is phlebotomy-requiring Polycythemia vera, or phlebotomy-requiring Polycythemia vera in a low risk patient.

In particular embodiments, the subject is a low risk Polycythemia vera patient, a high risk Polycythemia vera patient, a symptomatic phlebotomy-requiring Polycythemia vera patient, a high risk patient with phlebotomy-requiring Polycythemia vera or a low risk patient with phlebotomy-requiring Polycythemia vera.

In certain embodiments, the pharmaceutical composition is administered about weekly over a period of time.

In certain embodiments, the amount of the hepcidin analog or peptide administered is increased over a period of time.

In certain embodiments, the method results in a decrease in the subject's hematocrit level to ≤45%, a decrease in the subject's hematocrit of at least 3%, and/or an increase in the subject's serum ferritin. In some embodiments, the subject remains phlebotomy free during a course of treatment, e.g., treatment about once a week for a period of time.

### BRIEF DESCRIPTION OF THE FIGURES

Figures 1A and 1B describe the data obtained from the Cobas Iron2 Analysis experiment for Compound A and Compound B. Figure 1A is a graph showing serum iron levels and serum concentration of Compound A at the indicated times following treatment with Compound A. Figure 1B is a graph showing serum iron levels and serum concentration of Compound B at the indictaed times following treatment with Compound B.

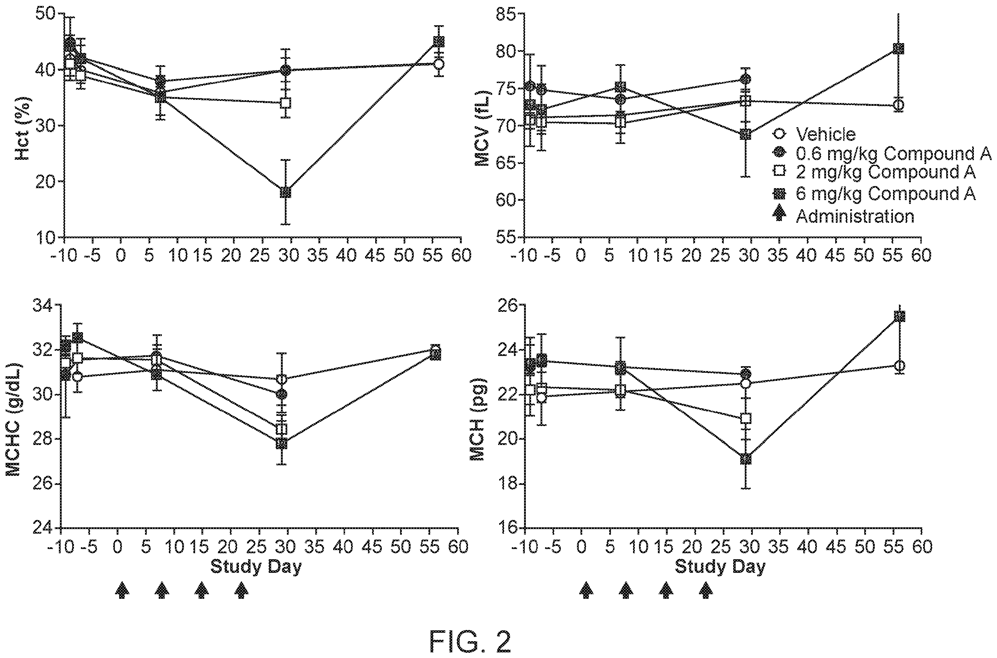

Figure 2 shows temporal profile of hematocrit and RBC indices in male Cynomolgus monkeys following subcutaneous administration of vehicle (∘) or Compound A at doses of 1 (•), 3 (□), and 10 (■) mg/kg/dose once weekly for 4 administrations with a 28-day recovery period. Compound A induced changes in secondary hematologic indices (mean corpuscular hemoglobin concentration, MCHC, and mean corpuscular hemoglobin, MCH) indicative of iron-restricted erythropoiesis. Each point represents mean ± SD of up to 6 animals in the Main treatment (all groups) and up to 2 animals in the Recovery phase (vehicle and 10 mg/kg Compound A). Arrows indicate when Compound A was administered.

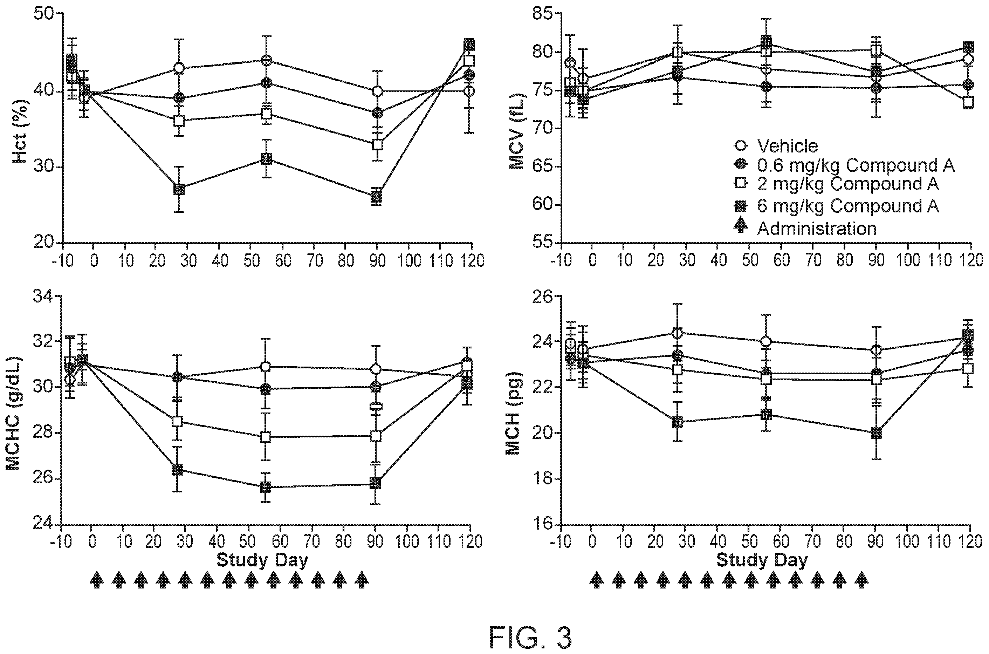

Figure 3 demonstrates that Compound A induces significant changes in hematocrit (Hct) and secondary hematologic indices. Alterations in Hct, mean corpuscular volume (MCV), mean corpuscular hemoglobin (MCH), mean corpuscular hemoglobin concentration (MCHC) were assessed after subcutaneous administration of vehicle (∘) or Compound A at doses of 0.6 (•), 2 (□), and 6 (■) mg/kg once weekly for 13 consecutive weeks followed by a 35-day recovery period. Arrows represent administration days starting on Day 1. Each point represents mean ± SD of up to 6 animals/gender in the Main phase and up to 2 animals/gender in the Recovery phase.
Figure 4 shows that bilirubin profiles are consistent with iron-restricted erythropoiesis in iron-replete cynomolgus monkeys. Total bilirubin levels in male (left) and female Cynomolgus monkeys (right) after subcutaneous administration of vehicle (∘) or Compound A at doses of 0.6 (•), 2 (□), and 6 (■) mg/kg once weekly for 13 consecutive weeks followed by a 35-day recovery period. Arrows represent administration days starting on Day 1. Each point represents mean ± SD of up to 6 animals/gender in the Main phase and up to 2 animals/gender in the Recovery phase.
Figure 5 shows platelet profiles in male (left) and female Cynomolgus monkeys (right) after subcutaneous administration of vehicle (∘) or Compound A at doses of 0.6 (•), 2 (□), and 6 (■) mg/kg once weekly for 13 consecutive weeks followed by a 35-day recovery period. Arrows represent administration days starting on Day 1. Each point represents mean ± SD of up to 6 animals/gender in the Main phase and up to 2 animals/gender in the Recovery phase.
Figure 6 provides a diagram of the phase II clinical trial design.
Figure 7 is a timeline showing the timing of therapeutic phlebotomy and treatment with the indicated dosages of Compound A for thirteen human PV patients.
Figure 8 is a graph showing ferritin (ng/mL) levels in PV patients treated with Compound A at the indicated time points. Subject numbers correlate to those shown in Table 9.
Figure 9 is a graph showing TSAT (%) in PV patients treated with Compound A at the indicated time points. Subject numbers correlate to those shown in Table 9.
Figure 10 is a graph showing MCV (fL) in PV patients treated with Compound A at the indicated time points. Subject numbers correlate to those shown in Table 9.
Figure 11 is a graph showing MCH (pg) in PV patients treated with Compound A at the indicated time points. Subject numbers correlate to those shown in Table 9.
Figure 12 is a graph showing hematocrit (%) in PV patients treated with Compound A at the indicated time points. Subject numbers correlate to those shown in Table 9.
Figure 13 is a graph showing erythrocytes (10⁶/uL) in PV patients treated with Compound A at the indicated time points. Subject numbers correlate to those shown in Table 9.
Figure 14 is a graph showing platelets (10³/uL) in PV patients prior to and after treatment with the indicated dosages of Compound A at the indicated time points. For Figures 14-16, the indicated subject number correlates to the subject number provided in Table 9 as follows: 1501-01 = 3; 1501-02 = 5; 1502-1 = 1; 1502-02 = 2; 1502-04 = 4; 1505-01 = 6; 1505-02 = 7; and 1509-01 = 8.
Figure 15 is a graph showing reticulocytes (%) in PV patients prior to and after treatment with the indictaed dosages of Compound A at the indicated time points.
Figure 16 is a graph showing leukocytes (/uL) in PV patients prior to and after treatment with the indictaed dosages of Compound A at the indicated time points.
Figures 17A and 17B show plasma concentrations of Compound A in PV patients at various times following administration of the indicated amounts of Compound A. Figure 17A shows data for individual time points, and Figure 17B shos averages over specified time intervals.

### DETAILED DESCRIPTION OF THE INVENTION

The present disclosure is generally directed to the use of hepcidin analogue peptides to treat and prevent Polycythemia vera (PV).

Hepcidin (also referred to as LEAP-1), a peptide hormone produced by the liver, is a regulator of iron homeostasis in humans and other mammals. Hepcidin acts by binding to its receptor, the iron export channel ferroportin, causing its internalization and degradation. Human hepcidin is a 25-amino acid peptide (Hep25). See Krause et al. (2000) FEBS Lett 480:147-150, and Park et al. (2001) J Biol Chem 276:7806-7810. The structure of the bioactive 25-amino acid form of hepcidin is a simple hairpin with 8 cysteines that form 4 disulfide bonds as described by Jordan et al. J Biol Chem 284:24155-67. The N terminal region is required for iron-regulatory function, and deletion of 5 N-terminal amino acid residues results in a loss of iron-regulatory function. See Nemeth et al. (2006) Blood 107:328-33.

Abnormal hepcidin activity is associated with iron overload diseases, including hereditary hemochromatosis (HH) and iron-loading anemias. Hereditary hemochromatosis is a genetic iron overload disease that is mainly caused by hepcidin deficiency or in some cases by hepcidin resistance. This allows excessive absorption of iron from the diet and development of iron overload. Clinical manifestations of HH may include liver disease (e.g., hepatic cirrhosis and hepatocellular carcinoma), diabetes, and heart failure. Iron-loading anemias are hereditary anemias with ineffective erythropoiesis such as β-thalassemia, which are accompanied by severe iron overload.

Hepcidin has a number of limitations that restrict its use as a drug, including a difficult synthesis process due in part to aggregation and precipitation of the protein during folding, which in turn leads to high cost of goods. The present disclosure provides hepcidin analogue peptides having hepcidin activity and also possessing other beneficial physical properties such as improved solubility, stability, and/or potency, so that hepcidin-like biologics might be produced affordably, and used to treat and prevent Polycythemia vera.

The present invention also relates generally to hepcidin analogue peptides and methods of making and using the same. In certain embodiments, the hepcidin analogues exhibit one or more hepcidin activity. In certain embodiments, the present invention relates to hepcidin peptide analogues comprising one or more peptide subunit that forms a cyclized structures through an intramolecular bond, e.g., an intramolecular disulfide bond. In particular embodiments, the cyclized structure has increased potency and selectivity as compared to non-cyclized hepcidin peptides and analogies thereof. In particular embodiments, hepcidin analogue peptides of the present invention exhibit increased half-lives, e.g., when delivered orally, as compared to hepcidin or previous hepcidin analogues.

### Definitions and Nomenclature

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well-known and commonly used in the art.

As used herein, the following terms have the meanings ascribed to them unless specified otherwise.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to." "Including" and "including but not limited to" are used interchangeably.

The terms "patient," "subject," and "individual" may be used interchangeably and refer to either a human or a non-human animal. These terms include mammals such as humans, primates, livestock animals (e.g., bovines, porcines), companion animals (e.g., canines, felines) and rodents (e.g., mice and rats). The term "mammal" refers to any mammalian species such as a human, mouse, rat, dog, cat, hamster, guinea pig, rabbit, livestock, and the like.

The term "peptide," as used herein, refers broadly to a sequence of two or more amino acids joined together by peptide bonds. It should be understood that this term does not connote a specific length of a polymer of amino acids, nor is it intended to imply or distinguish whether the polypeptide is produced using recombinant techniques, chemical or enzymatic synthesis, or is naturally occurring.

The term "peptide analogue," as used herein, refers broadly to peptide monomers and peptide dimers comprising one or more structural features and/or functional activities in common with hepcidin, or a functional region thereof. In certain embodiments, a peptide analogue includes peptides sharing substantial amino acid sequence identity with hepcidin, e.g., peptides that comprise one or more amino acid insertions, deletions, or substitutions as compared to a wild-type hepcidin, e.g., human hepcidin, amino acid sequence. In certain embodiments, a peptide analogue comprises one or more additional modification, such as, e.g., conjugation to another compound. Encompassed by the term "peptide analogue" is any peptide monomer or peptide dimer of the present invention. In certain instances, a "peptide analog" may also or alternatively be referred to herein as a "hepcidin analogue," "hepcidin peptide analogue," or a "hepcidin analogue peptide."

The recitations "sequence identity", "percent identity", "percent homology", or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

Calculations of sequence similarity or sequence identity between sequences (the terms are used interchangeably herein) can be performed as follows. To determine the percent identity of two amino acid sequences, or of two nucleic acid sequences, the sequences can be aligned for optimal comparison purposes (e.g., gaps can be introduced in one or both of a first and a second amino acid or nucleic acid sequence for optimal alignment and non-homologous sequences can be disregarded for comparison purposes). In certain embodiments, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two amino acid sequences is determined using the Needleman and Wunsch, (1970, J. Mol. Biol. 48: 444-453) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using an NWSgapdna.CMP matrix and a gap weight of 40, 50, 60, 70, or 80 and a length weight of 1, 2, 3, 4, 5, or 6. Another exemplary set of parameters includes a Blossum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The peptide sequences described herein can be used as a "query sequence" to perform a search against public databases to, for example, identify other family members or related sequences. Such searches can be performed using the NBLAST and XBLAST programs (version 2.0) of Altschul, et al., (1990, J. Mol. Biol, 215: 403-10). BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al. (Nucleic Acids Res. 25:3389-3402, 1997). When utilizing BLAST and Gapped BLAST programs, the default parameters of the respective programs (e.g., XBLAST and NBLAST) can be used.

The term "conservative substitution" as used herein denotes that one or more amino acids are replaced by another, biologically similar residue. Examples include substitution of amino acid residues with similar characteristics, e.g., small amino acids, acidic amino acids, polar amino acids, basic amino acids, hydrophobic amino acids and aromatic amino acids. See, for example, the table below. In some embodiments of the invention, one or more Met residues are substituted with norleucine (Nle) which is a bioisostere for Met, but which, as opposed to Met, is not readily oxidized. In some embodiments, one or more Trp residues are substituted with Phe, or one or more Phe residues are substituted with Trp, while in some embodiments, one or more Pro residues are substituted with Npc, or one or more Npc residues are substituted with Pro. Another example of a conservative substitution with a residue normally not found in endogenous, mammalian peptides and proteins is the conservative substitution of Arg or Lys with, for example, ornithine, canavanine, aminoethylcysteine or another basic amino acid. In some embodiments, another conservative substitution is the substitution of one or more Pro residues with bhPro or Leu or D-Npc (isonipecotic acid). For further information concerning phenotypically silent substitutions in peptides and proteins, see, for example, Bowie et. al. Science 247, 1306-1310, 1990. In the scheme below, conservative substitutions of amino acids are grouped by physicochemical properties. I: neutral, hydrophilic, II: acids and amides, III: basic, IV: hydrophobic, V: aromatic, bulky amino acids.

| **I** | **II** | **III** | **IV** | **V** |
|---|---|---|---|---|
| A | N | H | M | F |
| S | D | R | L | Y |
| T | E | K | I | W |
| P | Q | | V | |
| G | | | C | |

In the scheme below, conservative substitutions of amino acids are grouped by physicochemical properties. VI: neutral or hydrophobic, VII: acidic, VIII: basic, IX: polar, X: aromatic.

| **VI** | **VII** | **VIII** | **IX** | **X** |
|---|---|---|---|---|
| A | E | H | M | F |
| L | D | R | S | Y |
| I | | K | T | W |
| P | | | C | |
| G | | | N | |
| V | | | Q | |

The term "amino acid" or "any amino acid" as used here refers to any and all amino acids, including naturally occurring amino acids (e.g., a-amino acids), unnatural amino acids, modified amino acids, and non-natural amino acids. It includes both D- and L-amino acids. Natural amino acids include those found in nature, such as, e.g., the 23 amino acids that combine into peptide chains to form the building-blocks of a vast array of proteins. These are primarily L stereoisomers, although a few D-amino acids occur in bacterial envelopes and some antibiotics. The 20 "standard," natural amino acids are listed in the above tables. The "non-standard," natural amino acids are pyrrolysine (found in methanogenic organisms and other eukaryotes), selenocysteine (present in many noneukaryotes as well as most eukaryotes), and N-formylmethionine (encoded by the start codon AUG in bacteria, mitochondria and chloroplasts). "Unnatural" or "non-natural" amino acids are non-proteinogenic amino acids (i.e., those not naturally encoded or found in the genetic code) that either occur naturally or are chemically synthesized. Over 140 natural amino acids are known and thousands of more combinations are possible. Examples of "unnatural" amino acids include β-amino acids (β³ and β²), homo-amino acids, proline and pyruvic acid derivatives, 3-substituted alanine derivatives, glycine derivatives, ring-substituted phenylalanine and tyrosine derivatives, linear core amino acids, diamino acids, D-amino acids, and N-methyl amino acids. Unnatural or non-natural amino acids also include modified amino acids. "Modified" amino acids include amino acids (e.g., natural amino acids) that have been chemically modified to include a group, groups, or chemical moiety not naturally present on the amino acid.

As is clear to the skilled artisan, the peptide sequences disclosed herein are shown proceeding from left to right, with the left end of the sequence being the N-terminus of the peptide and the right end of the sequence being the C-terminus of the peptide. Among sequences disclosed herein are sequences incorporating a "Hy-" moiety at the amino terminus (N-terminus) of the sequence, and either an "-OH" moiety or an "-NH₂" moiety at the carboxy terminus (C-terminus) of the sequence. In such cases, and unless otherwise indicated, a "Hy-" moiety at the N-terminus of the sequence in question indicates a hydrogen atom, corresponding to the presence of a free primary or secondary amino group at the N-terminus, while an "-OH" or an "-NH₂" moiety at the C-terminus of the sequence indicates a hydroxy group or an amino group, corresponding to the presence of an amido (CONH₂) group at the C-terminus, respectively. In each sequence of the invention, a C-terminal "-OH" moiety may be substituted for a C-terminal "-NH₂" moiety, and vice-versa. It is further understood that the moiety at the amino terminus or carboxy terminus may be a bond, e.g., a covalent bond, particularly in situations where the amino terminus or carboxy terminus is bound to a linker or to another chemical moiety, e.g., a PEG moiety.

The term "NH₂," as used herein, refers to the free amino group present at the amino terminus of a polypeptide. The term "OH," as used herein, refers to the free carboxy group present at the carboxy terminus of a peptide. Further, the term "Ac," as used herein, refers to Acetyl protection through acylation of the C- or N-terminus of a polypeptide.

The term "carboxy," as used herein, refers to -CO₂H.

For the most part, the names of naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974)" Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**Table 1. Abbreviations of Non-Natural Amino Acids and Chemical Moieties**

| Abbreviation | Definition |
|---|---|
| DIG | Diglycolic acid |
| Dapa | Diaminopropionic acid |
| Daba | Diaminobutyric acid |
| Pen | Penicillamine |
| Sarc or Sar | Sarcosine |
| Cit | Citroline |
| Cav | Cavanine |
| NMe-Arg | N-Methyl-Arginine |
| NMe-Trp | N-Methyl-Tryptophan |
| NMe-Phe | N-Methyl-Phenylalanine |
| Ac- | Acetyl |
| 2-Nal | 2-Napthylalanine |
| 1-Nal | 1-Napthylalanine |
| Bip | Biphenylalanine |
| βAla | beta-Alanine |
| Aib | 2-aminoisobutyric acid |
| Azt | *azetidine*-2-carboxylic acid |
| Tic | (3S)-1,2,3,4-Tetrahydroisoquinoline-hydroxy-3-carboxylic acid |
| Phe(OMe) | Tyrosine (4-Methyl) |
| N-MeLys | N-Methyl-Lysine |
| N-MeLys(Ac) | N-e-Acetyl-D-lysine |
| Dpa | β,β diphenylalanine |
| NH₂ | Free Amine |
| CONH₂ | Amide |
| COOH | Acid |
| Phe(4-F) | 4-Fluoro-Phenylalanine |
| PEG3 | NH₂CH₂CH₂(OCH₂CH₂)₃CH₂CH₂CO₂H |
| m-PEG3 | CH₃OCH₂CH₂(OCH₂CH₂)₂CH₂CH₂CO₂H |
| m-PEG4 | CH₃OCH₂CH₂(OCH₂CH₂)₃CH₂CH₂CO₂H |
| m-PEG8 | CH₃OCH₂CH₂(OCH₂CH₂)₇CH₂CH₂CO₂H |
| PEG11 | O-(2-aminoethyl)-O'-(2-carboxyethyl)-undecaethyleneglycol NH₂CH₂CH₂(OCH₂CH₂)₁₁CH₂CH₂CO₂H |
| PEG13 | Bifunctional PEG linker with 13 PolyEthylene Glycol units |
| PEG25 | Bifunctional PEG linker with 25 PolyEthylene Glycol units |
| PEG1K | Bifunctional PEG linker with PolyEthylene Glycol Mol wt of 1000Da |
| PEG2K | Bifunctional PEG linker with PolyEthylene Glycol Mol wt of 2000Da |
| PEG3.4K | Bifunctional PEG linker with PolyEthylene Glycol Mol wt of 3400Da |
| PEG5K | Bifunctional PEG linker with PolyEthylene Glycol Mol wt of 5000Da |
| IDA or Ida | Iminodiacetic acid |
| IDA-Palm | (Palmityl)-Iminodiacetic acid |
| hPhe | homoPhenylalanine |
| Ahx | Aminohexanoic acid |
| DIG-OH | Glycolic monoacid |
| Triazine | Amino propyl Triazine di-acid |
| Boc-Triazine | Boc-Triazine di-acid |
| Trifluorobutyric acid | 4,4,4-Trifluorobutyric acid |
| 2-Methylltrifluorobutyric acid | 2-methyl-4,4,4-Butyric acid |
| Trifluorpentanoic acid | 5,5,5-Trifluoropentanoic acid |
| 1,4- Phenylenediacetic acid | para-Phenylenediacetic acid |
| 1,3 - Phenylenediacetic acid | meta-Phenylenediacetic acid |
| DTT | Dithiothreotol |
| Nle | Norleucine |
| βhTrp or bhTrp | β-homoTryptophane |
| βhPhe or bhPhe | β-homophenylalanine |
| Phe(4-CF₃) | 4-TrifluoromethylPhenylalanine |
| βGlu or bGlu | β-Glutamic acid |
| βhGlu or bhGlu | β-homoglutamic acid |
| 2-2-Indane | 2-Aminoindane-2-carboxylic acid |
| 1-1-Indane | 1-Aminoindane-1-carboxylic acid |
| hCha | homocyclohexylalanine |
| Cyclobutyl | Cyclobutylalanine |
| hLeu | Homoleucine |
| Gla | γ-Carboxy-glutamic acid |
| Aep | 3-(2-aminoethoxy)propanoic acid |
| Aea | (2-aminoethoxy)acetic acid |
| IsoGlu-octanoic acid | octanoyl-γ-Glu |
| K-octanoic acid | octanoyl-ε-Lys |
| Dapa(Palm) | Hexadecanoyl-β-Diaminopropionic acid |
| IsoGlu-Palm | hexadecanoyl-γ-Glu |
| C-StBu | S-tert-butylthio-cysteine |
| C-tBu | S-tert-butyl-cysteine |
| Dapa(AcBr) | NY-(bromoacetyl)-2,3- diaminopropionic acid |
| Tle | tert-Leucine |
| Phg | phenylglycine |
| Oic | octahydroindole-2-carboxylic acid |
| Chg | α-cyclohexylglycine |
| GP-(Hyp) | Gly-Pro-HydroxyPro |
| Inp | isonipecotic acid |
| Amc | 4-(aminomethyl)cyclohexane carboxylic acid |
| Betaine | (CH₃)₃NCH₂CH₂CO2H |
| D-Npc | Isonipecotic acid |
| Npc | Nipecotic acid |

Throughout the present specification, unless naturally occurring amino acids are referred to by their full name (e.g. alanine, arginine, etc.), they are designated by their conventional three-letter or single-letter abbreviations (e.g. Ala or A for alanine, Arg or R for arginine, etc.). In the case of less common or non-naturally occurring amino acids, unless they are referred to by their full name (e.g. sarcosine, ornithine, etc.), frequently employed three- or four-character codes are employed for residues thereof, including, Sar or Sarc (sarcosine, i.e. N-methylglycine), Aib (α-aminoisobutyric acid), Daba (2,4-diaminobutanoic acid), Dapa (2,3-diaminopropanoic acid), γ-Glu (y-glutamic acid), pGlu (pyroglutamic acid), Gaba (γ-aminobutanoic acid), β-Pro (pyrrolidine-3-carboxylic acid), 8Ado (8-amino-3,6-dioxaoctanoic acid), Abu (4-aminobutyric acid), bhPro (β-homo-proline), bhPhe (β-homo-L-phenylalanine), bhAsp (β-homo-aspartic acid]), Dpa (β,β diphenylalanine), Ida (Iminodiacetic acid), hCys (homocysteine), bhDpa (β-homo-β,β -diphenylalanine).

Furthermore, R¹ can in all sequences be substituted with isovaleric acids or equivalent. In some embodiments, wherein a peptide of the present invention is conjugated to an acidic compound such as, e.g., isovaleric acid, isobutyric acid, valeric acid, and the like, the presence of such a conjugation is referenced in the acid form. So, for example, but not to be limited in any way, instead of indicating a conjugation of isovaleric acid to a peptide by referencing isovaleroyl, in some embodiments, the present application may reference such a conjugation as isovaleric acid.

The term "L-amino acid," as used herein, refers to the "L" isomeric form of a peptide, and conversely the term "D-amino acid" refers to the "D" isomeric form of a peptide. In certain embodiments, the amino acid residues described herein are in the "L" isomeric form, however, residues in the "D" isomeric form can be substituted for any L-amino acid residue, as long as the desired functional is retained by the peptide.

Unless otherwise indicated, reference is made to the L-isomeric forms of the natural and unnatural amino acids in question possessing a chiral center. Where appropriate, the D-isomeric form of an amino acid is indicated in the conventional manner by the prefix "D" before the conventional three-letter code (e.g. Dasp, (D)Asp or D-Asp; Dphe, (D)Phe or D-Phe).

As used herein, a "lower homolog of Lys" refers to an amino acid having the structure of Lysine but with one or more fewer carbons in its side chain as compared to Lysine.

As used herein, a "higher homolog of Lys" refers to an amino acid having the structure of Lysine but with one or more additional carbon atoms in its side chain as compared to Lysine.

The term "DRP," as used herein, refers to disulfide rich peptides.

The term "dimer," as used herein, refers broadly to a peptide comprising two or more monomer subunits. Certain dimers comprise two DRPs. Dimers of the present invention include homodimers and heterodimers. A monomer subunit of a dimer may be linked at its Cor N-terminus, or it may be linked via internal amino acid residues. Each monomer subunit of a dimer may be linked through the same site, or each may be linked through a different site (e.g., C-terminus, N-terminus, or internal site).

As used herein, in the context of certain peptide sequences disclosed herein, parentheticals, e.g., (_), represent side chain conjugations and brackets, e.g., [__], represent unnatural amino acid substitutions or amino acids and conjugated side chains. Generally, where a linker is shown at the N-terminus of a peptide sequence, it indicates that the peptide is dimerized with another peptide, wherein the linker is attached to the N-terminus of the two peptides. Generally, where a linker is shown at the C-terminus of a peptide sequence or structure, it indicates that the peptide is dimerized with another peptide, wherein the linker is attached to the C-terminus of the two peptides.

The term "isostere replacement" or "isostere substitution" are used interchangeably herein to refer to any amino acid or other analog moiety having chemical and/or structural properties similar to a specified amino acid. In certain embodiments, an isostere replacement is a conservative substitution with a natural or unnatural amino acid.

The term "cyclized," as used herein, refers to a reaction in which one part of a polypeptide molecule becomes linked to another part of the polypeptide molecule to form a closed ring, such as by forming a disulfide bridge or other similar bond.

The term "subunit," as used herein, refers to one of a pair of polypeptide monomers that are joined to form a dimer peptide composition.

The term "linker moiety," as used herein, refers broadly to a chemical structure that is capable of linking or joining together two peptide monomer subunits to form a dimer.

The term "solvate" in the context of the present invention refers to a complex of defined stoichiometry formed between a solute (e.g., a hepcidin analogue or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

The term "pharmaceutically acceptable salt," as used herein, represents salts or zwitterionic forms of the peptides or compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methane sulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. A pharmaceutically acceptable salt may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type N(R1)(R2)(R3)(R4)+, where R1, R2, R3 and R4 independently will typically designate hydrogen, optionally substituted C1-6-alkyl or optionally substituted C2-6-alkenyl. Examples of relevant C1-6-alkyl groups include methyl, ethyl, 1-propyl and 2-propyl groups. Examples of C2-6-alkenyl groups of possible relevance include ethenyl, 1-propenyl and 2-propenyl. Other examples of pharmaceutically acceptable salts are described in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977). Also, for a review on suitable salts, see Handbook of Pharmaceutical Salts: Properties, Selection, and Use by Stahl and Wermuth (Wiley-VCH, 2002). Other suitable base salts are formed from bases which form non-toxic salts. Representative examples include the aluminum, arginine, benzathine, calcium, choline, diethylamine, diolamine, glycine, lysine, magnesium, meglumine, olamine, potassium, sodium, tromethamine, and zinc salts. Hemisalts of acids and bases may also be formed, e.g., hemisulphate and hemicalcium salts.

The term "N(alpha)Methylation", as used herein, describes the methylation of the alpha amine of an amino acid, also generally termed as an N-methylation.

The term "sym methylation" or "Arg-Me-sym", as used herein, describes the symmetrical methylation of the two nitrogens of the guanidine group of arginine. Further, the term "asym methylation" or "Arg-Me-asym" describes the methylation of a single nitrogen of the guanidine group of arginine.

The term "acylating organic compounds", as used herein refers to various compounds with carboxylic acid functionality that are used to acylate the N-terminus of an amino acid subunit prior to forming a C-terminal dimer. Non-limiting examples of acylating organic compounds include cyclopropylacetic acid, 4-Fluorobenzoic acid, 4-fluorophenylacetic acid, 3-Phenylpropionic acid, Succinic acid, Glutaric acid, Cyclopentane carboxylic acid, 3,3,3-trifluoropropeonic acid, 3-Fluoromethylbutyric acid, Tetrahedro-2H-Pyran-4-carboxylic acid.

The term "alkyl" includes a straight chain or branched, noncyclic or cyclic, saturated aliphatic hydrocarbon containing from 1 to 24 carbon atoms. Representative saturated straight chain alkyls include, but are not limited to, methyl, ethyl, n-propyl, n-butyl, n-pentyl, n-hexyl, and the like, while saturated branched alkyls include, without limitation, isopropyl, sec-butyl, isobutyl, tert-butyl, isopentyl, and the like. Representative saturated cyclic alkyls include, but are not limited to, cyclopropyl, cyclobutyl, cyclopentyl, cyclohexyl, and the like, while unsaturated cyclic alkyls include, without limitation, cyclopentenyl, cyclohexenyl, and the like.

As used herein, a "therapeutically effective amount" of the peptide agonists of the invention is meant to describe a sufficient amount of the peptide agonist to treat an hepcidin-related disease, including but not limited to any of the diseases and disorders described herein (for example, a disease of iron metabolism). In particular embodiments, the therapeutically effective amount will achieve a desired benefit/risk ratio applicable to any medical treatment.

Hematocrit is the ratio of the volume of red cells to the volume of whole blood. Normal range for hematocrit is different between the sexes and is approximately 45% to 52% for men and 37% to 48% for women. A clinical goal for treatment of PV is to achieve a hematocrit below 45%. Hematocrit may also be referred to as hematocrit levels herein, and it is understood that a numerical value for a hematocrit level, e.g., 45, means a hematocrit of 45%.

### Peptide Analogues of Hepcidin

The present invention provides peptide analogues of hepcidin, which may be monomers or dimers (collectively "hepcidin analogues").

A hepcidin analogue described herein may bind ferroportin, e.g., human ferroportin. The hepcidin analogues may specifically bind human ferroportin. As used herein, "specifically binds" refers to a specific binding agent's preferential interaction with a given ligand over other agents in a sample. For example, a specific binding agent that specifically binds a given ligand, binds the given ligand, under suitable conditions, in an amount or a degree that is observable over that of any nonspecific interaction with other components in the sample. Suitable conditions are those that allow interaction between a given specific binding agent and a given ligand. These conditions include pH, temperature, concentration, solvent, time of incubation, and the like, and may differ among given specific binding agent and ligand pairs, but may be readily determined by those skilled in the art. A hepcidin analogue described herein may bind ferroportin with greater specificity than a hepcidin reference compound (e.g., any one of the hepcidin reference compounds provided herein). A hepcidin analogue of the present invention may exhibit ferroportin specificity that is at least about 10%, 20%, 30%, 40%, 50%, 60%, 70%, 80%, 90%, 100%, 200%, 300%, 400%, 500%, 700%, 1000%, or 10,000% higher than a hepcidin reference compound (e.g., any one of the hepcidin reference compounds provided herein. A hepcidin analogue of the present invention may exhibit ferroportin specificity that is at least about 5 fold, or at least about 10, 20, 50, or 100 fold higher than a hepcidin reference compound (e.g., any one of the hepcidin reference compounds provided herein.

The hepcidin analogue described herein may exhibit a hepcidin activity, which activity may be an *in vitro* or an *in vivo* activity, e.g., an *in vivo* or an *in vitro* activity described herein. A hepcidin analogue described herein may exhibit at least about 50%, 60%, 70%, 80%, 90%, 95%, 97%, 98%, 99%, or greater than 99% of the activity exhibited by a hepcidin reference compound (e.g., any one of the hepcidin reference compounds provided herein.

A hepcidin analogue described herein may exhibit at least about 50%, 60%, 70%, 80%, 90%, 95%, 97%, 98%, 99%, or greater than 99% of the ferroportin binding ability that is exhibited by a reference hepcidin. A hepcidin analogue described herein may have a lower IC₅₀ (i.e., higher binding affinity) for binding to ferroportin, (e.g., human ferroportin) compared to a reference hepcidin. A hepcidin analogue described herein may have an IC₅₀ in a ferroportin competitive binding assay which is at least about 10%, 20%, 30%, 40%, 50%, 60%, 70%, 80%, 90%, 100%, 200%, 300%, 400%, 500%, 700%, or 1000% lower than a reference hepcidin.

A hepcidin analogue described herein may exhibit increased hepcidin activity as compared to a hepcidin reference peptide. The activity may be an *in vitro* or an *in vivo* activity, e.g., an *in vivo* or an *in vitro* activity described herein. A hepcidin analogue described herein may exhibit 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater hepcidin activity than a reference hepcidin. A hepcidin analogue described herein may exhibit at least about 10%, 20%, 30%, 40%, 50%, 60%, 70%, 80%, 90%, 95%, 97%, 98%, 99% or greater than 99%, 100%, 200% 300%, 400%, 500%, 700%, or 1000% greater activity than a reference hepcidin.

A peptide analogue described herein may exhibit at least about 50%, 60%, 70%, 80%, 90%, 95%, 97%, 98%, 99%, or greater than 99%, 100%, 200% 300%, 400%, 500%, 700%, or 1000% greater *in vitro* activity for inducing the degradation of human ferroportin protein as that of a reference hepcidin, wherein the activity is measured according to a method described herein.

Apeptide or a peptide dimer described herein may exhibit at least about 50%, 60%, 70%, 80%, 90%, 95%, 97%, 98%, 99%, or greater than 99%, 100%, 200% 300%, 400%, 500%, 700%, or 1000% greater *in vivo* activity for inducing the reduction of free plasma iron in an individual as does a reference hepcidin, wherein the activity is measured according to a method described herein.

The activity may be an *in vitro* or an *in vivo* activity, e.g., an *in vivo* or an *in vitro* activity described herein. A hepcidin analogue described herein may exhibit 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater or at least about 10%, 20%, 30%, 40%, 50%, 60%, 70%, 80%, 90%, 100%, 200%, 300%, 400%, 500% , 700%, or 1000% greater activity than a reference hepcidin, wherein the activity is an *in vitro* activity for inducing the degradation of ferroportin, e.g., as measured according to the Examples herein; or wherein the activity is an *in vivo* activity for reducing free plasma iron, e.g., as measured according to the Examples herein.

A hepcidin analogue described herein may mimic the hepcidin activity of Hep25, the bioactive human 25-amino acid form, are herein referred to as "mini-hepcidins". As used herein, in certain embodiments, a compound (e.g., a hepcidin analogue) having a "hepcidin activity" means that the compound has the ability to lower plasma iron concentrations in subjects (e.g. mice or humans), when administered thereto (e.g. parenterally injected or orally administered), in a dose-dependent and time-dependent manner. See e.g. as demonstrated in Rivera et al. (2005), Blood 106:2196-9. The peptides described herein may lower the plasma iron concentration in a subject by at least about 1.2, 1.5, 2, 3, 4, 5, 6, 7, 8, 9, or 10-fold, or at least about 5%, 10%, 20%, 25%, 30%, 40%, 50%, 60%, 70%, 80%, 90%, or about 99%.

The hepcidin analogues described herein may have *in vitro* activity as assayed by the ability to cause the internalization and degradation of ferroportin in a ferroportin-expressing cell line as taught in Nemeth et al. (2006) Blood 107:328-33. *in vitro* activity may be measured by the dose-dependent loss of fluorescence of cells engineered to display ferroportin fused to green fluorescent protein as in Nemeth et al. (2006) Blood 107:328-33. Aliquots of cells are incubated for 24 hours with graded concentrations of a reference preparation of Hep25 or a mini-hepcidin. As provided herein, the EC₅₀ values are provided as the concentration of a given compound (e.g. a hepcidin analogue peptide or peptide dimer of the present invention) that elicits 50% of the maximal loss of fluorescence generated by a reference compound. The EC₅₀ of the Hep25 preparations in this assay range from 5 to 15 nM and in certain embodiments, preferred hepcidin analogues of the present invention have EC₅₀ values in *in vitro* activity assays of about 1,000 nM or less. A hepcidin analogue described herein may have an EC₅₀ in an *in vitro* activity assay (e.g., as described in Nemeth et al. (2006) Blood 107:328-33 or the Example herein) of less than about any one of 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 40, 50, 60, 70, 80, 90, 100, 200 or 500 nM. A hepcidin analogue or biotherapeutic composition (e.g., any one of the pharmaceutical compositions described herein) has an EC₅₀ value of about 1nM or less.

Other methods known in the art for calculating the hepcidin activity and *in vitro* activity of the hepcidin analogues according to the present invention may be used. For example, in certain embodiments, the *in vitro* activity of the hepcidin analogues or the reference peptides is measured by their ability to internalize cellular ferroportin, which is determined by immunohistochemistry or flow cytometry using antibodies which recognizes extracellular epitopes of ferroportin. Alternatively, in certain embodiments, the *in vitro* activity of the hepcidin analogues or the reference peptides is measured by their dose-dependent ability to inhibit the efflux of iron from ferroportin-expressing cells that are preloaded with radioisotopes or stable isotopes of iron, as in Nemeth et al. (2006) Blood 107:328-33.

The hepcidin analogues described herein may exhibit increased stability (e.g., as measured by half-life, rate of protein degradation) as compared to a reference hepcidin. The stability of a hepcidin analogue described herein may be increased at least about 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater or at least about 10%, 20%, 30%, 40%, 50%, 60%, 70%, 80%, 90%, 100%, 200%, 300%, 400%, or 500% greater than a reference hepcidin. The stability may be a stability that is described herein. The stability may be a plasma stability, e.g., as optionally measured according to the method described herein. The stability may be stability when delivered orally.

A hepcidin analogue described herein may exhibits a longer half-life than a reference hepcidin. A hepcidin analogue described herein may have a half-life under a given set of conditions (e.g., temperature, pH) of at least about 5 minutes, at least about 10 minutes, at least about 20 minutes, at least about 30 minutes, at least about 45 minutes, at least about 1 hour, at least about 2 hour, at least about 3 hours, at least about 4 hours, at least about 5 hours, at least about 6 hours, at least about 12 hours, at least about 18 hours, at least about 1 day, at least about 2 days, at least about 4 days, at least about 7 days, at least about 10 days, at least about two weeks, at least about three weeks, at least about 1 month, at least about 2 months, at least about 3 months, or more, or any intervening half-life or range in between, about 5 minutes, about 10 minutes, about 20 minutes, about 30 minutes, about 45 minutes, about 1 hour, about 2 hour, about 3 hours, about 4 hours, about 5 hours, about 6 hours, about 12 hours, about 18 hours, about 1 day, about 2 days, about 4 days, about 7 days, about 10 days, about two weeks, about three weeks, about 1 month, about 2 months, about 3 months, or more, or any intervening half-life or range in between. The half-life of a hepcidin analogue described herein may be extended due to its conjugation to one or more lipophilic substituent or half-life extension moiety, e.g., any of the lipophilic substituents or half-life extension moieties disclosed herein. The half-life of a hepcidin analogue described herein may be extended due to its conjugation to one or more polymeric moieties, e.g., any of the polymeric moieties or half-life extension moieties disclosed herein. Ahepcidin analogue described herein may have a half-life as described above under the given set of conditions wherein the temperature is about 25 °C, about 4 °C, or about 37 °C, and the pH is a physiological pH, or a pH about 7.4.

A hepcidin analogue described herein, comprising a conjugated half-life extension moiety, may have an increased serum half-life following oral, intravenous or subcutaneous administration as compared to the same analogue but lacking the conjugated half-life extension moiety. The serum half-life of a hepcidin analogue described herein following any of oral, intravenous or subcutaneous administration may be at least 12 hours, at least 24 hours, at least 30 hours, at least 36 hours, at least 48 hours, at least 72 hours or at least 168 h. In particular embodiments, it is between 12 and 168 hours, between 24 and 168 hours, between 36 and 168 hours, or between 48 and 168 hours.

A hepcidin analogue described herein, comprising a conjugated half-life extension moiety, may result in decreased concentration of serum iron following oral, intravenous or subcutaneous administration to a subject. The subject's serum iron concentration may be decreased to less than 10%, less than 20%, less than 25%, less than 30%, less than 40%, less than 50%, less than 60%, less than 70%, less than 80%, or less than 90% of the serum iron concentration in the absence of administration of the hepcidin analogue to the subject. The decreased serum iron concentration may remain for a least 1 hour, at least 4 hours, at least 10 hours, at least 12 hours, at least 24 hours, at least 36 hours, at least 48 hours, or at least 72 hours following administration to the subject. It may remain for between 12 and 168 hours, between 24 and 168 hours, between 36 and 168 hours, or between 48 and 168 hours. The serum iron concentration of the subject may be reduced to less than 20% at about 4 hours or about 10 hours following administration to the subject, e.g., intravenously, orally, or subcutaneously. The serum iron concentration of the subject may be reduced to less than 50% or less than 60% for about 24 to about 30 hours following administration, e.g., intravenously, orally, or subcutaneously.

The half-life may be measured *in vitro* using any suitable method known in the art, e.g., the stability of a hepcidin analogue may be determined by incubating the hepcidin analogue with pre-warmed human serum (Sigma) at 37 ° C. Samples are taken at various time points, typically up to 24 hours, and the stability of the sample is analyzed by separating the hepcidin analogue from the serum proteins and then analyzing for the presence of the hepcidin analogue of interest using LC-MS.

The stability of the hepcidin analogue may be measured *in vivo* using any suitable method known in the art, e.g., the stability of the hepcidin analogue may be determined in vivo by administering the peptide or peptide dimer to a subject such as a human or any mammal (e.g., mouse) and then samples are taken from the subject via blood draw at various time points, typically up to 24 hours. Samples are then analyzed as described above in regard to the *in vitro* method of measuring half-life. In vivo stability of a hepcidin analogue described herein may be determined via the method disclosed in the Examples herein.

The hepcidin analogue may exhibit improved solubility or improved aggregation characteristics as compared to a reference hepcidin. Solubility may be determined via any suitable method known in the art. Suitable methods known in the art for determining solubility include incubating peptides (e.g., a hepcidin analogue of the present invention) in various buffers (Acetate pH4.0, Acetate pH5.0, Phos/Citrate pH5.0, Phos Citrate pH6.0, Phos pH 6.0, Phos pH 7.0, Phos pH7.5, Strong PBS pH 7.5, Tris pH7.5, Tris pH 8.0, Glycine pH 9.0, Water, Acetic acid (pH 5.0 and other known in the art) and testing for aggregation or solubility using standard techniques. These include, but are not limited to, visual precipitation, dynamic light scattering, Circular Dichroism and fluorescent dyes to measure surface hydrophobicity, and detect aggregation or fibrillation, for example. Improved solubility may mean the peptide (e.g., the hepcidin analogue of the present invention) is more soluble in a given liquid than is a reference hepcidin.

The hepcidin analogue may exhibit a solubility that is increased at least about 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater or at least about 10%, 20%, 30%, 40%, 50%, 60%, 70%, 80%, 90%, 100%, 200%, 300%, 400%, or 500% greater than a reference hepcidin in a particular solution or buffer, e.g., in water or in a buffer known in the art or disclosed herein.

The hepcidin analogue may exhibit decreased aggregation, wherein the aggregation of the peptide in a solution is at least about 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold less or at least about 10%, 20%, 30%, 40%, 50%, 60%, 70%, 80%, 90%, 100%, 200%, 300%, 400%, or 500% less than a reference hepcidin in a particular solution or buffer, e.g., in water or in a buffer known in the art or disclosed herein.

The hepcidin analogue may exhibit less degradation (i.e., more degradation stability), e.g., greater than or about 10% less, greater than or about 20% less, greater than or about 30% less, greater than or about 40 less, or greater than or about 50% less than a reference hepcidin. In some embodiments, degradation stability is determined via any suitable method known in the art. Suitable methods known in the art for determining degradation stability include the method described in Hawe et al J Pharm Sci, VOL. 101, NO. 3, 2012, p 895-913. Such methods are in some embodiments used to select potent sequences with enhanced shelf lives.

The hepcidin analogue of the present invention may be synthetically manufactured. In other embodiments, the hepcidin analogue of the present invention is recombinantly manufactured.

The various hepcidin analogue monomer and dimer peptides described herein may be constructed solely of natural amino acids. Alternatively, these hepcidin analogues may include unnatural or non-natural amino acids including, but not limited to, modified amino acids. Modified amino acids may include natural amino acids that have been chemically modified to include a group, groups, or chemical moiety not naturally present on the amino acid. The hepcidin analogues described herein may additionally include D-amino acids. Still further, the hepcidin analogue peptide monomers and dimers may include amino acid analogs. A peptide analogue described herein may comprise any of those described herein, wherein one or more natural amino acid residues of the peptide analogue is substituted with an unnatural or non-natural amino acid, or a D-amino acid.

The hepcidin analogues described herein may include one or more modified or unnatural amino acids. For example, a hepcidin analogue may include one or more of Daba, Dapa, Pen, Sar, Cit, Cav, HLeu, 2-Nal, 1-Nal, d-1-Nal, d-2-Nal, Bip, Phe(4-OMe), Tyr(4-OMe), βhTrp, βhPhe, Phe(4-CF₃), 2-2-Indane, 1-1-Indane, Cyclobutyl, βhPhe, hLeu, Gla, Phe(4-NH₂), hPhe, 1-Nal, Nle, 3-3-diPhe, cyclobutyl-Ala, Cha, Bip, β-Glu, Phe(4-Guan), homo amino acids, D-amino acids, and various N-methylated amino acids. One having skill in the art will appreciate that other modified or unnatural amino acids, and various other substitutions of natural amino acids with modified or unnatural amino acids, may be made to achieve similar desired results, and that such substitutions are within the teaching of the present invention.

Compounds described herein include isotopically-labeled compounds, which are identical to those recited in the various formulas and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, fluorine and chlorine, such as ²H, ³H, ¹3C, ¹⁴C, ¹⁵N, ¹⁸O, ¹⁷O, ³⁵S, ¹⁸F, ³⁶Cl, respectively. Certain isotopically-labeled compounds described herein, for example those into which radioactive isotopes such as ³H and ¹⁴C are incorporated, are useful in drug and/or substrate tissue distribution assays. Further, substitution with isotopes such as deuterium, i.e., ²H, can afford certain therapeutic advantages resulting from greater metabolic stability, for example increased in vivo half-life or reduced dosage requirements.

Also disclosed herein are any of the peptide monomers or dimers described herein linked to a linker moiety, including any of the specific linker moieties described herein.

The hepcidin analogues described herein may include peptides, e.g., monomers or dimers, comprising a peptide monomer subunit having at least 85%, at least 90%, at least 92%, at least 94%, at least 95%, at least 98%, or at least 99% amino acid sequence identity to a hepcidin analogue peptide sequence described herein (e.g., any one of the peptides disclosed herein), including but not limited to any of the amino acid sequences shown in Tables 2 and 3.

A peptide analogue described herein, or a monomer subunit of a dimer peptide analogue described herein, may comprise or consist of 7 to 35 amino acid residues, 8 to 35 amino acid residues, 9 to 35 amino acid residues, 10 to 35 amino acid residues, 7 to 25 amino acid residues, 8 to 25 amino acid residues, 9 to 25 amino acid residues, 10 to 25 amino acid residues, 7 to 18 amino acid residues, 8 to 18 amino acid residues, 9 to 18 amino acid residues, or 10 to 18 amino acid residues, and, optionally, one or more additional non-amino acid moieties, such as a conjugated chemical moiety, e.g., a half-life extension moiety, a PEG or linker moiety. A monomer subunit of a hepcidin analogue may comprise or consist of 7, 8, 9, 10, 11, 12, 13, 14, 15, 16, 17, 18, 19, 20, 21, 22, 23, 24, 25, 26, 27, 28, 29, 30, 31, 32, 33, 34, or 35 amino acid residues. A monomer subunit of a hepcidin analogue described herein may comprise or consist of 10 to 18 amino acid residues and, optionally, one or more additional non-amino acid moieties, such as a conjugated chemical moiety, e.g., a PEG or linker moiety. The monomer subunit may comprise or consist of 7 to 35 amino acid residues, 9 to 18 amino acid residues, or 10 to 18 amino acid residuesX may comprise or consist of 7 to 35 amino acid residues, 8 to 35 amino acid residues, 9 to 35 amino acid residues, 10 to 35 amino acid residues, 7 to 25 amino acid residues, 8 to 25 amino acid residues, 9 to 25 amino acid residues, 10 to 25 amino acid residues, 7 to 18 amino acid residues, 8 to 18 amino acid residues, 9 to 18 amino acid residues, or 10 to 18 amino acid residues.

Also described herein are hepcidin analogues disclosed in PCT Patent Application Publication Nos. WO2014/145561, WO2015/200916, or WO2017/117411.

### Peptide Monomer Hepcidin Analogues

The present invention provides a hepcidin analogue or a pharmaceutically acceptable salt or solvate thereof, for use in a method of treating polycythemia vera in a human subject in need thereof, wherein the hepcidin analogue is: 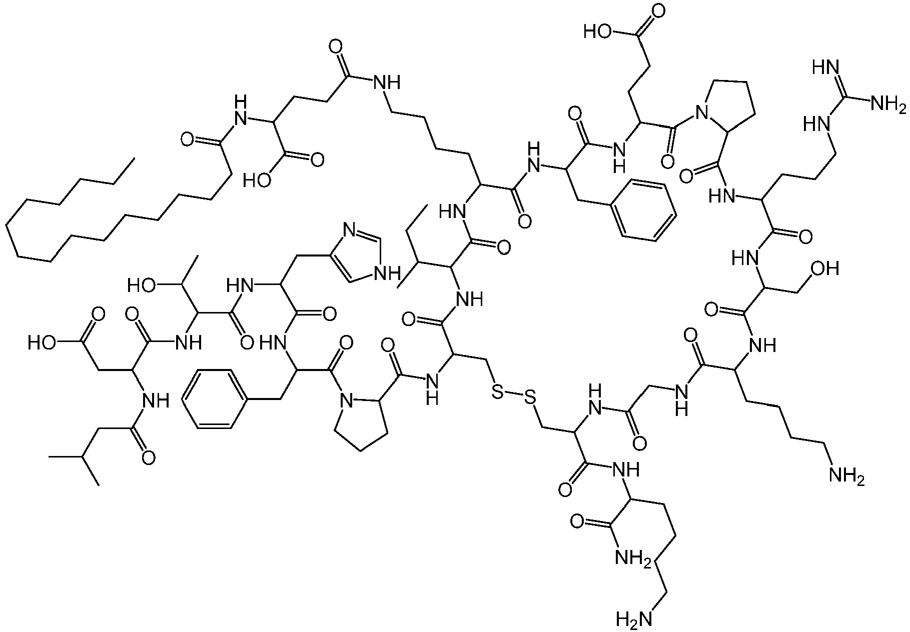 Isovaleric acid-DTHFPCI(K(isoGlu-Palm))FEPRSKGCK-NH2 (SEQ ID NO:45);
wherein the amino acids are L-amino acids; and wherein the hepcidin analogue or pharmaceutically acceptable salt thereof is administered via subcutaneous injection; the subject is administered from about 20 mg to about 100 mg of the hepcidin analogue or pharmaceutically acceptable salt thereof; and the hepcidin analogue or pharmaceutically acceptable salt thereof is provided to the subject about twice a week, about once a week, or about once every two weeks.

Also described herein is a method of treating Polycythemia vera in a subject in need thereof, comprising administering to the subject an effective amount of a hepcidin analogue or pharmaceutically acceptable salt thereof, or a pharmaceutical composition comprising a hepcidin analogue or pharmaceutically acceptable salt thereof and a pharmaceutically acceptable carrier, diluent or excipient, wherein the hepcidin analogue comprises a peptide comprising Formula (I):

R1-X-Y-R2 (I) (SEQ ID NO: 1)

or a pharmaceutically acceptable salt or solvate thereof,
wherein
R1 is hydrogen, a C1-C6 alkyl, a C6-C12 aryl, a C1-C20 alkanoyl or pGlu;
R2 is NH₂ or OH;
X is a peptide sequence having the formula II

X1-X2-X3-X4-X5-X6-X7-X8-X9-X10 (II) (SEQ ID NO:2)

wherein
X1 is Asp, Ala, Ida, pGlu, bhAsp, Leu, D-Asp or absent;
X2 is Thr, Ala, or D-Thr;
X3 is His, Lys, or D-His;
X4 is Phe, Ala, Dpa or D-Phe;
X5 is Pro, Gly, Arg, Lys, Ala, D-Pro or bhPro;
X6 is Ile, Cys, Arg, Lys, D-Ile or D-Cys;
X7 is Cys, Ile, Leu, Val, Phe, D-Ile or D-Cys;
X8 is Ile, Arg, Phe, Gln, Lys, Glu, Val, Leu or D-Ile;
X9 is Phe or bhPhe; and
X10 is Lys, Phe or absent;
wherein if Y is absent, X7 is Ile; and
Y is a peptide sequence having the formula III

Y1-Y2-Y3-Y4-Y5-Y6-Y7-Y8-Y9-Y10-Y11-Y12-Y13-Y14-Y15 (III) (SEQ ID NO:3)

wherein
Y1 is Gly, Cys, Ala, Phe, Pro, Glu, Lys, D-Pro, Val, Ser or absent;
Y2 is Pro, Ala, Cys, Gly or absent;
Y3 is Arg, Lys, Pro, Gly, His, Ala, Trp or absent;
Y4 is Ser, Arg, Gly, Trp, Ala, His, Tyr or absent;
Y5 is Lys, Met, Arg, Ala or absent;
Y6 is Gly, Ser, Lys, Ile, Ala, Pro, Val or absent;
Y7 is Trp, Lys, Gly, Ala, Ile, Val or absent;
Y8 is Val, Thr, Gly, Cys, Met, Tyr, Ala, Glu, Lys, Asp, Arg or absent;
Y9 is Cys, Tyr or absent;
Y10 is Met, Lys, Arg, Tyr or absent;
Y11 is Arg, Met, Cys, Lys or absent;
Y12 is Arg, Lys, Ala or absent;
Y13 is Arg, Cys, Lys, Val or absent;
Y14 is Arg, Lys, Pro, Cys, Thr or absent; and
Y15 is Thr, Arg or absent;
wherein said peptide of formula I is optionally PEGylated on R1, X, or Y, and wherein a side chain of an amino acid of the peptide is optionally conjugated to a lipophilic substituent or polymeric moiety.

Alternatively, formula II is shown as above for X1, X2, X4, X5, X6, X7, X8, X9, and X1, but X3 is His, Lys, D-His, or D-Lys.

R1 may be hydrogen, isovaleric acid, isobutyric acid or acetyl. For example, R1 may be isovaleric acid, or isobutyric acid. Alternatively, R1 is isovaleric acid.

X may be a peptide sequence having formula IV:

X1-Thr-His-X4-X5-X6-X7-X8-Phe-X10 (IV) (SEQ ID NO:4)

wherein
X1 is Asp, Ida, pGlu, bhAsp or absent;
X4 is Phe or Dpa;
X5 is Pro or bhPro;
X6 is Ile, Cys or Arg;
X7 is Cys, Ile, Leu or Val;
X8 is Ile, Lys, Glu, Phe, Gln or Arg; and
X10 is Lys or absent.

X may be a peptide sequence having formula V:

X1-Thr-His-X4-X5-Cys-Ile-X8-Phe-X10 (V) (SEQ ID NO:5)

X1 is Asp, Ida, pGlu, bhAsp or absent;
X4 is Phe or Dpa;
X5 is Pro or bhPro;
X8 is Ile, Lys, Glu, Phe, Gln or Arg; and
X10 is Lys or absent.

The peptide may beaccording to formula VI:

R¹-X-Y-R² (VI) (SEQ ID NO:6)

or a pharmaceutically acceptable salt thereof, wherein:
R¹ is hydrogen, isovaleric acid, isobutyric acid or acetyl;
R² is -NH₂ or -OH;
X is a peptide sequence having a formula VII:

   X1-Thr-His-X4-X5-Cys-Ile-X8-Phe-X10 (VII) (SEQ ID NO:7)
wherein
X1 is Asp, Ida, pGlu, bhAsp or absent;
X4 is Phe or Dpa;
X5 is Pro or bhPro;
X8 is Ile, Lys, Glu, Phe, Gln or Arg; and
X10 is Lys or absent;
wherein Y is a peptide sequence having a formula VIII:

   Y1-Pro-Y3-Ser-Y5-Y6-Y7-Y8-Cys-Y10 (VIII) (SEQ ID NO:8)
wherein
Y1 is Gly, Glu, Val or Lys;
Y3 is Arg or Lys;
Y5 is Arg or Lys;
Y6 is Gly, Ser, Lys, Ile or Arg;
Y7 is Trp or absent;
Y8 is Val, Thr, Asp, Glu or absent; and
Y10 is Lys or absent;
wherein the peptide comprises a disulfide bond between the two Cys;
wherein said peptide of formula I is optionally PEGylated on R¹, X, or Y;
wherein a side chain of an amino acid of the peptide is optionally conjugated to a lipophilic substituent or polymeric moiety; and
wherein Ida is iminodiacetic acid; pGlu is pyroglutamic acid; bhAsp is β-homoaspartic acid; and bhPro is β-homoproline.

The peptide may comprise one of the following sequences:
DTHFPICIFGPRSKGWVC (SEQ ID NO:9);
DTHFPCIIFGPRSKGWVCK (SEQ ID NO:10);
DTHFPCIIFEPRSKGWVCK (SEQ ID NO:11);
DTHFPCIIFGPRSKGWACK (SEQ ID NO:12);
DTHFPCIIFGPRSKGWVCKK (SEQ ID NO:13);
DTHFPCIIFVCHRPKGCYRRVCR (SEQ ID NO:14);
DTHFPCIKFGPRSKGWVCK (SEQ ID NO:15);
DTHFPCIKFKPRSKGWVCK (SEQ ID NO:16);
DTHFPCIIFGPRSRGWVCK (SEQ ID NO:17);
DTHFPCIKFGPKSKGWVCK (SEQ ID NO:18);
DTHFPCIKFEPRSKGCK (SEQ ID NO:19);
DTHFPCIKFEPKSKGWECK (SEQ ID NO:20);
DTHFPCIKFEPRSKKCK (SEQ ID NO:21);
DTHFPCIKFEPRSKGCKK (SEQ ID NO:22);
DTHFPCIKFKPRSKGCK (SEQ ID NO:23);
DTHFPCIKFEPKSKGCK (SEQ ID NO:24);
DTHFPCIKF (SEQ ID NO:25);
DTHFPCIIF (SEQ ID NO:26); or
DTKFPCIIF (SEQ ID NO:27),
wherein said peptide is optionally PEGylated on R1, X, or Y, and wherein a side chain of an amino acid of the peptide is optionally conjugated to a lipophilic substituent or polymeric moiety.

The hepcidin analogue or peptide may comprise one of the following sequences:
Isovaleric acid-DTHFPICIFGPRSKGWVC-NH₂ (SEQ ID NO:9);
Isovaleric acid-DTHFPCIIFGPRSKGWVCK-NH₂ (SEQ ID NO:10)_{;}
Isovaleric acid-DTHFPCIIFEPRSKGWVCK-NH₂ (SEQ ID NO:11)_{;}
Isovaleric acid-DTHFPCIIFGPRSKGWACK-NH₂ (SEQ ID NO:12)_{;}
Isovaleric acid-DTHFPCIIFGPRSKGWVCKK-NH₂ (SEQ ID NO:13)_{;}
Isovaleric acid-DTHFPCIIFVCHRPKGCYRRVCR-NH₂ (SEQ ID NO:14)_{;}
Isovaleric acid-DTHFPCI(K(PEG8))FGPRSKGWVCK-NH₂ (SEQ ID NO:28);
Isovaleric acid-DTHFPCIKF(K(PEG8))PRSKGWVCK-NH₂ (SEQ ID NO:16)_{;}
Isovaleric acid-DTHFPICIFGPRS(K(PEG8))GWVC-NH₂ (SEQ ID NO:29);
Isovaleric acid-DTHFPICIFGPRS(K(PEG4))GWVC-NH₂ (SEQ ID NO:30);
Isovaleric acid-DTHFPCIIFGPRSRGWVC(K(PEG8))-NH₂ (SEQ ID NO:31);
Isovaleric acid-DTHFPCIIFGPRSRGWVC(K(PEG4))-NH₂(SEQ ID NO:32);
Isovaleric acid-DTHFPCIIFGPRSRGWVC(K(PEG2))-NH₂ (SEQ ID NO:33);
Isovaleric acid-DTHFPCI(K(Palm))FGPRSKGWVCK-NH₂ (SEQ ID NO:34);
Isovaleric acid-DTHFPCIKF)K(Palm))PRSKGWVCK-NH₂(SEQ ID NO:35);
Isovaleric acid-DTHFPCIKFGP(K(Palm))SKGWVCK-NH₂(SEQ ID NO:36);
Isovaleric acid-DTHFPCIKFGPRS(K(Palm))GWVCK-NH₂ (SEQ ID NO:37);
Isovaleric acid-DTHFPCIKFGPRSKGWVC(K(Palm))NH₂ (SEQ ID NO:38);
Isovaleric acid-DTHFPCI(K(PEG3-Palm))FGPRSKGWVCK-NH₂ (SEQ ID NO:39);
Isovaleric acid-DTHFPCIKF(K(PEG3-Palm))PRSKGWVCK-NH₂ (SEQ ID NO:40)_{;}
Isovaleric acid-DTHFPCIKFGP(K(PEG3-Palm))SKGWVCK-NH₂(SEQ ID NO:41)_{;}
Isovaleric acid-DTHFPCIKFGPRS(K(PEG3-Palm))GWVCK-NH₂ (SEQ ID NO:42)_{;}
Isovaleric acid-DTHFPCIKFGPRSKGWVC(K(PEG3-Palm))-NH₂ (SEQ ID NO:43)_{;}
Isovaleric acid-DTHFPCIKFGPRSKGWVC(K(PEG8))-NH₂ (SEQ ID NO:44)_{;}
Isovaleric acid-DTHFPCIKF-K(isoGlu-Palm)-PRSKGCK-NH₂(SEQ ID NO:46)_{;}
Isovaleric acid-DTHFPCIKFEP(K(isoGlu-Palm))SKGCK-NH₂ (SEQ ID NO:47)_{;}
Isovaleric acid-DTHFPCIKFEP(K(isoGlu-Palm))SKGWECK-NH₂ (SEQ ID NO:20)_{;}
Isovaleric acid-DTHFPCIKFEPRS(K(isoGlu-Palm))GCK-NH₂(SEQ ID NO:48)_{;}
Isovaleric acid-DTHFPCIKFEPRSK(K(isoGlu-Palm))CK-NH₂ (SEQ ID NO:21);
Isovaleric acid-DTHFPCIKFEPRSKGCK(K(isoGlu-Palm))-NH₂ (SEQ ID NO:49);
Isovaleric acid-DTHFPCI-K(Dapa-Palm)-FEPRSKGCK-NH₂(SEQ ID NO:50)_{;}
Isovaleric acid-DTHFPCIK(F(Dapa-Palm))PRSKGCK-NH₂(SEQ ID NO:23) ;
Isovaleric acid-DTHFPCIKFEP(K(Dapa-Palm))SKGCK-NH₂ (SEQ ID NO:24);
Isovaleric acid-DTHFPCIKFEPRS(K(Dapa-Palm))GCK-NH₂(SEQ ID NO:51) ;
Isovaleric acid-DTHFPCIKFEPRSK(K(Dapa-Palm))CK-NH₂(SEQ ID NO:52) ;
Isovaleric acid-DTHFPCIKFEPRSKGC(K(Dapa-Palm))K-NH₂ (SEQ ID NO:53);
Isovaleric acid-DTHFPCIKFEPRSKGC(K(Dapa-Palm))-NH₂ (SEQ ID NO:54);
Isovaleric acid-DTHFPCIKF(K(PEG11-Palm))PRSK[Sar]CK-NH₂ (SEQ ID NO:55);
Isolvaleric acid-DTHFPCIKF-NH₂ (SEQ ID NO:25);
Hy-DTHFPCIKF-NH₂ (SEQ ID NO:25);
Isolvaleric acid-DTHFPCIIF-NH₂ (SEQ ID NO:26);
Hy-DTHFPCIIKF-NH₂ (SEQ ID NO:26);
Isovaleric acid-DTKFPCIIF-NH₂ (SEQ ID NO:27); or
Hy-DTKFPCIIF-NH₂ (SEQ ID NO:27).

In one example, the hepcidin analogue or peptide is: Isovaleric acid-DTHFPCIIFGPRSKGWVCK-NH₂ (SEQ ID NO:10).

In one example, the hepcidin analogue or peptide is: Isovaleric acid-DTHFPCIIFEPRSKGWVCK-NH₂ (SEQ ID NO:11).

In one example, the hepcidin analogue or peptide is: Isovaleric acid-DTHFPCI(K(PEG8))FGPRSKGWVCK-NH₂ (SEQ ID NO:28).

In one example, the hepcidin analogue or peptide is: Isovaleric acid-DTHFPCIKF(K(PEG8))PRSKGWVCK-NH₂ (SEQ ID NO:16).

In one example, the hepcidin analogue or peptide is: Isovaleric acid-DTHFPCIIFGPRSRGWVC(K(PEG8))-NH₂ (SEQ ID NO:31).

In one example, the hepcidin analogue or peptide is: Isovaleric acid-DTHFPCI(K(Palm))FGPRSKGWVCK-NH₂ (SEQ ID NO:34).

In one example, the hepcidin analogue or peptide is: Isovaleric acid-DTHFPCIKF(K(Palm))PRSKGWVCK-NH₂ (SEQ ID NO:35).

In one example, the hepcidin analogue or peptide is: Isovaleric acid-DTHFPCIKFGP(K(Palm))SKGWVCK-NH₂ (SEQ ID NO:36).

In one example, the hepcidin analogue or peptide is: Isovaleric acid-DTHFPCIKFGPRSKGWVC(K(Palm))-NH₂ (SEQ ID NO:38)

In one example, the hepcidin analogue or peptide is: Isovaleric acid-DTHFPCI(K(PEG3-Palm))FGPRSKGWVCK-NH₂ (SEQ ID NO:39).

In one example, the hepcidin analogue or peptide is: Isovaleric acid-DTHFPCIKF(K(PEG3-Palm))PRSKGWVCK-NH₂ (SEQ ID NO:40).

In one example, the hepcidin analogue or peptide is: Isovaleric acid-DTHFPCIKFGP(K(PEG3-Palm))SKGWVCK-NH₂(SEQ ID NO:41).

In one example, the hepcidin analogue or peptide is: Isovaleric acid-DTHFPCIKFGPRS(K(PEG3-Palm))GWVCK-NH₂ (SEQ ID NO:42).

In one example, the hepcidin analogue or peptide is: Isovaleric acid-DTHFPCIKFGPRSKGWVC(K(PEG3-Palm))-NH₂ (SEQ ID NO:43).

In one example, the hepcidin analogue or peptide is: Isovaleric acid-DTHFPCIKFGPRSKGWVC(K(PEG8))-NH₂ (SEQ ID NO:44).

In one example, the hepcidin analogue or peptide is: Isovaleric acid-DTHFPCIKF(K(isoGlu-Palm))PRSKGCK-NH₂ (SEQ ID NO:46).

In one example, the hepcidin analogue or peptide is: Isovaleric acid-DTHFPCIKFEP(K(isoGlu-Palm))SKGCK-NH₂ (SEQ ID NO:47).

In one example, the hepcidin analogue or peptide is: Isovaleric acid-DTHFPCIKFEPRS(K(isoGlu-Palm))GCK-NH₂(SEQ ID NO:48).

In one example, the hepcidin analogue or peptide is: Isovaleric acid-DTHFPCI(K(Dapa-Palm))FEPRSKGCK-NH₂ (SEQ ID NO: 50).

In one example, the hepcidin analogue or peptide is: Isovaleric acid-DTHFPCIKFEP(K(Dapa-Palm))SKGCK-NH₂ (SEQ ID NO:24)

In one example, the hepcidin analogue or peptide is selected from the group consisting of:
(a) 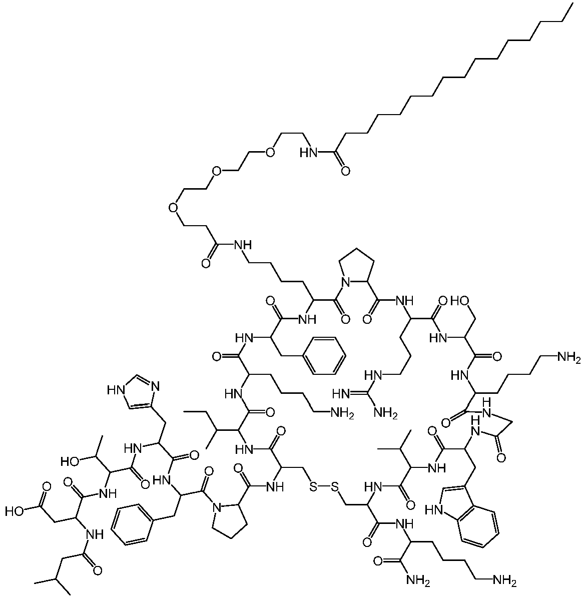 Isovaleric acid-DTHFPCIKF(K(PEG3-Palm))PRSKGWVCK-NH₂ (SEQ ID NO:40);
(b) 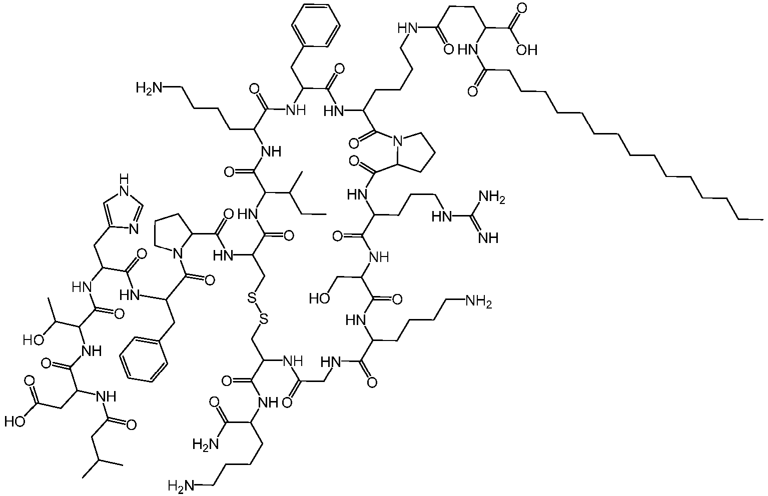 Isovaleric acid-DTHFPCIKF(K(isoGlu-Palm))PRSKGCK-NH₂ (SEQ ID NO:46);
(c) 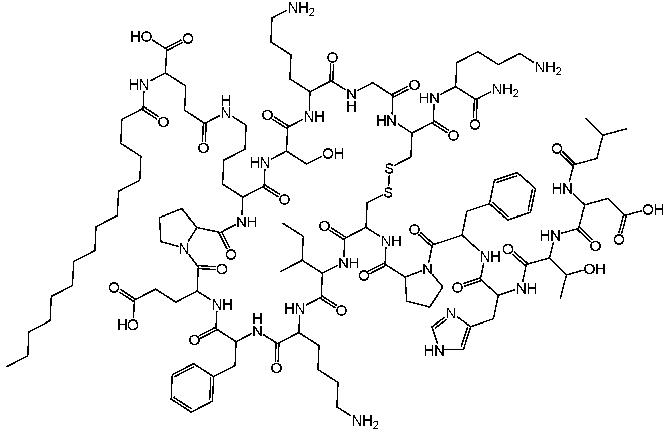 Isovaleric acid-DTHFPCIKFEP(K(isoGlu-Palm))SKGCK-NH₂ (SEQ ID NO:47); and
(d) 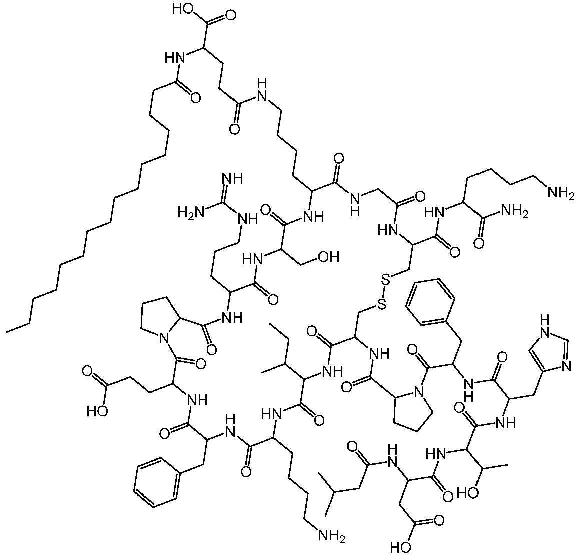 Isovaleric acid-DTHFPCIKFEPRS(K(isoGlu-Palm))GCK-NH₂(SEQ ID NO:48), and
   pharmaceutically acceptable salts of any of the afore-mentioned, wherein the amino acids are L-amino acids.

**In** some embodiments, the hepcidin analogues of the present invention and as further described herein are active in a dimer conformation, in particular when free cysteine residues are present in the peptide. In certain embodiments, this occurs either as a synthesized dimer or, in particular, when a free cysteine monomer peptide is present and under oxidizing conditions, dimerizes. In some embodiments, the dimer is a homodimer. In other embodiments, the dimer is a heterodimer.

In certain embodiments, a hepcidin analogue dimer of the present invention is a peptide dimer comprising two hepcidin analogue peptide monomers of the invention.

Also disclosed herein is a polypeptide comprising an amino acid sequence set forth herein, or having any amino acid sequence with at least 85%, at least 90%, at least 92%, at least 94%, or at least 95% identity to any of these amino acid sequences. Also disclosed herein is a dimer comprising two polypeptides, each comprising an amino acid sequence set forth herein, or having any amino acid sequence with at least 85%, at least 90%, at least 92%, at least 94%, or at least 95% identity to any of these amino acid sequences.

In particular embodiments, the monomer subunits may be dimerized by a disulfide bridge between two cysteine residues, one in each peptide monomer subunit, or they may be dimerized by another suitable linker moiety, including those described herein. Some of the monomer subunits are shown having C- and/or N-termini that both comprise free amine. Thus, to produce a peptide dimer inhibitor, the monomer subunit may be modified to eliminate either the C- or N-terminal free amine, thereby permitting dimerization at the remaining free amine. Further, in some instances, a terminal end of one or more monomer subunits is acylated with an acylating organic compound selected from the group consisting of 2-me-Trifluorobutyl, Trifluoropentyl, Acetyl, Octonyl, Butyl, Pentyl, Hexyl, Palmityl, Trifluoromethyl butyric, cyclopentane carboxylic, cyclopropylacetic, 4-fluorobenzoic, 4-fluorophenyl acetic, 3-Phenylpropionic, tetrahedro-2H-pyran-4carboxylic, succinic acid, and glutaric acid. In some instances, monomer subunits comprise both a free carboxy terminal and a free amino terminal, whereby a user may selectively modify the subunit to achieve dimerization at a desired terminus. One having skill in the art will, therefore, appreciate that the monomer subunits of the instant invention may be selectively modified to achieve a single, specific amine for a desired dimerization.

It is further understood that the C-terminal residues of the monomer subunits disclosed herein may be amides, unless otherwise indicated. Further, it is understood that, in certain embodiments, dimerization at the C-terminus is facilitated by using a suitable amino acid with a side chain having amine functionality, as is generally understood in the art. Regarding the N-terminal residues, it is generally understood that dimerization may be achieved through the free amine of the terminal residue, or may be achieved by using a suitable amino acid side chain having a free amine, as is generally understood in the art.

Moreover, it is understood that the side chains of one or more internal residue comprised in the hepcidin analogue peptide monomers of the present invention can be utilized for the purpose of dimerization. In such embodiments, the side chain is in some embodiments a suitable natural amino acid (e.g., Lys), or alternatively it is an unnatural amino acid comprising a side chain suitable for conjugation, e.g., to a suitable linker moiety, as defined herein.

The linker moieties connecting monomer subunits may include any structure, length, and/or size that is compatible with the teachings herein. For example, a linker moiety may be selected from the non-limiting group consisting of: cysteine, lysine, DIG, PEG4, PEG4-biotin, PEG13, PEG25, PEG1K, PEG2K, PEG3.4K, PEG4K, PEG5K, IDA, IDA-Palm, ADA, Boc-IDA, Glutaric acid, Isophthalic acid, 1,3-phenylenediacetic acid, 1,4-phenylenediacetic acid, 1,2-phenylenediacetic acid, Triazine, Boc-Triazine, IDA-biotin, PEG4-Biotin, AADA, suitable aliphatics, aromatics, heteroaromatics, and polyethylene glycol based linkers having a molecular weight from approximately 400Da to approximately 40,000Da. Non-limiting examples of suitable linker moieties are provided in Table 2. Any of these linker moieties may alternatively link a half-life extension moiety to a hepcidin analogue.

**Table 2. Illustrative Linker Moieties**

| Abbreviation | Description | Structure |
|---|---|---|
| DIG | DIGlycolic acid | 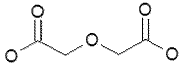 |
| PEG4 | Bifunctional PEG linker with 4 PolyEthylene Glycol units | 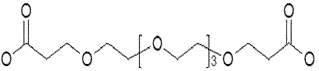 |
| PEG13 | Bifunctional PEG linker with 13 PolyEthylene Glycol units | 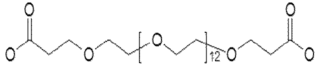 |
| PEG25 | Bifunctional PEG linker with 25 PolyEthylene Glycol units | 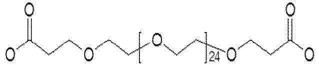 |
| PEG1K | Bifunctional PEG linker with PolyEthylene Glycol Mol wt of 1000Da | |
| PEG2K | Bifunctional PEG linker with PolyEthylene Glycol Mol wt of 2000Da | |
| PEG3.4K | Bifunctional PEG linker with PolyEthylene Glycol Mol wt of 3400Da | |
| PEG5K | Bifunctional PEG linker with PolyEthylene Glycol Mol wt of 5000Da | |
| DIG | Diglycolic acid | 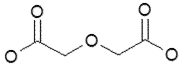 |
| β-Ala-IDA | β-Ala-Iminodiacetic acid | 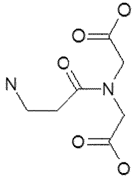 |
| Boc- β-Ala-IDA | Boc-β-Ala-Iminodiacetic acid | 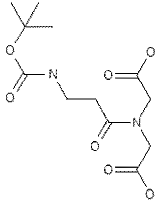 |
| Ac- β-Ala-IDA | Ac-β-Ala-Iminodiacetic acid | 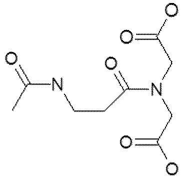 |
| Palm-β-Ala-IDA- | Palmityl-β-Ala-Iminodiacetic acid | 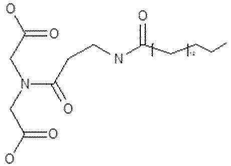 |
| GTA | Glutaric acid | 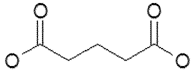 |
| PMA | Pemilic acid | 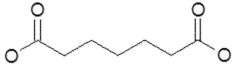 |
| AZA | Azelaic acid | 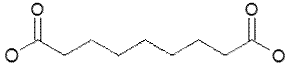 |
| DDA | Dodecanedioic acid | 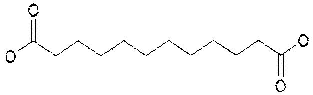 |
| IPA | Isopthalic acid | 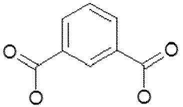 |
| 1,3-PDA | 1,3- Phenylenediacetic acid | 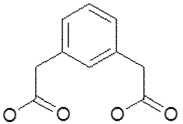 |
| 1,4-PDA | *1*,*4*- Phenylenediacetic acid | 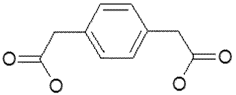 |
| 1,2-PDA | 1,2 - Phenylenediacetic acid | 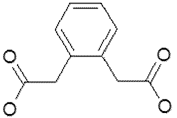 |
| Triazine | Amino propyl Triazine di-acid | 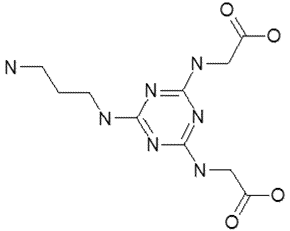 |
| Boc-Triazine | Boc-Triazine di-acid | 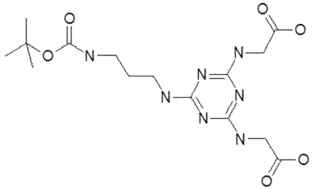 |
| IDA | Iminodiacetic acid | 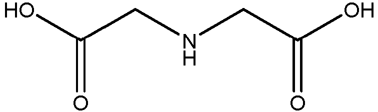 |
| AIDA | n-Acetyl imino acetic acid | 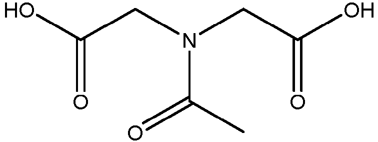 |
| Biotin-β-ala-IDA- | N-Biotin-β -Ala-Iminodiacetic acid | 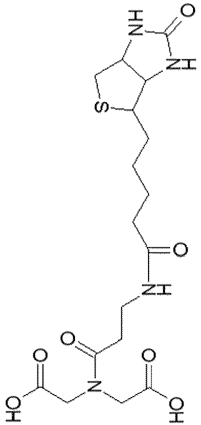 |
| Lys | Lysine | 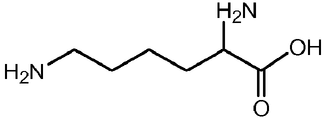 |

One having skill in the art will appreciate that the C- and N-terminal and internal linker moieties disclosed herein are non-limiting examples of suitable linker moieties, and that any suitable linker moiety may be included.

In certain examples of hepcidin analogue peptide dimers, the N-terminus of each peptide monomer subunit is connected by a linker moiety.

In certain examples of hepcidin analogue peptide dimers, the C-terminus of each peptide monomer subunit is connected by a linker moiety.

In certain examples, the side chains of one or more internal amino acid residues (e.g., Lys residues) comprised in each peptide monomer subunit of a hepcidin analogue peptide dimer are connected by a linker moiety.

In certain examples of hepcidin analogue peptide dimers, the C-terminus, the N terminus, or an internal amino acid (e.g., a lysine sidechain) of each peptide monomer subunit is connected by a linker moiety and at least two cysteine or Pen residues of the hepcidin analogue peptide dimers are linked by a disulfide bridge. For example, a peptide dimer may have a general structure shown below. Non-limiting schematic examples of such hepcidin analogues are shown in the following illustration:

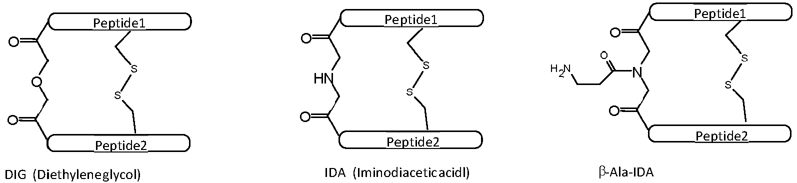

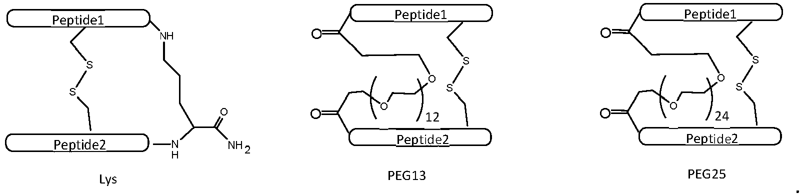

### Peptide Analogue Conjugates

Hepcidin analogues, including both monomers and dimers, may comprise one or more conjugated chemical substituents, such as lipophilic substituents and polymeric moieties, collectively referred to herein as half-life extension moieties. Without wishing to be bound by any particular theory, it is believed that the lipophilic substituent binds to albumin in the bloodstream, thereby shielding the hepcidin analogue from enzymatic degradation, and thus enhancing its half-life. In addition, it is believed that polymeric moieties enhance half-life and reduce clearance in the bloodstream, and in some cases enhance permeability through the epithelium and retention in the lamina propria. Moreover, it is also surmised that these substituents in some cases may enhance permeability through the epithelium and retention in the lamina propria. The skilled person will be well aware of suitable techniques for preparing the compounds employed in the context of the invention. For examples of non-limiting suitable chemistry, see, e.g., WO98/08871, WO00/55184, WO00/55119, Madsen et al (J. Med. Chem. 2007, 50, 6126-32), and Knudsen et al. 2000 (J. Med Chem. 43, 1664-1669).

The side chains of one or more amino acid residues (e.g., Lys residues) in a hepcidin analogue as described herein may be further conjugated (e.g., covalently attached) to a lipophilic substituent or other half-life extension moiety. The lipophilic substituent may be covalently bonded to an atom in the amino acid side chain, or alternatively may be conjugated to the amino acid side chain via one or more spacers or linker moieties. The spacer or linker moiety, when present, may provide spacing between the hepcidin analogue and the lipophilic substituent. For example, the half-life extension moiety may be conjugated to the hepcidin analogue via a linker moiety, which in certain embodiments is a linker moiety shown in Table 2 or disclosed or depicted in any of Tables 2-7.

The lipophilic substituent or half-life extension moiety may comprise a hydrocarbon chain having from 4 to 30 C atoms, for example at least 8 or 12 C atoms, and preferably 24 C atoms or fewer, or 20 C atoms or fewer. The hydrocarbon chain may be linear or branched and may be saturated or unsaturated. The hydrocarbon chain may be substituted with a moiety which forms part of the attachment to the amino acid side chain or the spacer, for example an acyl group, a sulfonyl group, an N atom, an O atom or an S atom. In some examples, the hydrocarbon chain is substituted with an acyl group, and accordingly the hydrocarbon chain may form part of an alkanoyl group, for example palmitoyl, caproyl, lauroyl, myristoyl or stearoyl.

A lipophilic substituent may be conjugated to any amino acid side chain in a hepcidin analogue as described herein. For example, the amino acid side chain may include a carboxy, hydroxyl, thiol, amide or amine group, for forming an ester, a sulphonyl ester, a thioester, an amide or a sulphonamide with the spacer or lipophilic substituent. For example, the lipophilic substituent may be conjugated to Asn, Asp, Glu, Gln, His, Lys, Arg, Ser, Thr, Tyr, Trp, Cys or Dbu, Dpr or Orn. The lipophilic substituent may be conjugated to Lys. An amino acid shown as Lys in any of the formula provided herein may be replaced by, e.g., Dbu, Dpr or Orn where a lipophilic substituent is added.

Alternatively or additionally, the side-chains of one or more amino acid residues in a hepcidin analogue of the invention may be conjugated to a polymeric moiety or other half-life extension moiety, for example, in order to increase solubility and/or half-life *in vivo* (e.g., in plasma) and/or bioavailability. Such modifications are also known to reduce clearance (e.g. renal clearance) of therapeutic proteins and peptides.

As used herein, "Polyethylene glycol" or "PEG" is a polyether compound of general formula H-(O-CH2-CH2)n-OH. PEGs are also known as polyethylene oxides (PEOs) or polyoxyethylenes (POEs), depending on their molecular weight PEO, PEE, or POG, as used herein, refers to an oligomer or polymer of ethylene oxide. The three names are chemically synonymous, but PEG has tended to refer to oligomers and polymers with a molecular mass below 20,000 g/mol, PEO to polymers with a molecular mass above 20,000 g/mol, and POE to a polymer of any molecular mass. PEG and PEO are liquids or low-melting solids, depending on their molecular weights. Throughout this disclosure, the 3 names are used indistinguishably. PEGs are prepared by polymerization of ethylene oxide and are commercially available over a wide range of molecular weights from 300 g/mol to 10,000,000 g/mol. While PEG and PEO with different molecular weights find use in different applications, and have different physical properties (e.g. viscosity) due to chain length effects, their chemical properties are nearly identical. The polymeric moiety is preferably water-soluble (amphiphilic or hydrophilic), non-toxic, and pharmaceutically inert. Suitable polymeric moieties include polyethylene glycols (PEG), homo- or co-polymers of PEG, a monomethyl-substituted polymer of PEG (mPEG), or polyoxyethylene glycerol (POG). See, for example, Int. J. Hematology 68:1 (1998); Bioconjugate Chem. 6:150 (1995); and Crit. Rev. Therap. Drug Carrier Sys. 9:249 (1992). Also encompassed are PEGs that are prepared for purpose of half-life extension, for example, mono-activated, alkoxy-terminated polyalkylene oxides (POA's) such as mono-methoxy-terminated polyethyelene glycols (mPEG's); bis activated polyethylene oxides (glycols) or other PEG derivatives are also contemplated. Suitable polymers will vary substantially by weights ranging from about 200 to about 40,000 are usually selected for the purposes of the present invention. In certain embodiments, PEGs having molecular weights from 200 to 2,000 daltons or from 200 to 500 daltons are used. Different forms of PEG may also be used, depending on the initiator used for the polymerization process, e.g., a common initiator is a monofunctional methyl ether PEG, or methoxypoly(ethylene glycol), abbreviated mPEG. Other suitable initiators are known in the art and are suitable for use in the present invention.

Lower-molecular-weight PEGs are also available as pure oligomers, referred to as monodisperse, uniform, or discrete. These are used in certain embodiments of the present invention.

PEGs are also available with different geometries: branched PEGs have three to ten PEG chains emanating from a central core group; star PEGs have 10 to 100 PEG chains emanating from a central core group; and comb PEGs have multiple PEG chains normally grafted onto a polymer backbone. PEGs can also be linear. The numbers that are often included in the names of PEGs indicate their average molecular weights (e.g. a PEG with n = 9 would have an average molecular weight of approximately 400 daltons, and would be labeled PEG 400.

As used herein, "PEGylation" is the act of coupling (e.g., covalently) a PEG structure to the hepcidin analogue of the invention, which is in certain embodiments referred to as a "PEGylated hepcidin analogue". In certain embodiments, the PEG of the PEGylated side chain is a PEG with a molecular weight from about 200 to about 40,000. In certain embodiments, the PEG portion of the conjugated half-life extension moiety is PEG3, PEG4, PEGS, PEG6, PEG7, PEG8, PEG9, PEG10, or PEG11. In particular embodiments, it is PEG11. In certain embodiments, the PEG of a PEGylated spacer is PEG3 or PEG8. In some embodiments, a spacer is PEGylated. In certain embodiments, the PEG of a PEGylated spacer is PEG3, PEG4, PEG5, PEG6, PEG7, PEG8, PEG9, PEG10, or PEG11. In certain embodiments, the PEG of a PEGylated spacer is PEG3 or PEG8.

A hepcidin analogue peptide (or a dimer thereof) may be conjugated with a PEG that is attached covalently, e.g., through an amide, a thiol, via click chemistry, or via any other suitable means known in the art. In particular embodiments PEG is attached through an amide bond and, as such, certain PEG derivatives used will be appropriately functionalized. For example, PEG11, which is O-(2-aminoethyl)-O'-(2-carboxyethyl)-undecaethyleneglycol, has both an amine and carboxylic acid for attachment to a peptide. PEG25 contains a diacid and 25 glycol moieties.

Other suitable polymeric moieties include poly-amino acids such as poly-lysine, poly-aspartic acid and poly-glutamic acid (see for example Gombotz, et al. (1995), Bioconjugate Chem., vol. 6: 332-351; Hudecz, et al. (1992), Bioconjugate Chem., vol. 3, 49-57 and Tsukada, et al. (1984), J. Natl. Cancer Inst., vol. 73, : 721-729. The polymeric moiety may be straight-chain or branched, and may have a molecular weight of 500-40,000 Da, for example 500-10,000 Da, 1000-5000 Da, 10,000-20,000 Da, or 20,000-40,000 Da.

A hepcidin analogue may comprise two or more such polymeric moieties, in which case the total molecular weight of all such moieties will generally fall within the ranges provided above.

The polymeric moiety may be coupled (by covalent linkage) to an amino, carboxyl or thiol group of an amino acid side chain. Certain examples are the thiol group of Cys residues and the epsilon amino group of Lys residues, and the carboxyl groups of Asp and Glu residues may also be involved.

The skilled worker will be well aware of suitable techniques which can be used to perform the coupling reaction. For example, a PEG moiety bearing a methoxy group can be coupled to a Cys thiol group by a maleimido linkage using reagents commercially available from Nektar Therapeutics AL. See also WO 2008/101017, and the references cited above, for details of suitable chemistry. A maleimide-functionalised PEG may also be conjugated to the side-chain sulfhydryl group of a Cys residue.

As used herein, disulfide bond oxidation can occur within a single step or is a two-step process. As used herein, for a single oxidation step, the trityl protecting group is often employed during assembly, allowing deprotection during cleavage, followed by solution oxidation. When a second disulfide bond is required, one has the option of native or selective oxidation. For selective oxidation requiring orthogonal protecting groups, Acm and Trityl is used as the protecting groups for cysteine. Cleavage results in the removal of one protecting pair of cysteine allowing oxidation of this pair. The second oxidative deprotection step of the cysteine protected Acm group is then performed. For native oxidation, the trityl protecting group is used for all cysteines, allowing for natural folding of the peptide.

A skilled worker will be well aware of suitable techniques which can be used to perform the oxidation step.

In some examples, a hepcidin analogue may comprise a half-life extension moiety, which may be selected from but is not limited to the following: Ahx-Palm, PEG2-Palm, PEG11-Palm, isoGlu-Palm, dapa-Palm, isoGlu-Lauric acid, isoGlu-Mysteric acid, and isoGlu-Isovaleric acid.

For example, the half-life extension moiety may have the structure shown below, wherein n=0 to 24 or n=14 to 24:

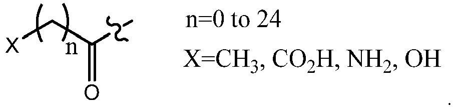

A hepcidin analogue as described herein may comprise a conjugated half-life extension moiety shown in Table 3.

**Table 3. Illustrative Half-Life Extension Moieties**

| # | **Conjugates** |
|---|---|
| C1 | 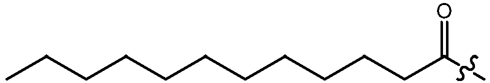 C12 (Lauric acid) |
| C2 | 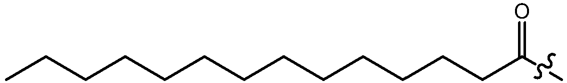 C14 (Mysteric acid) |
| C3 | 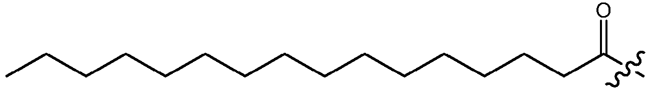 C16 (Palm or Palmitic acid) |
| C4 | 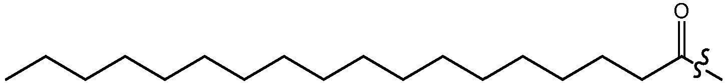 C18 (Stearic acid) |
| C5 | 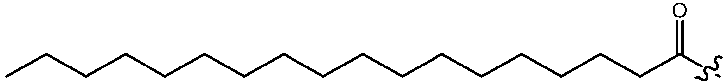 C20 |
| C6 | 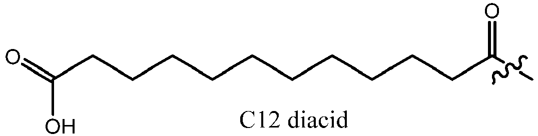 |
| C7 | 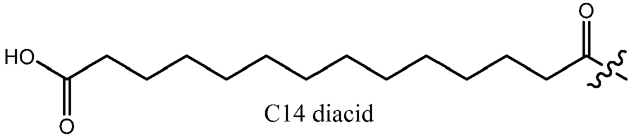 |
| C8 | 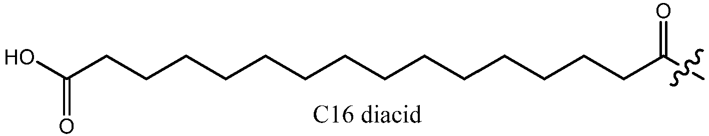 |
| C9 | 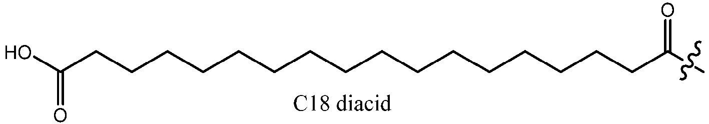 |
| C10 | 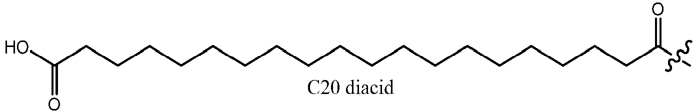 |
| C11 | 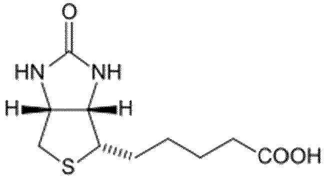 Biotin |
| C12 | 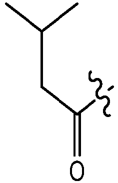 Isovaleric acid |

A half-life extension moiety may be conjugated directly to a hepcidin analogue, or may be conjugated to a hepcidin analogue peptide via a linker moiety, e.g., any of those depicted in Tables 2 or 4.

**Table 4. Illustrative Linker Moieties**

| # | **Linker Moiety** |
|---|---|
| L1 | 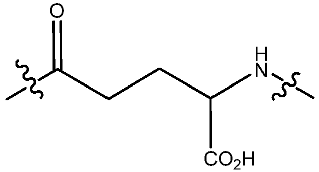 IsoGlu |
| L2 | 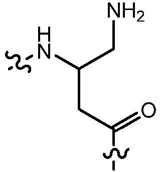 Dapa |
| L3 | 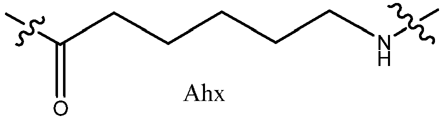 |
| L4 | Lipdic based linkers: |
| | 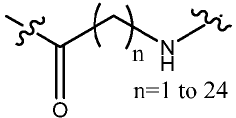 |
| L5 | 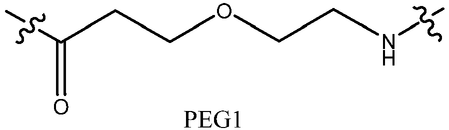 |
| L6 | 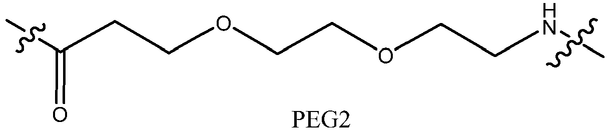 |
| L7 | 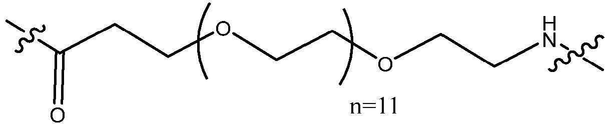 PEG11 (40 atoms) |
| L8 | 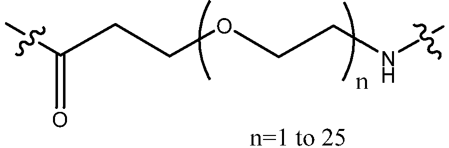 PEG based linkers |
| L9 | 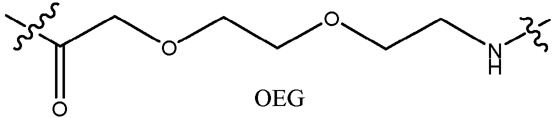 |
| L10 | 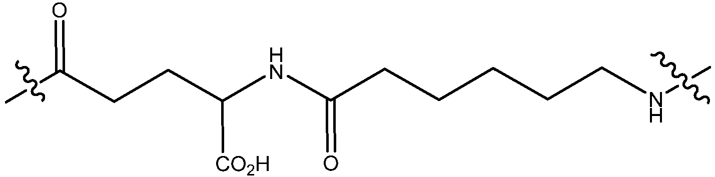 IsoGlu-Ahx |
| L11 | 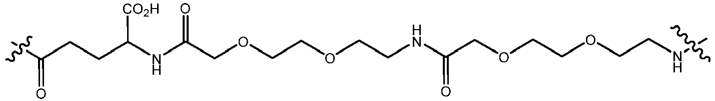 IsoGlu-OEG-OEG |
| L12 | 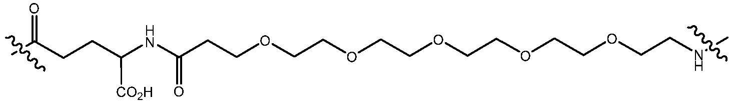 IsoGlu-PEG5 |
| L13 | 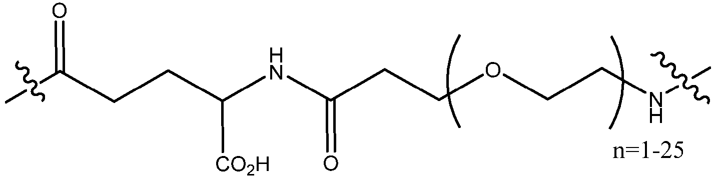 IsoGlu-PEGn |
| L14 | 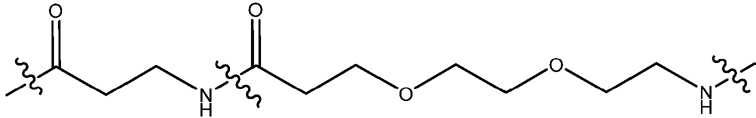 βAla-PEG2 |
| L15 | 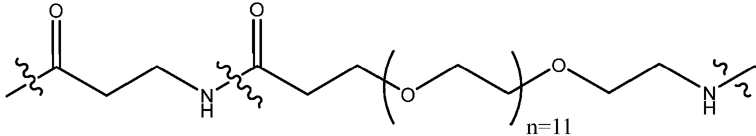 βAla-PEG11 (40 atoms) |

With reference to linker structures shown in Table 4, reference to n=1 to 24 or n= 1 to 25, or the like, (e.g., in L4, L8 or L13) indicates that n may be any integer within the recited range. For example, for L4 shown in Table 4, n could be 1, 2, 3, etc., wherein when n=5, L4 has the structure shown in L3 (Ahx).

Ahepcidin analogue as described herein may comprise any of the linker moieties shown in Table 4 and any of the half-life extension moieties shown in Table 3, including any of the following combinations shown in Table 5.

**Table 5. Illustrative Combinations of Linkers and Half-Life Extension Moieties in Hepcidin Analogues**

| Linker | Half-Life Extension Moiety | | Linker | Half-Life Extension Moiety | | Linker | Half-Life Extension Moiety |
|---|---|---|---|---|---|---|---|
| L1 | C1 | | L1 | C2 | | L1 | C3 |
| L2 | C1 | | L2 | C2 | | L2 | C3 |
| L3 | C1 | | L3 | C2 | | L3 | C3 |
| L4 | C1 | | L4 | C2 | | L4 | C3 |
| L5 | C1 | | L5 | C2 | | L5 | C3 |
| L6 | C1 | | L6 | C2 | | L6 | C3 |
| L7 | C1 | | L7 | C2 | | L7 | C3 |
| L8 | C1 | | L8 | C2 | | L8 | C3 |
| L9 | C1 | | L9 | C2 | | L9 | C3 |
| L10 | C1 | | L10 | C2 | | L10 | C3 |
| L11 | C1 | | L11 | C2 | | L11 | C3 |
| L12 | C1 | | L12 | C2 | | L12 | C3 |
| L13 | C1 | | L13 | C2 | | L13 | C3 |
| L14 | C1 | | L14 | C2 | | L14 | C3 |
| L15 | C1 | | L15 | C2 | | L15 | C3 |
| L1 | C4 | | L1 | C5 | | L1 | C6 |
| L2 | C4 | | L2 | C5 | | L2 | C6 |
| L3 | C4 | | L3 | C5 | | L3 | C6 |
| L4 | C4 | | L4 | C5 | | L4 | C6 |
| L5 | C4 | | L5 | C5 | | L5 | C6 |
| L6 | C4 | | L6 | C5 | | L6 | C6 |
| L7 | C4 | | L7 | C5 | | L7 | C6 |
| L8 | C4 | | L8 | C5 | | L8 | C6 |
| L9 | C4 | | L9 | C5 | | L9 | C6 |
| L10 | C4 | | L10 | C5 | | L10 | C6 |
| L11 | C4 | | L11 | C5 | | L11 | C6 |
| L12 | C4 | | L12 | C5 | | L12 | C6 |
| L13 | C4 | | L13 | C5 | | L13 | C6 |
| L14 | C4 | | L14 | C5 | | L14 | C6 |
| L15 | C4 | | L15 | C5 | | L15 | C6 |
| L1 | C7 | | L1 | C8 | | L1 | C9 |
| L2 | C7 | | L2 | C8 | | L2 | C9 |
| L3 | C7 | | L3 | C8 | | L3 | C9 |
| L4 | C7 | | L4 | C8 | | L4 | C9 |
| L5 | C7 | | L5 | C8 | | L5 | C9 |
| L6 | C7 | | L6 | C8 | | L6 | C9 |
| L7 | C7 | | L7 | C8 | | L7 | C9 |
| L8 | C7 | | L8 | C8 | | L8 | C9 |
| L9 | C7 | | L9 | C8 | | L9 | C9 |
| L10 | C7 | | L10 | C8 | | L10 | C9 |
| L11 | C7 | | L11 | C8 | | L11 | C9 |
| L12 | C7 | | L12 | C8 | | L12 | C9 |
| L13 | C7 | | L13 | C8 | | L13 | C9 |
| L14 | C7 | | L14 | C8 | | L14 | C9 |
| L15 | C7 | | L15 | C8 | | L15 | C9 |
| L1 | C10 | | L1 | C11 | | L1 | C12 |
| L2 | C10 | | L2 | C11 | | L2 | C12 |
| L3 | C10 | | L3 | C11 | | L3 | C12 |
| L4 | C10 | | L4 | C11 | | L4 | C12 |
| L5 | C10 | | L5 | C11 | | L5 | C12 |
| L6 | C10 | | L6 | C11 | | L6 | C12 |
| L7 | C10 | | L7 | C11 | | L7 | C12 |
| L8 | C10 | | L8 | C11 | | L8 | C12 |
| L9 | C10 | | L9 | C11 | | L9 | C12 |
| L10 | C10 | | L10 | C11 | | L10 | C12 |
| L11 | C10 | | L11 | C11 | | L11 | C12 |
| L12 | C10 | | L12 | C11 | | L12 | C12 |
| L13 | C10 | | L13 | C11 | | L13 | C12 |
| L14 | C10 | | L14 | C11 | | L14 | C12 |
| L15 | C10 | | L15 | C11 | | L15 | C12 |

A hepcidin analogue may comprise two or more linkers. The two or more linkers may be concatamerized, i.e., bound to each other. Also described herein are polynucleotides that encode a polypeptide having a peptide sequence present in any of the hepcidin analogues described herein.

Also described herein are vectors, e.g., expression vectors, comprising a polynucleotide as discussed above.

### Methods of Treatment

Polycythemia vera (PV) is a chronic, progressive trilineage clonal disorder signified by increased myeloid, erythroid, and megakaryocytic cell proliferation/accumulation and is characterized by the World Health Organization (WHO) as a myeloproliferative neoplasm (Arber et al., Blood, 2016,127(20):2391-405). Diagnosis is defined by two criteria; the first being increased red blood cell mass, bone marrow biopsy showing trilineage hypercellularity and presence of JAK2V617F or JAK2 exon 12 mutations and the second criteria incorporates polycythemia, bone marrow biopsy confirmation and subnormal serum erythropoietin levels (Arber et al., Blood, 2016,127(20):2391-405).

During red blood cell production in normocythemic individuals, erythropoiesis is regulated by erythropoietin which relies on JAK2 however when JAK2 is constitutively activated erythropoietin-independent red blood cell production leads to polycythemia. About 95% of PV patients possess the JAK2V617F mutation (Rampal et a., Blood. 2014, 123(22):e123-33,). PV may progress to myelofibrosis or undergo leukemic transformation. Polycythemia in PV men is characterized by Hgb >16.5 g/dL or Hct >49% and a Hgb >16.0 g/dL or Hct >48% in women. A hematocrit above 44% in PV patients is associated with a steep increase in the number of thromboembolic complications suffered (Pearson et al., Lancet. 1978;2:1219-1221). A bone marrow biopsy is taken to confirm PV, from essential thrombocythemia, and must show hypercellularity for age with trilineage growth.

An estimated 148,000 people in the United States are living with PV with a median age at diagnosis of 61 years (Stein et al., J Clin Oncol. 2015 Nov 20;33(33):3953-60). Polycythemia symptoms, related to blood hyperviscosity include fatigue, bone pain, headaches, lightheadedness, visual disturbances, atypical chest pain, pruritus, erythromelalgia, and paresthesia (Tefferi et al., Blood Cancer J. 2018, 8(1):3). Clinical features include splenomegaly, thrombotic and bleeding complications, and risk of leukemic transformation.

PV is classified into two risk categories which define the subsequent treatment regimen: high-risk (age >60 years and history of thrombosis) and low-risk (age <60 years in age and absence of a thrombosis history) (Tefferi & Barbui, Am J Hematol. 2017 Jan;92(1):94-108). In the US, an alternative classification may be used: high-risk (age >60 years) and low-risk (age <60 years in age). All PV patients undergo therapeutic phlebotomy to reduce hematocrit and once-daily aspirin (81 mg) in order to prevent thrombohemorrhagic complications. Prior to the incorporation of therapeutic phlebotomy, the median survival for untreated PV was less than 2 years, with death attributed to thrombotic complications (Tefferi et al., 2018). With current treatments, the median survival, from time of diagnosis, in patients younger than 60 years is 24 years compared to 14 years in those aged greater than 60 years. The hematocrit target for therapeutic phlebotomy is <45% for men and <42% for women and <36% during pregnancy (Streiff et al., Blood. 2002, 99 (4): 1144-9), corresponding to Hgb levels of 15, 14, and 12 g/dL, respectively. The goal of therapeutic phlebotomy is to create a chronic state of iron deficiency thereby decreasing red blood cell production. Moreover, iron-deficient red blood cells are less viscous than normocythemic blood due to their reduced size. Fortunately, following exercise evaluation confirmed chronically iron-deficient PV patients do not experience the aerobic deficits associated with iron deficiency anticipated with their normocythemic counterparts (Rector et al., Medicine (Baltimore). 1982 Nov;61(6):382-9).

Cytoreductive therapy is used for high-risk patients and those low-risk patients who cannot tolerate phlebotomy, exhibit progressive splenomegaly, or have platelet counts >1500x10⁹/L or progressive leukocytosis. Cytoreductive agents include but are not limited to, hydroxyurea, interferon alfa, ruxolitinib (Jakafi^{®}), and busulphan. In the US, hydroxyurea is the first-line treatment for PV patients who are older than 40 years as it effectively improves myelosuppression and reduces the risk for thrombosis compared with the use of phlebotomy alone. Concerns about hydroxyurea long-term risk for secondary leukemia however are warranted. After a median follow-up of more than 8 years, the Polycythemia Vera Study Group reported that 5.4% of evaluated patients with PV developed leukemia after receiving hydroxyurea, compared with 1.5% of those treated with phlebotomy alone (Fruchtman et al., Semin Hematol. 1997, 34:17-23). Patients who are either intolerant of, or resistant to, hydroxyurea can be managed with pegylated IFN-α or busulphan. IFN-α in preferred in patients who are younger than 65 years of age and busulphan in older individuals.

Approximately one in four patients with PV are considered uncontrolled as they have an inadequate response to or are intolerant of hydroxyurea. Jakafi^{®} (ruxolitinib) is a JAK1/JAK2 inhibitor approved by the U.S. Food and Drug Administration for PV patients who are resistant to or intolerant of hydroxyurea. The approval was based on the Phase 3 study, termed RESPONSE-Randomized, open-label, multicenter phase 3 study of Efficacy and Safety in Polycythemia vera subjects who are resistant to or intolerant of hydroxyurea: JAK inhibitor INC424 tablets versus best available care (BAT). The trial evaluated patients with phlebotomy-dependent PV and splenomegaly who were either intolerant of, or resistant to, hydroxyurea and were randomized to ruxolitinib (n=110) or best available (n=112). The composite primary end point included hematocrit control (<45%) and spleen reduction (≥ 35% reduction) at week 32. After week 32, patients who were randomized to the best available therapy could cross over to ruxolitinib. At 32 weeks, 77% of patients randomized to ruxolitinib met at least 1 component of the primary end point, but only 1% of the patients receiving BAT achieved the primary end point. The majority (91%) of ruxolitinib-treated patients who achieved the primary end point had a confirmed response at week 48, and the probability of maintaining a primary response for 1 year was 94%. The rate of thromboembolic events was lower in the ruxolitinib group, with only 1 event (portal vein thrombosis) reported through week 32, compared with 6 events among patients receiving BAT. The RESPONSE trial investigators concluded that in patients with PV who had an inadequate response to, or were intolerant of, hydroxyurea, ruxolitinib was superior to BAT in controlling hematocrit without phlebotomy, normalizing blood cell counts, reducing spleen volume, and improving PV-associated symptoms, including pruritus, fatigue, and night sweats.

Patients in the US receive approximately 8 phlebotomies per year with the procedure causing distress, bother, and inconvenience (Boccia et al., Blood, 2017, 130(Suppl 1), 5271). A considerable amount of time is spent undergoing phlebotomies encompassing approximately half a work day per procedure. Moreover, a lot of redox/metabolic cycling occurs (need more around this) so if the ultimate goal of phlebotomy therapy in polycythemia vera is to achieve a state of chronic iron deficiency that limits erythropoiesis then this can be theoretically obtained with hepcidin/hepcidin mimetics.

Hepcidin, a 25-amino-acid peptide, governs systemic iron homeostasis and is generated by the liver in response to plasma iron concentration and iron stores. Hepcidin inhibits the cellular iron exporter ferroportin (FPN-1), which is expressed on the surfaces of cells that are involved in iron absorption, recycling, and storage. Hepcidin modulates systemic iron restriction and exogenous administration of an exogenous hepcidin mimetic decreased Hgb concentrations and splenomegaly in a murine PV model (Casu et al, Blood. 2016;128(2):265-276).

When JAK2 is constitutively activated erythropoietin-independent red blood cell production is triggered leading to polycythemia. An approach to prevent the effect of mutated Jak2 is to induce iron restriction with hepcidin or hepcidin mimetic peptides. Low iron level inhibits erythropoietin signaling at a point that is downstream of Jak2 thus providing an override signal. When hepcidin mimetic peptides are administered to transgenic mice that express a human Jak2 gene carrying the polycythemia causing mutation the increased red cell production and hematocrit that is characteristic of polycythemia vera is reversed back to the normal range. Reference (Casu et al Blood. 2016; 128(2):265-276).

The present invention provides a hepcidin analogue or a pharmaceutically acceptable salt or solvate thereof, for use in a method of treating polycythemia vera in a human subject in need thereof, wherein the hepcidin analogue is: 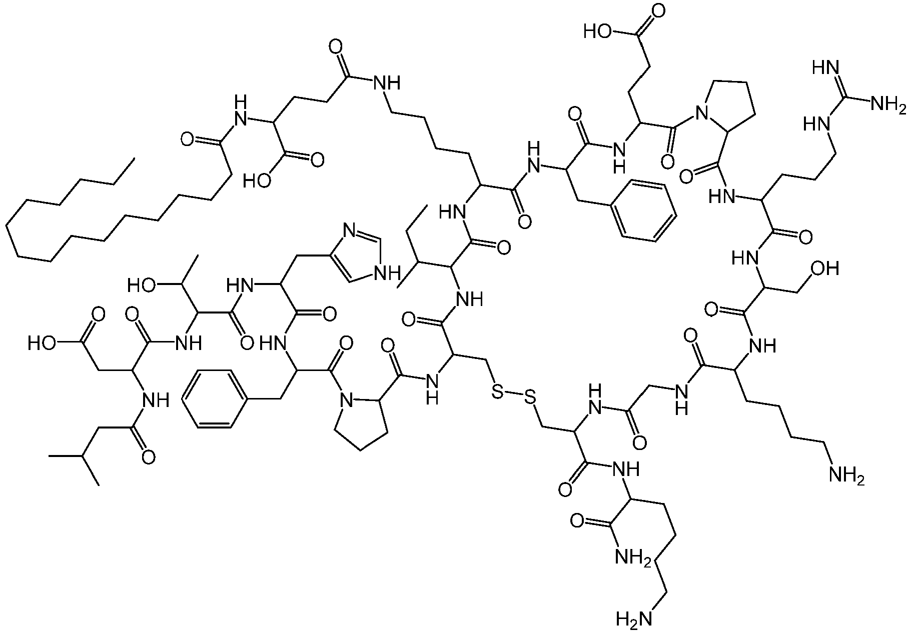 Isovaleric acid-DTHFPCI(K(isoGlu-Palm))FEPRSKGCK-NH2 (SEQ ID NO:45);
wherein the amino acids are L-amino acids; and wherein the hepcidin analogue or pharmaceutically acceptable salt thereof is administered via subcutaneous inj ection; the subject is administered from about 20 mg to about 100 mg of the hepcidin analogue or pharmaceutically acceptable salt thereof; and the hepcidin analogue or pharmaceutically acceptable salt thereof is provided to the subject about twice a week, about once a week, or about once every two weeks. In some embodiments, the hepcidin analogue that is administered to the subject is present in a composition (e.g., a pharmaceutical composition). It is understood that throughout, reference to a hepcidin analogue includes pharmaceutically acceptable salts of such hepcidin analogues.

Also disclosed herein is a method for treating a subject afflicted with a disease or disorder characterized by increased activity or expression of ferroportin, wherein the method comprises administering to the individual a hepcidin analogue or composition of the present invention in an amount sufficient to (partially or fully) bind to and agonize ferroportin in the subject. Also disclosed herein is a method for treating a subject afflicted with a disease or disorder characterized by dysregulated iron metabolism, wherein the method comprises administering to the subject a hepcidin analogue or composition of the present invention.

In certain embodiments, the Polycythemia vera is phlebotomy-requiring Polycythemia vera. In some embodiments, the Polycythemia vera is phlebotomy-requiring Polycythemia vera in a low risk patient. In some embodiments, the subject is a high risk Polycythemia vera patient. In some embodiments, the subject is a low risk Polycythemia vera patient. In some embodiments, the subject is a symptomatic phlebotomy-requiring Polycythemia vera patient. In some embodiments, the subject is a low risk patient with phlebotomy-requiring Polycythemia vera. In some embodiments, the subject is a high risk patient with phlebotomy-requiring Polycythemia vera. In some embodiments, the subject is diagnosed with Polycythemia vera and has received at least three phlebotomies to goal hematocrit <45% in the 24 weeks prior to administration of the pharmaceutical composition to the subject. In some embodiments, the subject is a mammal, e.g., a human.

In accordance with the present invention the subject is administered from about 20 mg to about 100 mg of the hepcidin analogue or pharmaceutically acceptable salt thereof. In particular embodiments the hepcidin analogue or pharmaceutically acceptable salt thereof is provided to the subject at a dosage of about 20 mg to about 50 mg or about 20 mg to about 40 mg, e.g., about 20 mg, about 25 mg, about 30 mg, about 40 mg, about 50 mg, about 60 mg, about 70 mg, or about 80 mg.

Also disclosed herein is a method of treating Polycythemia vera in a human subject in need thereof, comprising administering to the subject an effective amount of subject a hepcidin analogue or pharmaceutically acceptable salt thereof, peptide, or composition disclosed herein, e.g., a peptide having the structure of SEQ ID NO:40, SEQ ID NO:46; SEQ ID NO:47, or SEQ ID NO:48. The Polycythemia vera may be phlebotomy-requiring Polycythemia vera, for example phlebotomy-requiring Polycythemia vera in a low risk patient or a high risk patient. The subject may be a symptomatic phlebotomy-requiring Polycythemia vera patient. The subject may be a low risk patient with phlebotomy-requiring Polycythemia vera. Alternatively, the subject is a high risk patient with phlebotomy-requiring Polycythemia vera. The subject may be diagnosed with Polycythemia vera and has received at least three phlebotomies to goal hematocrit <45% in the 24 weeks prior to administration of the pharmaceutical composition to the subject.

The effective amount may be from about 5 mg to about 200 mg or about 10 mg to about 100 mg, e.g., about 10 mg, about 15 mg, about 20 mg, about 25 mg, about 30 mg, about 40 mg, about 50 mg, about 60 mg, about 70 mg, about 80 mg, about 100 mg, or about 120 mg.

Also disclosed herein is a method of treating a human subject having phlebotomy-requiring Polycythemia vera, comprising subcutaneously administering to the subject an effective amount of a hepcidin analogue disclosed herein, e.g., a peptide having the structure of SEQ ID NO:40, SEQ ID NO:46; SEQ ID NO:47, or SEQ ID NO:48. The effective amount may be from about 10 mg to about 100 mg, e.g., about 10 mg, about 15 mg, about 20 mg, about 25 mg, about 30 mg, about 40 mg, about 50 mg, about 60 mg, about 70 mg, about 80 mg, or about 100 mg, and the hepcidin analog is administered to the subject about twice a week, about once a week, about once every other week, or about once a month. In particular embodiments, the subject is administered about 15 mg, about 20 mg, about 30 mg, about 40 mg, about 50 mg, about 60 mg, about 70 mg, or about 80 mg of the hepcidin analogue or pharmaceutically acceptable salt thereof about once every week. When the hepcidin analogue is administered to a woman, a reduced amount of the hepcidin analogue may be administered, e.g., about 10 mg to about 60 mg, e.g., about 10 mg, about 15 mg, about 20 mg, about 25 mg, about 30 mg, about 40 mg, about 50 mg, or about 60 mg. The hepcidin analogue or pharmaceutically acceptable salt thereof may be administered about once every two weeks or about once a month. The hepcidin analogue or pharmaceutically acceptable salt thereof may be administered multiple times over a period of time, e.g., a time period of at least six months, at least or about one year, at least or about two years, at least or about five years, or for the subject's lifetime.

In some embodiments, the hepcidin analogue or pharmaceutically acceptable salt thereof or peptide is administered in a composition (e.g., a pharmaceutical composition). In accordance with the present invention, the hepcidin analogue or pharmaceutically acceptable salt thereof (or composition) is administered via subcutaneous injection. In some embodiments, the hepcidin analogue or pharmaceutically acceptable salt thereof (or composition) is administered about weekly over a period of time, e.g., as long as needed. A hepcidin analog or peptide (or composition) is administered about every three days, about twice a week, about every four days, about every five days, about weekly, about once every two weeks, about once a month, about once every six weeks, about once every eight weeks, about once every two months, or about once every three months. In some examples, it is administered about once a week or about once every two weeks. In certain examples, it is administered about once a week. Alternatively, it is administered about once every two weeks, about once a month, or about once every two months. In accordance with the present invention, the hepcidin analogue or pharmaceutically acceptable salt thereof is provided to the subject about twice a week, about once a week, or about once every two weeks.

The effective amount of the hepcidin analogue or pharmaceutically acceptable salt thereof may be an amount sufficient to achieve a plasma or serum concentration of the hepcidin analogue or pharmaceutically acceptable salt thereof in the subject from about 5 ng/mL to about 3500 ng/mL, about 100 ng/mL to about 3000 ng/mL, about 5 ng/mL to about 900 ng/mL, or from about 5 ng/mL to about 250 ng/mL, or from about 20 ng/mL to 150 ng/mL. The effective amount optimally maintains patients in a desirable range of hematocrit values defined herein.

Also described herein is a method of treating PV in a subject, comprising providing an effective amount of a hepcidin analogue or pharmaceutically acceptable salt thereof to the subject, wherein the subject is provided with an amount of the hepcidin analogue or pharmaceutically acceptable salt thereof that achieves a plasma or serum concentration of the hepcidin analogue or pharmaceutically acceptable salt thereof of from about 2 ng/mL to about 3500 ng/mL, about 5 ng/mL to about 3500 ng/mL, about 100 ng/mL to about 3000 ng/mL, about 5 ng/mL to about 900 ng/mL, from about 5 ng/mL to about 250 ng/mL, or from about 20 ng/mL to 150 ng/mL. For example, the plasma or serum concentration to be achieved is at least about 25 ng/mL, at least about 50 ng/ mL, at least about 100 ng/mL, at least about 200 ng/mL, at least about 500 ng/mL, at least about 1000 ng/mL, at least about 1500 ng/mL, at least about 2000 ng/mL, at least about 2500 ng/mL, or at least about 3000 ng/mL. The plasma or serum concentration to be achieved may be about 200 ng/mL to about 3200 ng/mL or about 1000 ng/mL to about 3200 ng/mL, or about 1000 ng/mL to about 2000 ng/mL, or about 2000 ng/mL to about 3000 ng/mL, e.g., for Compound A. For example, the plasma or serum concentration to be achieved may be at least 4 ng/mL, at least 5 ng/mL, at least 8 ng/mL, at least 10 ng/mL, at least 12 ng/mL/mL, at least 15 ng/mL, at least 17 ng/mL, or at least 20 ng/mL, e.g., for Compound A. The plasma or serum concentration to be achieved may be at least about 4 ng/mL or at least about 17 ng/mL, e.g., for Compound A. For example, this plasma or serum level is achieved following one administration and maintained until the next administration of the hepcidin analogue, e.g., Compound A or Compound B. This plasma or serum level may be achieved and maintained for at least four days, at least five days, at least six days, or at least one week following administration of the hepcidin analogue, e.g., Compound A or Compound B. The plasma or serum concentration to be achieved may be at least about 20 ng/mL, at least about 30 ng/mL, at least about 50 ng/mL, at least about 100 ng/mL, at least about 150 ng/mL, at least about 200 ng/mL, at least about 500 ng/mL, at least about 1000 ng/mL, at least about 1500 ng/mL, at least about 2000 ng/mL, at least about 2500 ng/mL, or at least about 3000 ng/mL, e.g., for Compound A. Tthe plasma or serum concentration to be achieved may be at least about 50 ng/mL within 20-48 hours following administration, at least about 100 ng/mL within 20-48 hours following administration, at least about 250 ng/mL within 20-48 hours following administration, at least about 400 ng/mL within 20-48 hours following administration, at least about 500 ng/mL within 20-48 hours following administration, at least about 800 ng/mL within 20-48 hours following administration, or at least about 1000 ng/mL within 20-48 hours following administration, e.g., for Compound A.The plasma or serum concentration to be achieved may be about 25 ng/mL to about 1000 ng/mL within 20-48 hours following administration, about 100 ng/mL to about 1000 ng/mL within 20-48 hours following administration, about 200 ng/mL to about 1000 ng/mL within 20-48 hours following administration, about 400 ng/mL to about 850 ng/mL within 20-48 hours following administration, e.g., for Compound A. For example, the plasma or serum concentration to be achieved may be about 25 ng/mL to about 125 ng/mL, or from about 50 ng/mL to about 125 ng/mL, e.g., for Compound B. In certain embodiments, the plasma or serum concentration to be achieved is at least about 25 ng/mL, at least about 50 ng/ mL, or at least about 100 ng/mL, e.g., for Compound B.This plasma or serum concentration may be the maximum plasma or serum concentration following administration of the hepcidin analogue or pharmaceutically acceptable salt thereof. This plasma or serum concentration may be maintained during a period of time following administration of the hepcidin analogue or pharmaceutically acceptable salt thereof, e.g., a period of time of at least two days, at least three days, at least four days, at least five days, at least six days, at least one week, at least 9 days, at least 12 days, or at least two weeks. For example, the plasma or serum concentration achieved may be about 200 ng/mL to about 3200 ng/mL or about 1000 ng/mL to about 3200 ng/mL, or about 1000 ng/mL to about 2000 ng/mL, or about 2000 ng/mL to about 3000 ng/mL, e.g., for Compound A, e.g., for about 4 hours, about 8 hours, about 12 hours, about 24 hours, or about 48 hours. The plasma or serum concentration achieved may be at least about 200 ng/mL, at least about 500 ng/mL, at least about 1000 ng/mL, at least about 1500 ng/mL, at least about 2000 ng/mL, at least about 2500 ng/mL, or at least about 3000 ng/mL, e.g., for Compound A, for at least about 4 hours, about 8 hours, about 12 hours, about 24 hours, about 48 hours, or about 72 hours. The plasma or serum concentration achieved may be about 25 ng/mL to about 125 ng/mL, or from about 50 ng/mL to about 125 ng/mL, e.g., for Compound B, e.g., for about 4 hours, about 8 hours, about 12 hours, about 24 hours, or about 48 hours. The plasma or serum concentration achieved may be at least about 25 ng/mL, at least about 50 ng/ mL, or at least about 100 ng/mL, e.g., for Compound B, e.g., for about 4 hours, about 8 hours, about 12 hours, about 24 hours, or about 48 hours.

In certain embodiments, the subject is also treated with cytoreductive therapy, e.g., hydroxyurea, interferon, or ruxolitinib. When the hepcidin analogue is used in combination with cytoreductive therapy, a reduced amount of the hepcidin analogue may be administered.

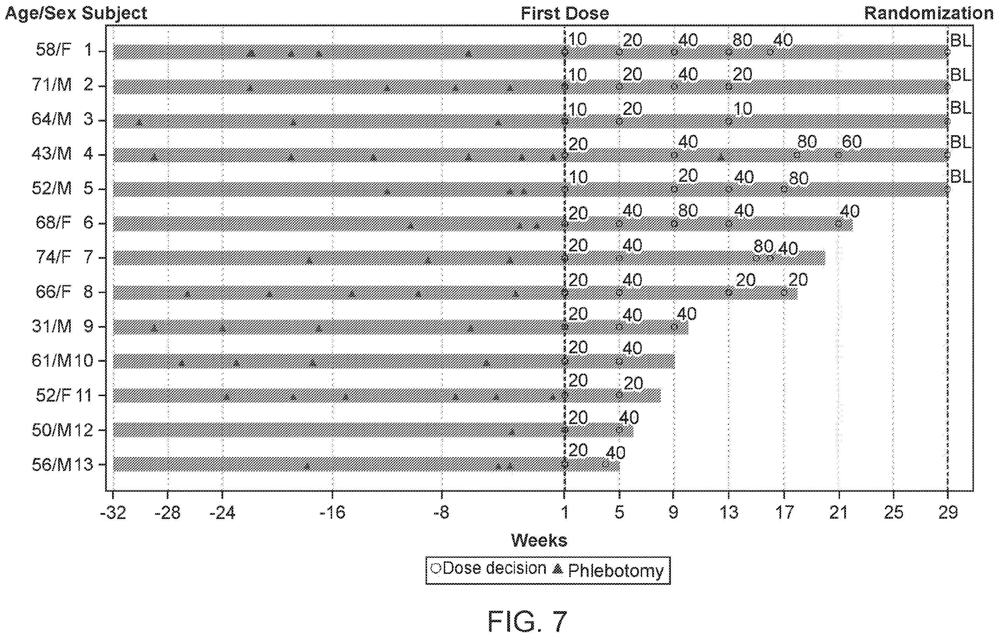

Any of the methods disclosed herein may further comprise a step of determining the subject's hematocrit following administration of the hepcidin analogue. As shown in Figure 7, hematocrit levels can be maintained at a desired level by titrating the amount of hepcidin analogue administered to a patient. Thus, the methods may be used to identify and administer a concentration/dose of hepcidin analogue that is optimally suited to maintain the hematocrit below a target level. The target level for humans may be 45% or lower, e.g., as measured herein. The target level for men may be 45% or lower (e.g., about 37% to about 45%); in some instances, the target threshold level for women (e.g., non-pregnant) is 43% or lower, or 42% or lower (e.g., about 35% to about 42% or 43%); and in some instances, the target level for pregnant women is 36% or lower (e.g., about 30% to about 36%). The method may achieve a hematocrit below 45% (or to a defined level, e.g. 42%) for a patient based on an understanding of the relationship between hepcidin analogue dose and the hematocrit response (i.e., reduction in hematocrit). Thus, the disclosure provides dosing regimens that can maximize the time that the patients' hematocrit is below a desired target level and minimize fluctuations above this target level.

The subject's hematocrit may be determined during a time period between about one day to about 7 days following administration of the hepcidin analogue. If the subject's hematocrit is determined to be above 45%, then a higher dose of the hepcidin analogue may be administered to the subject at the next scheduled treatment, as compared to the dose administered immediately prior to when the hematocrit was determined. If the subject's hematocrit is determined to be above the desired target level for the subject's sex and pregnancy status, then a higher dose of the hepcidin analogue may be administered to the subject at the next scheduled treatment, as compared to the dose administered immediately prior to when the hematocrit was determined. If the subject's hematocrit is determined to be under a threshold level, e.g., under 42%, under 40%, under 37.5%, under 36%, or under 35%, then a lower dose of the hepcidin analogue may be administered to the subject at the next scheduled treatment, as compared to the dose administered immediately prior to when the hematocrit was determined. If the subject's hematocrit is determined to be within an acceptable range, e.g., between 35% and 42%, between 35% and 45%, between 37.5% and 45%, between 40% and 45%, or between 40% and 44%, then the same dose of the hepcidin analogue may be administered to the subject at the next scheduled treatment, as compared to the dose administered immediately prior to when the hematocrit was determined. The acceptable range may vary depending on the subject's sex and pregnancy status. For example, the acceptable range for a male may be about 37% to about 45%, the acceptable range for a non-pregnant female may be about 35% to about 42% or 43%, and the acceptable range for a pregnant female may be about about 30% to about 36%.

The subject's hematocrit level may be determined multiple times over the course of treatment to monitor the effectiveness of the administered dosage, and the dosage altered as needed to maintain the subject's hematocrit within a target range, e.g., 30% to 35%, 35%-41%, 35% to 45%, 37.5% to 45%, 40% to 45%, or 40% to 43%. For example, the subject's hematocrit level may be determined about every 2 weeks, about every three weeks, about every four weeks, or about every eight weeks over the course of treatment. In certain embodiments, it may be determined about every four weeks over the course of treatment. In particular embodiments, the target range is the acceptable range for the subject's sex and pregnancy status. In certain embodiments, the amount of the hepcidin analogue or pharmaceutically acceptable salt thereof administered to the subject may be maintained or adjusted, wherein the amount is increased if the subject's determined hematocrit is greater than 45%, wherein the amount is descreased if the subject's determined hematocrit is less than either 37.5% or 40%, and maintaining the amount if the subject's determined hematocrit is between 37.5% and 45% or between 40% and 44%.

Also described herein is a method for treating PV, comprising subcutaneously administering to a patient diagnosed with PV a hepcidin analogue, e.g., Compound A, at a dosage of between about 10 mg to about 80 mg, about once a week over a period of time of at least seven weeks, wherein the method results in the subject not needing or having a therapeutic phlebotomy during the seven weeks. The dosage may be e.g. about 10 mg, about 15 mg, about 20 mg, about 25 mg, about 30 mg, about 40 mg, about 50 mg, about 60 mg, about 70 mg, or about 80 mg. The subject may have received one or more therapeutic phlembotomy before treatment with the hepcidin analogue, for example within eight weeks before treatment with the hepcidin analogue. The subject may be a male, and his hematocrit level is maintained at <45% for the at least seven weeks. Alternatively, the subject may be a non-pregnant female, and her hematocrit level is maintained at <42% or <43% for the at least seven weeks. Alternatively, the subject may be a pregnant female, and her hematocrit level is maintained at <36% for the at least seven weeks. The hematocrit level may be maintained for at least least eight weeks, at least 12 weeks, at least 16 weeks, at least 6 months, at least one year, or at least two years following the first administration of the hepcidin analogue, and during treatment.

The subject's hematocrit level may be measured one or more times during the seven weeks, and the next weekly dose is increased if the subject's hematocrit level is above an acceptable range, or decreased if the subject's hematocrit level is below an acceptable range. For example, in a male subject the acceptable range may be about 37% to about 45%. The increased or reduced dosage may also be between about 10 mg to about 80 mg. The dosage may be increased or reduced by about 5 mg, about 10 mg, about 15 mg, or about 20 mg. The dosage may be about 10 mg, about 15 mg, about 20 mg, about 25 mg, about 30 mg, about 40 mg, about 50 mg, about 60 mg, about 70 mg, or about 80 mg. In a non-pregnant female subject, the acceptable range may be about 35% to about 43%. In a pregnant female subject, the acceptable range may be about about 30% to about 36%. The subject may not need or have a phlebotomy for at least eight weeks, at least 12 weeks, at least 16 weeks, at least 6 months, at least one year, or at least two years following the first administration of the hepcidin analogue, and during treatment. The treatment may continue for at least eight weeks, at least 12 weeks, at least 16 weeks, at least 6 months, at least one year, or at least two years.

The method may comprise multiple administrations of an effective amount of the hepcidin analogue or pharmaceutically acceptable salt thereof over a period of time, e.g., wherein the hepcidin analogue or pharmaceutically acceptable salt thereof is administered to the subject about once a week or about twice a week over the period of time. The effective amount may vary from one administration to another administration, or it may remain the same. The period of time may vary, and be, for example, one week to 10 years, one month to 10 years, one month to five years, one month to two years, or four months to one year. In accordance with the present invention, the hepcidin analogue is:
( Isovaleric acid-DTHFPCI(K(isoGlu-Palm))FEPRSKGCK-NH₂ (SEQ ID NO:45), or a pharmaceutically acceptable salt thereof;
, optionally wherein the hepcidin analogue comprises a disulfide bond between two Cys amino acids. The dosage may vary from one administration to another administration, or it may remain the same. In certain embodiments, the dosage is titrated based on the subject's hematocrit levels over the period of time. Thus, in certain embodiments, the method further comprises determining the subject's hematocrit following one or more of the multiple administrations of the hepcidin analogue or pharmaceutically acceptable salt thereof, and the dosage amount given to the subject during the next administration is either maintained or adjusted based on the hematocrit value. For example, the next administered amount may be increased as compared to the previously administered amount, if the subject's determined hematocrit is above an acceptable range based on the subject's sex and pregnancy status; the next administered amount may be descreased as compared to the previously administered amount if the subject's determined hematocrit is below the acceptable range; or the next administered amount may be the same as the previously administered amount, if the subject's determined hematocrit is within the acceptable range. In certain embodiments, the subject's hematocrit is measured about three days, about four days, about five days, about six days, or about seven days following administration of the hepcidin analogue or phharmacetuically acceptable salt thereof. The subject's hematocrit level may be determined following each administration of the hepcidin analogue or pharmaceteutically acceptable salt thereof, or it may be determined only following certain administrations of the hepcidin analogue or pharmaceutically acceptable salt thereof, e.g., one every two weeks, once a month, once every two months, once every four months, or once every six months.

The method may result in the subject's hematocrit level being <45%. In certain embodiments, the subject's hematocrit is maintained within a range of about 37.5% to about 45% (or within the acceptable range for the subject's sex and pregnancy status) over a period of time, e.g., for at least one month, at least two months, at least six month, or longer. In particular embodiments, the method or therapeutic regimen results in a decrease in hematocrit (Hct%) of at least 3%, at least 5%, at least 10% and/or a reduction in phlebotomies of at least 10%, at least 20%, at least 40%, or at least 50% (e.g., in phlebotomy requiring patients). As used herein, a reduction in hematocrit of 3% means an absolute reduction, e.g., a reduction from 46% to 43%.

The method may result in an increase in the subject's serum ferritin levels. In particular embodiments, the serum ferritin levels increase by at least 20%, at least 30%, at least 50%, at least 100%, or at least 200% during a therapeutic regimen, or for at least one month, at least two months, at least six month, or longer. In particular embodiments, the subject's serum ferritin level is maintained within a range of about 25 ng/mL to about 150 ng/mL over a period of time, e.g., for at least one month, at least two months, at least six month, or longer.

The method may result in or causes a decrease in the subject's transferrin saturation (TSAT) level and/or serum iron level of at least 60% or at least 80%. In some embodiments, the subject's TSAT level is decreased to less than 40%. In some embodiments of the methods disclosed herein, the method results in a modest increase or no change in TSAT and/or serum iron levels, and in certain embodiments, TSAT and/or serum iron levels remain below normal levels.

The method may result in an increase in the subject's MCV and/or MCH. In particular embodiments, the MCV and/or MCH increases by at least 10%, at least 20%, at least 30%, at least 50%, at least 100%, or at least 200% during a therapeutic regimen, or for at least one month, at least two months, at least six month, or longer

The method may result in causes a decrease in the subject's hematocrit and/or erythrocyte count of at least 10%, at least 20%, at least 30%, at least 40%, at least 50%, at least 60% or at least 80%. In some embodiments, the subject's TSAT level is decreased to less than 40%. In some embodiments of the methods disclosed herein, the method results in a modest increase or no change in TSAT and/or serum iron levels, and in certain embodiments, TSAT and/or serum iron levels remain below normal levels.

The method may result in the subject not needing or having a therapeutic phlebotomy, e.g., for at least 7 weeks, at least 8 weeks, at least 9 weeks, at least 10 weeks, at least 11 weeks, or at least 12 weeks.
on the subject may be a low risk or high risk PV patient. In particular embodiments, a low risk PV patient is a PV patient less than 60 years old. In particular embodiments, a high risk PV patient is a PV patient greater than or equal to 60 years old.

In particular embodiments, a therapeutic regimen comprises two or more, three or more, four or more, or serial administrations of a hepcidin analogue over a period of time, e.g., about once a week or about once every two weeks for the period of time, for at least one month, at least two months, at least six month, or longer.

In some examples, the method does not result in a substantial change in the subject's platelet counts, e.g., an increase or decrease greater than 50%. For example, the subject's platelet counts do not increase or decrease by more than 10%, 20%, 30%, 40% or 50%.

In some examples, the method does not result in a substantial change in the subject's red blood cell counts, e.g., an increase or decrease greater than 50%. For example, the subject's red blood cell counts do not increase or decrease by more than 10%, 20%, 30%, 40% or 50%.

In some examples, the method does not result in a substantial change in the subject's leukocyte or white blood cell counts, e.g., an increase or decrease greater than 50%. For example s, the subject's leukocyte or white blood cell counts do not increase or decrease by more than 10%, 20%, 30%, 40% or 50%.

In some examples, the method results in a therapeutic benefit to the subject, which may include lessening or alleviation of one or more symptoms of PV. Such symptoms include, but are not limited to, itchiness, hair loss, fatigue, headache, visual disturbances, night sweats, and thrombotic events.

The hepcidin analogue of the present invention (i.e., a first therapeutic agent) may be provided to the subject in combination with a second therapeutic agent. In certain embodiments, the second therapeutic agent is provided to the subject before and/or simultaneously with and/or after the hepcidin analogue is administered to the subject. For example, the second therapeutic agent is iron chelator e.g. selected from Deferoxamine and Deferasirox (Exjade ^{™}). The subject may also be administered a third therapeutic agent.

The present invention provides compositions (for example pharmaceutical compositions) comprising one or more hepcidin analogues of the present invention and a pharmaceutically acceptable carrier, excipient or diluent. A pharmaceutically acceptable carrier, diluent or excipient refers to a non-toxic solid, semi-solid or liquid filler, diluent, encapsulating material or formulation auxiliary of any type.

The term "pharmaceutically acceptable carrier" includes any of the standard pharmaceutical carriers. Pharmaceutically acceptable carriers for therapeutic use are well known in the pharmaceutical art and are described, for example, in "Remington's Pharmaceutical Sciences", 17th edition, Alfonso R. Gennaro (Ed.), Mark Publishing Company, Easton, PA, USA, 1985. For example, sterile saline and phosphate-buffered saline at slightly acidic or physiological pH may be used. Suitable pH-buffering agents may, e.g., be phosphate, citrate, acetate, tris(hydroxymethyl)aminomethane (TRIS), N-tris(hydroxymethyl)methyl-3-aminopropanesulfonic acid (TAPS), ammonium bicarbonate, diethanolamine, histidine, arginine, lysine or acetate (e.g. as sodium acetate), or mixtures thereof. The term further encompasses any carrier agents listed in the US Pharmacopeia for use in animals, including humans.

Also disclosed herein are compositions comprising two or more hepcidin analogues disclosed herein. For example, the combination may be selected from one of the following: (i) any two or more of the hepcidin analogue peptide monomers shown therein; (ii) any two or more of the hepcidin analogue peptide dimers disclosed herein; (iii) any one or more of the hepcidin analogue peptide monomers disclosed herein, and any one or more of the hepcidin analogue peptide dimers disclosed herein.

It is to be understood that the inclusion of a hepcidin analogue of the invention in a pharmaceutical composition also encompasses inclusion of a pharmaceutically acceptable salt or solvate of a hepcidin analogue of the invention. In particular embodiments, the pharmaceutical compositions further comprise one or more pharmaceutically acceptable carrier, excipient, or vehicle.

Compounds described herein include isotopically-labeled compounds, which are identical to those recited in the various formulas and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, fluorine and chlorine, such as ²H, ³H, ¹3C, ¹⁴C, ¹⁵N, ¹⁸O, ¹⁷O, ³⁵S, ¹⁸F, ³⁶Cl, respectively. Certain isotopically-labeled compounds described herein, for example those into which radioactive isotopes such as ³H and ¹⁴C are incorporated, are useful in drug and/or substrate tissue distribution assays. Further, substitution with isotopes such as deuterium, i.e., ²H, can afford certain therapeutic advantages resulting from greater metabolic stability, for example increased in vivo half-life or reduced dosage requirements.

The hepcidin analogues of the present invention may be formulated as pharmaceutical compositions which are suited for administration with or without storage, and which typically comprise a therapeutically effective amount of at least one hepcidin analogue of the invention, together with a pharmaceutically acceptable carrier, excipient or vehicle.

In some embodiments, the hepcidin analogue pharmaceutical compositions of the invention are in unit dosage form. In such forms, the composition is divided into unit doses containing appropriate quantities of the active component or components. The unit dosage form may be presented as a packaged preparation, the package containing discrete quantities of the preparation, for example, packaged tablets, capsules or powders in vials or ampoules. The unit dosage form may also be, e.g., a capsule, cachet or tablet in itself, or it may be an appropriate number of any of these packaged forms. A unit dosage form may also be provided in single-dose injectable form, for example in the form of a pen device containing a liquid-phase (typically aqueous) composition. Compositions may be formulated for any suitable route and means of administration, e.g., any one of the routes and means of administration disclosed herein.

In particular embodiments, the hepcidin analogue, or the pharmaceutical composition comprising a hepcidin analogue, is suspended in a sustained-release matrix. A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix desirably is chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. One embodiment of a biodegradable matrix is a matrix of one of either polylactide, polyglycolide, or polylactide co-glycolide (co-polymers of lactic acid and glycolic acid).

In accordance with the present invention, the hepcidin analogue of the present invention is administered via subcutaneous injection. Other methods of administration of hepcidin analogue-containing compositions include oral, intracisternal, intravaginal, intraperitoneal, intrarectal, topical (as by powders, ointments, drops, suppository, or transdermal patch, including delivery intravitreally, intranasally, and via inhalation) or buccal. The term "parenteral" as used herein refers to modes of administration which include intravenous, intramuscular, intraperitoneal, intrasternal, subcutaneous, intradermal and intra-articular injection and infusion. Accordingly, compositions may be formulated for delivery by any of these routes of administration.

In certain embodiments, pharmaceutical compositions for parenteral injection comprise pharmaceutically acceptable sterile aqueous or nonaqueous solutions, dispersions, suspensions or emulsions, or sterile powders, for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, betacyclodextrin, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants. These compositions may also contain adjuvants such as preservative, wetting agents, emulsifying agents, and dispersing agents. Prolonged absorption of an injectable pharmaceutical form may be brought about by the inclusion of agents which delay absorption, such as aluminum monostearate and gelatin.

Injectable depot forms include those made by forming microencapsule matrices of the hepcidin analogue in one or more biodegradable polymers such as polylactide-polyglycolide, poly(orthoesters), poly(anhydrides), and (poly)glycols, such as PEG. Depending upon the ratio of peptide to polymer and the nature of the particular polymer employed, the rate of release of the hepcidin analogue can be controlled. Depot injectable formulations are also prepared by entrapping the hepcidin analogue in liposomes or microemulsions compatible with body tissues.

The injectable formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Hepcidin analogues of the present invention may also be administered in liposomes or other lipid-based carriers. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a hepcidin analogue of the present invention, stabilizers, preservatives, excipients, and the like. In certain embodiments, the lipids comprise phospholipids, including the phosphatidyl cholines (lecithins) and serines, both natural and synthetic. Methods to form liposomes are known in the art.

Pharmaceutical compositions to be used in the invention suitable for parenteral administration may comprise sterile aqueous solutions and/or suspensions of the peptide inhibitors made isotonic with the blood of the recipient, generally using sodium chloride, glycerin, glucose, mannitol, sorbitol, and the like.

Also described herein are pharmaceutical composition for oral delivery. Compositions and hepcidin analogues may be prepared for oral administration according to any of the methods, techniques, and/or delivery vehicles described herein. Further, one having skill in the art will appreciate that the hepcidin analogues described herein may be modified or integrated into a system or delivery vehicle that is not disclosed herein, yet is well known in the art and compatible for use in oral delivery of peptides.

Formulations for oral administration may comprise adjuvants (e.g. resorcinols and/or nonionic surfactants such as polyoxyethylene oleyl ether and n-hexadecylpolyethylene ether) to artificially increase the permeability of the intestinal walls, and/or enzymatic inhibitors (e.g. pancreatic trypsin inhibitors, diisopropylfluorophosphate (DFF) or trasylol) to inhibit enzymatic degradation. The hepcidin analogue of a solid-type dosage form for oral administration can be mixed with at least one additive, such as sucrose, lactose, cellulose, mannitol, trehalose, raffinose, maltitol, dextran, starches, agar, alginates, chitins, chitosans, pectins, gum tragacanth, gum arabic, gelatin, collagen, casein, albumin, synthetic or semisynthetic polymer, or glyceride. These dosage forms can also contain other type(s) of additives, e.g., inactive diluting agent, lubricant such as magnesium stearate, paraben, preserving agent such as sorbic acid, ascorbic acid, alpha-tocopherol, antioxidants such as cysteine, disintegrators, binders, thickeners, buffering agents, pH adjusting agents, sweetening agents, flavoring agents or perfuming agents.

Oral dosage forms or unit doses compatible for use with hepcidin analogues as described herein may include a mixture of hepcidin analogue and nondrug components or excipients, as well as other non-reusable materials that may be considered either as an ingredient or packaging. Oral compositions may include at least one of a liquid, a solid, and a semi-solid dosage forms. An oral dosage form may comprise an effective amount of hepcidin analogue, wherein the dosage form comprises at least one of a pill, a tablet, a capsule, a gel, a paste, a drink, a syrup, ointment, and suppository. The oral dosage form may be designed and configured to achieve delayed release of the hepcidin analogue in the subject's small intestine and/or colon.

An oral pharmaceutical composition comprising a hepcidin analogue may comprise an enteric coating that is designed to delay release of the hepcidin analogue in the small intestine. A pharmaceutical composition may comprise a hepcidin analogue and a protease inhibitor, such as aprotinin, in a delayed release pharmaceutical formulation. Pharmaceutical compositions of the instant invention may comprise an enteric coat that is soluble in gastric juice at a pH of about 5.0 or higher. Such an enteric coating may comprise a polymer having dissociable carboxylic groups, such as derivatives of cellulose, including hydroxypropylmethyl cellulose phthalate, cellulose acetate phthalate and cellulose acetate trimellitate and similar derivatives of cellulose and other carbohydrate polymers.

The enteric coating may be designed to protect and release the pharmaceutical composition in a controlled manner within the subject's lower gastrointestinal system, and to avoid systemic side effects. In addition to enteric coatings, hepcidin analogues may be encapsulated, coated, engaged or otherwise associated within any compatible oral drug delivery system or component. For example, a hepcidin analogue may be provided in a lipid carrier system comprising at least one of polymeric hydrogels, nanoparticles, microspheres, micelles, and other lipid systems.

To overcome peptide degradation in the small intestine, a hepcidin analogue may be contained in a hydrogel polymer carrier system, whereby the hydrogel polymer protects the hepcidin analogue from proteolysis in the small intestine and/or colon. The hepcidin analogues may further be formulated for compatible use with a carrier system that is designed to increase the dissolution kinetics and enhance intestinal absorption of the peptide. These methods include the use of liposomes, micelles and nanoparticles to increase GI tract permeation of peptides.

Various bioresponsive systems may also be combined with one or more hepcidin analogue to provide a pharmaceutical agent for oral delivery. A hepcidin analogue may be used in combination with a bioresponsive system, such as hydrogels and mucoadhesive polymers with hydrogen bonding groups (e.g., PEG, poly(methacrylic) acid [PMAA], cellulose, Eudragit^{®}, chitosan and alginate) to provide a therapeutic agent for oral administration. Also described is a method for optimizing or prolonging drug residence time for a hepcidin analogue disclosed herein, wherein the surface of the hepcidin analogue surface is modified to comprise mucoadhesive properties through hydrogen bonds, polymers with linked mucins or/and hydrophobic interactions. These modified peptide molecules may demonstrate increase drug residence time within the subject. Moreover, targeted mucoadhesive systems may specifically bind to receptors at the enterocytes and M-cell surfaces, thereby further increasing the uptake of particles containing the hepcidin analogue.

Also disclosed herein is a method for oral delivery of a hepcidin analogue, wherein the hepcidin analogue is provided to a subject in combination with permeation enhancers that promote the transport of the peptides across the intestinal mucosa by increasing paracellular or transcellular permeation. For example, a permeation enhancer is combined with a hepcidin analogue, wherein the permeation enhancer comprises at least one of a long-chain fatty acid, a bile salt, an amphiphilic surfactant, and a chelating agent. A permeation enhancer comprising sodium N-[hydroxybenzoyl)amino] caprylate may be used to form a weak noncovalent association with the hepcidin analogue, wherein the permeation enhancer favors membrane transport and further dissociation once reaching the blood circulation. A hepcidin analogue may be conjugated to oligoarginine, thereby increasing cellular penetration of the peptide into various cell types. A noncovalent bond may be provided between a peptide inhibitor and a permeation enhancer selected from the group consisting of a cyclodextrin (CD) and a dendrimers, wherein the permeation enhancer reduces peptide aggregation and increasing stability and solubility for the hepcidin analogue molecule.

The hepcidin analogue may have a half-life of at least several hours to one day *in vitro* or *in vivo* (e.g., when administered to a human subject) sufficient for daily (q.d.) or twice daily (b.i.d.) dosing of a therapeutically effective amount. For example, the hepcidin analogue may have a half-life of three days or longer sufficient for weekly (q.w.) dosing of a therapeutically effective amount; or a half-life of eight days or longer sufficient for bi-weekly (b.i.w.) or monthly dosing of a therapeutically effective amount. The hepcidin analogue may be derivatized or modified such that is has a longer half-life as compared to the underivatized or unmodified hepcidin analogue. The hepcidin analogue may contain one or more chemical modifications to increase serum half-life.

When used in at least one of the treatments or delivery systems described herein, a hepcidin analogue of the present invention may be employed in pure form or, where such forms exist, in pharmaceutically acceptable salt form.

### Dosages

The total daily usage of the hepcidin analogues and compositions of the present invention can be decided by the attending physician within the scope of sound medical judgment. The specific therapeutically effective dose level for any particular subject will depend upon a variety of factors including: a) the disorder being treated and the severity of the disorder; b) activity of the specific compound employed; c) the specific composition employed, the age, body weight, general health, sex and diet of the patient; d) the time of administration, route of administration, and rate of excretion of the specific hepcidin analogue employed; e) the duration of the treatment; f) drugs used in combination or coincidental with the specific hepcidin analogue employed, and like factors well known in the medical arts.

**In** particular embodiments, the total daily dose of the hepcidin analogues of the invention to be administered to a human or other mammal host in single or divided doses may be in amounts, for example, from 0.0001 to 300 mg/kg body weight daily or 1 to 300 mg/kg body weight daily. In certain embodiments, a dosage of a hepcidin analogue of the present invention is in the range from about 0.0001 to about 100 mg/kg body weight per day, such as from about 0.0005 to about 50 mg/kg body weight per day, such as from about 0.001 to about 10 mg/kg body weight per day, e.g. from about 0.01 to about 1 mg/kg body weight per day, administered in one or more doses, such as from one to three doses.

**In** particular embodiments, a total dosage is about 10 mg to about 100 mg, or about 10 mg to about 70 mg, about 10 mg to about 60 mg, about 20 mg to about 50 mg, about 20 mg to about 40 mg, about 30 mg, about 25 mg, about 20 mg, about 15 mg, or about 10 mg, e.g., for a human patient. In particular embodiments, the hepcidin analogue is provided to the subject once a week. In another particular embodiments, the hepcidin analogue is provided to the subject twice a week e.g., for a human patient.

**In** a more particular embodiments, a total dosage is about 10 mg, about 15 mg, about 20 mg, about 25 mg, about 30 mg, about 40 mg, about 50 mg, about 60 mg, about 70 mg, or about 80 mg once or twice a week for a human patient. In a more particular embodiments, a total dosage is about 10 mg, about 15 mg, about 20 mg, about 25 mg, about 30 mg, about 40 mg, about 50 mg, about 60 mg, about 70 mg, or about 80 mg about every other week or about once a month for a human patient.

In accordance with the present invention, the hepcidin analogue or pharmaceutically acceptable salt thereof of the present invention is provided to the subject about twice a week, about once a week, or about once every two weeks.

Such regular hepcidin analogue administration regimens of the invention may, in certain circumstances such as, e.g., during chronic long-term administration, be advantageously interrupted for a period of time so that the medicated subject reduces the level of or stops taking the medication, often referred to as taking a "drug holiday." Drug holidays are useful for, e.g., maintaining or regaining sensitivity to a drug especially during long-term chronic treatment, or to reduce unwanted side-effects of long-term chronic treatment of the subject with the drug. The timing of a drug holiday depends on the timing of the regular dosing regimen and the purpose for taking the drug holiday (e.g., to regain drug sensitivity and/or to reduce unwanted side effects of continuous, long- term administration). In some embodiments, the drug holiday may be a reduction in the dosage of the drug (e.g. to below the therapeutically effective amount for a certain interval of time). In other embodiments, administration of the drug is stopped for a certain interval of time before administration is started again using the same or a different dosing regimen (e.g. at a lower or higher dose and/or frequency of administration). A drug holiday of the invention may thus be selected from a wide range of time-periods and dosage regimens. An exemplary drug holiday is two or more days, one or more weeks, or one or more months, up to about 24 months of drug holiday. So, for example, a regular daily dosing regimen with a peptide, a peptide analogue, or a dimer of the invention may, for example, be interrupted by a drug holiday of a week, or two weeks, or four weeks, after which time the preceding, regular dosage regimen (e.g. a daily or a weekly dosing regimen) is resumed. A variety of other drug holiday regimens are envisioned to be useful for administering the hepcidin analogues of the invention.

Thus, the hepcidin analogues may be delivered via an administration regime which comprises two or more administration phases separated by respective drug holiday phases.

During each administration phase, the hepcidin analogue is administered to the recipient subject in a therapeutically effective amount according to a pre-determined administration pattern. The administration pattern may comprise continuous administration of the drug to the recipient subject over the duration of the administration phase. Alternatively, the administration pattern may comprise administration of a plurality of doses of the hepcidin analogue to the recipient subject, wherein said doses are spaced by dosing intervals.

A dosing pattern may comprise at least two doses per administration phase, at least five doses per administration phase, at least 10 doses per administration phase, at least 20 doses per administration phase, at least 30 doses per administration phase, or more.

Said dosing intervals may be regular dosing intervals, which may be as set out above, including once daily, twice daily, once every two, three, four, five or six days, once or twice weekly, once or twice monthly, or a regular and even less frequent dosing interval, depending on the particular dosage formulation, bioavailability, and pharmacokinetic profile of the hepcidin analogue of the present invention.

An administration phase may have a duration of at least two days, at least a week, at least 2 weeks, at least 4 weeks, at least a month, at least 2 months, at least 3 months, at least 6 months, or more.

Where an administration pattern comprises a plurality of doses, the duration of the following drug holiday phase is longer than the dosing interval used in that administration pattern. Where the dosing interval is irregular, the duration of the drug holiday phase may be greater than the mean interval between doses over the course of the administration phase. Alternatively the duration of the drug holiday may be longer than the longest interval between consecutive doses during the administration phase.

The duration of the drug holiday phase may be at least twice that of the relevant dosing interval (or mean thereof), at least 3 times, at least 4 times, at least 5 times, at least 10 times, or at least 20 times that of the relevant dosing interval or mean thereof.

Within these constraints, a drug holiday phase may have a duration of at least two days, at least a week, at least 2 weeks, at least 4 weeks, at least a month, at least 2 months, at least 3 months, at least 6 months, or more, depending on the administration pattern during the previous administration phase.

An administration regime comprises at least 2 administration phases. Consecutive administration phases are separated by respective drug holiday phases. Thus the administration regime may comprise at least 3, at least 4, at least 5, at least 10, at least 15, at least 20, at least 25, or at least 30 administration phases, or more, each separated by respective drug holiday phases.

Consecutive administration phases may utilise the same administration pattern, although this may not always be desirable or necessary. However, if other drugs or active agents are administered in combination with a hepcidin analogue of the invention, then typically the same combination of drugs or active agents is given in consecutive administration phases. In certain embodiments, the recipient subject is human.

Also described herein are methods of treating a disease of iron metabolism in a subject, such as a mammalian subject, and preferably a human subject, comprising administering at least one hepcidin analogue, or composition as disclosed herein to the subject. The hepcidin analogue or the composition may be administered in a therapeutically effective amount. Also described herein are methods of treating diabetes (Type I or Type II), insulin resistance, or glucose intolerance in a subject, such as a mammalian subject, and preferably a human subject, comprising administering at least one hepcidin analogue or composition as disclosed herein to the subject. The hepcidin analogue or composition may be administered in a therapeutically effective amount.

Also described is a process for manufacturing a hepcidin analogue or a hepcidin analogue composition (e.g., a pharmaceutical composition), as disclosed herein.

Also described is a device comprising at least one hepcidin analogue of the present invention, or pharmaceutically acceptable salt or solvate thereof for delivery of the hepcidin analogue to a subject.

Also described are methods of binding a ferroportin or inducing ferroportin internalization and degradation which comprises contacting the ferroportin with at least one hepcidin analogue, or hepcidin analogue composition as disclosed herein.

Also described are kits comprising at least one hepcidin analogue, or hepcidin analogue composition (e.g., pharmaceutical composition) as disclosed herein packaged together with a reagent, a device, instructional material, or a combination thereof.

Also described is a method of administering a hepcidin analogue or hepcidin analogue composition (e.g., pharmaceutical composition) to a subject *via* implant or osmotic pump, by cartridge or micro pump, or by other means appreciated by the skilled artisan, as well-known in the art.

Also described are complexes which comprise at least one hepcidin analogue as disclosed herein bound to a ferroportin, preferably a human ferroportin, or an antibody, such as an antibody which specifically binds a hepcidin analogue as disclosed herein, Hep25, or a combination thereof.

The hepcidin analogue of the present invention may have a measurement (e.g., an EC50) of less than 500 nM within the Fpn internalization assay. As a skilled person will realize, the function of the hepcidin analogue is dependent on the tertiary structure of the hepcidin analogue and the binding surface presented. It is therefore possible to make minor changes to the sequence encoding the hepcidin analogue that do not affect the fold or are not on the binding surface and maintain functionAlso described herein is a hepcidin analogue having 85% or higher (e.g., 85%, 90%, 91%, 92%, 93%, 94%, 95%, 96%, 97%, 98%, 99%, or 99.5%) identity or homology to an amino acid sequence of any hepcidin analogue described herein that exhibits an activity (e.g., hepcidin activity), or lessens a symptom of a disease or indication for which hepcidin is involved.

Also disclosed herein are hepcidin analogues having 85% or higher (e.g., 85%, 90%, 91%, 92%, 93%, 94%, 95%, 96%, 97%, 98%, 99%, or 99.5%) identity or homology to an amino acid sequence of any hepcidin analogue presented herein, or a peptide according to any one of the formulae or hepcidin analogues described herein.

A hepcidin analogue may comprise functional fragments or variants thereof that have at most 10, 9, 8, 7, 6, 5, 4, 3, 2, or 1 amino acid substitutions compared to one or more of the specific peptide analogue sequences recited herein.

In addition to the methods described in the Examples herein, the hepcidin analogues of the present invention may be produced using methods known in the art including chemical synthesis, biosynthesis or in vitro synthesis using recombinant DNA methods, and solid phase synthesis. See e.g. Kelly & Winkler (1990) Genetic Engineering Principles and Methods, vol. 12, J. K. Setlow ed., Plenum Press, NY, pp. 1-19; Merrifield (1964) J Amer Chem Soc 85:2149; Houghten (1985) PNAS USA 82:5131-5135; and Stewart & Young (1984) Solid Phase Peptide Synthesis, 2ed. Pierce, Rockford, IL. The hepcidin analogues of the present invention may be purified using protein purification techniques known in the art such as reverse phase high-performance liquid chromatography (HPLC), ion-exchange or immunoaffinity chromatography, filtration or size exclusion, or electrophoresis. See Olsnes, S. and A. Pihl (1973) Biochem. 12(16):3121-3126; and Scopes (1982) Protein Purification, Springer- Verlag, NY. Alternatively, the hepcidin analogues of the present invention may be made by recombinant DNA techniques known in the art. Thus, polynucleotides that encode the polypeptides of the present invention are contemplated herein. The polynucleotides may be isolated. As used herein "isolated polynucleotides" refers to polynucleotides that are in an environment different from that in which the polynucleotide naturally occurs.

### EXAMPLES

The following examples demonstrate certain specific embodiments of the present invention. The following examples were carried out using standard techniques that are well known and routine to those of skill in the art, except where otherwise described in detail. It is to be understood that these examples are for illustrative purposes only and do not purport to be wholly definitive as to conditions or scope of the invention. As such, they should not be construed in any way as limiting the scope of the present invention.

### ABBREVIATIONS:

- DCM:: dichloromethane
- DMF:: N,N-dimethylformamide
- NMP:: N-methylpyrolidone
- HBTU:: O-(Benzotriazol-1-yl)-N,N,N',N'-tetramethyluronium hexafluorophosphate
- HATU:: 2-(7-aza-1H-benzotriazole-1-yl)-1,1,3,3-tetramethyluronium hexafluorophosphate
- DCC:: Dicyclohexylcarbodiimide
- NHS:: N-hydoxysuccinimide
- DIPEA:: diisopropylethylamine
- EtOH:: ethanol
- Et2O:: diethyl ether
- Hy:: hydrogen
- TFA:: trifluoroacetic acid
- TIS:: triisopropylsilane
- ACN:: acetonitrile
- HPLC:: high performance liquid chromatography
- ESI-MS:: electron spray ionization mass spectrometry
- PBS:: phosphate-buffered saline
- Boc:: t-butoxycarbonyl
- Fmoc:: Fluorenylmethyloxycarbonyl
- Acm:: acetamidomethyl
- IVA:: Isovaleric acid (or Isovaleryl)

K( ): In the peptide sequences provided herein, wherein a compound or chemical group is presented in parentheses directly after a Lysine residue, it is to be understood that the compound or chemical group in the parentheses is a side chain conjugated to the Lysine residue. So, e.g., but not to be limited in any way, K-[(PEG8)]- indicates that a PEG8 moiety is conjugated to a side chain of this Lysine.

Palm: Indicates conjugation of a palmitic acid (palmitoyl).

As used herein "C( )" refers to a cysteine residue involved in a particular disulfide bridge. For example, in Hepcidin, there are four disulfide bridges: the first between the two C(1) residues; the second between the two C(2) residues; the third between the two C(3) residues; and the fourth between the two C(4) residues. Accordingly, in some embodiments, the sequence for Hepcidin is written as follows:
Hy-DTHFPIC(1)IFC(2)C(3)GC(2)C(4)HRSKC(3)GMC(4)C(1)KT-OH; and the sequence for other peptides may also optionally be written in the same manner.

### EXAMPLE 1

### SYNTHESIS OF PEPTIDE ANALOGUES

Unless otherwise specified, reagents and solvents employed in the following were available commercially in standard laboratory reagent or analytical grade, and were used without further purification.

### Procedure for solid-phase synthesis of peptides

Peptide analogues of the invention were chemically synthesized using optimized 9-fluorenylmethoxy carbonyl (Fmoc) solid phase peptide synthesis protocols. For C-terminal amides, rink-amide resin was used, although wang and trityl resins were also used to produce C-terminal acids. The side chain protecting groups were as follows: Glu, Thr and Tyr: O-tButyl; Trp and Lys: t-Boc (t-butyloxycarbonyl); Arg: N-gamma-2,2,4,6,7-pentamethyldihydrobenzofuran-5-sulfonyl; His, Gln, Asn, Cys: Trityl. For selective disulfide bridge formation, Acm (acetamidomethyl) was also used as a Cys protecting group. For coupling, a four to ten-fold excess of a solution containing Fmoc amino acid, HBTU and DIPEA (1:1:1.1) in DMF was added to swelled resin [HBTU: O-(Benzotriazol-1-yl)-N,N,N',N'-tetramethyluronium hexafluorophosphate; DIPEA: diisopropylethylamine; DMF: dimethylformamide]. HATU (O-(7-azabenzotriazol-1-yl)-1,1,3,3,-tetramethyluronium hexafluorophosphate) was used instead of HBTU to improve coupling efficiency in difficult regions. Fmoc protecting group removal was achieved by treatment with a DMF, piperidine (2:1) solution.

### Procedure for cleavage of peptides off resin

Side chain deprotection and cleavage of the peptide analogues of the invention (e.g., Compound No. 2) was achieved by stirring dry resin in a solution containing trifluoroacetic acid, water, ethanedithiol and tri-isopropylsilane (90:5:2.5:2.5) for 2 to 4 hours. Following TFA removal, peptide was precipitated using ice-cold diethyl ether. The solution was centrifuged and the ether was decanted, followed by a second diethyl ether wash. The peptide was dissolved in an acetonitrile, water solution (1:1) containing 0.1% TFA (trifluoroacetic acid) and the resulting solution was filtered. The linear peptide quality was assessed using electrospray ionization mass spectrometry (ESI-MS).

### Procedure for purification of peptides

Purification of the peptides of the invention (e.g., Compound No. 2) was achieved using reverse-phase high performance liquid chromatography (RP-HPLC). Analysis was performed using a C18 column (3µm, 50 x 2mm) with a flow rate of 1 mL/min. Purification of the linear peptides was achieved using preparative RP-HPLC with a C18 column (5µm, 250 x 21.2 mm) with a flow rate of 20 mL/min. Separation was achieved using linear gradients of buffer B in A (Buffer A: Aqueous 0.05% TFA; Buffer B: 0.043% TFA, 90% acetonitrile in water).

### Procedure for oxidation of peptides

Method A (Single disulfide oxidation). Oxidation of the unprotected peptides of the invention was achieved by adding drop-wise iodine in MeOH (1 mg per 1 mL) to the peptide in a solution (ACN: H₂O, 7: 3, 0.5% TFA). After stirring for 2 min, ascorbic acid portion wise was added until the solution was clear and the sample was immediately loaded onto the HPLC for purification.

Method B (Selective oxidation of two disulfides). When more than one disulfide was present, selective oxidation was often performed. Oxidation of the free cysteines was achieved at pH 7.6 NH₄CO₃ solution at 1mg /10 mL of peptide. After 24 h stirring and prior to purification the solution was acidified to pH 3 with TFA followed by lyophilization. The resulting single oxidized peptides (with ACM protected cysteines) were then oxidized / selective deprotection using iodine solution. The peptide (1 mg per 2 mL) was dissolved in MeOH/H₂0, 80:20 iodine dissolved in the reaction solvent was added to the reaction (final concentration: 5 mg/mL) at room temperature. The solution was stirred for 7 minutes before ascorbic acid was added portion wise until the solution is clear. The solution was then loaded directly onto the HPLC.

Method C (Native oxidation). When more than one disulfide was present and when not performing selective oxidations, native oxidation was performed. Native oxidation was achieved with 100 mM NH4CO3 (pH7.4) solution in the presence of oxidized and reduced glutathione (peptide/GSH/GSSG, 1:100:10 molar ratio) of (peptide: GSSG: GSH, 1:10, 100). After 24 h stirring and prior to RP-HPLC purification the solution was acidified to pH 3 with TFA followed by lyophilization.

Procedure of cysteine oxidation to produce dimers. Oxidation of the unprotected peptides of the invention was achieved by adding drop-wise iodine in MeOH (1 mg per 1 mL) to the peptide in a solution (ACN: H2O, 7: 3, 0.5% TFA). After stirring for 2 min, ascorbic acid portion wise was added until the solution was clear and the sample was immediately loaded onto the HPLC for purification.

### Procedure for dimerization.

Glyoxylic acid (DIG), IDA, or Fmoc-β-Ala-IDA was pre-activated as the N-hydoxysuccinimide ester by treating 1 equivalent (abbreviated "eq") of the acid with 2.2 eq of both N-hydoxysuccinimide (NHS) and dicyclohexyl carbodiimide (DCC) in NMP (N-methyl pyrolidone) at a 0.1 M final concentration. For the PEG13 and PEG25 linkers, these chemical entities were purchased pre-formed as the activated succinimide ester. The activated ester ~0.4 eq was added slowly to the peptide in NMP (1mg/mL) portionwise. The solution was left stirring for 10 min before 2-3 additional aliquots of the linker ~0.05 eq were slowly added. The solution was left stirring for a further 3 h before the solvent was removed under *vaccuo* and the residue was purified by reverse phase HPLC. An additional step of stirring the peptide in 20% piperidine in DMF (2 x 10 min) before an additional reverse phase HPLC purification was performed.

One of skill in the art will appreciate that standard methods of peptide synthesis may be used to generate the compounds of the invention.

### Linker activation and dimerization

Peptide monomer subunits were linked to form hepcidin analogue peptide dimers as described below.

Small Scale DIG Linker Activation Procedure: 5mL of NMP was added to a glass vial containing IDA diacid (304.2 mg, 1 mmol), N-hydroxysuccinimide (NHS, 253.2 mg, 2.2 eq. 2.2mmol) and a stirring bar. The mixture was stirred at room temperature to completely dissolve the solid starting materials. N, N'-Dicyclohexylcarbodiimide (DCC, 453.9mg, 2.2 eq., 2.2 mmol) was then added to the mixture. Precipitation appeared within 10 min and the reaction mixture was further stirred at room temperature overnight. The reaction mixture was then filtered to remove the precipitated dicyclohexylurea (DCU). The activated linker was kept in a closed vial prior to use for dimerization. The nominal concentration of the activated linker was approximately 0.20 M.

For dimerization using PEG linkers, there was no pre-activation step involved. Commercially available pre-activated bi-functional PEG linkers were used.

Dimerization Procedure: 2mL of anhydrous DMF was added to a vial containing peptide monomer (0.1 mmol). The pH of the peptide was the adjusted to 8~9 with DIEA. Activated linker (IDA or PEG13, PEG 25) (0.48eq relative to monomer, 0.048 mmol) was then added to the monomer solution. The reaction mixture was stirred at room temperature for one hour. Completion of the dimerization reaction was monitored using analytical HPLC. The time for completion of dimerization reaction varied depending upon the linker. After completion of reaction, the peptide was precipitated in cold ether and centrifuged. The supernatant ether layer was discarded. The precipitation step was repeated twice. The crude dimer was then purified using reverse phase HPLC (Luna C18 support, 10u, 100A, Mobile phase A: water containing 0.1% TFA, mobile phase B: Acetonitrile (ACN) containing 0.1% TFA, gradient of 15%B and change to 45%B over 60min, flow rate 15ml/min). Fractions containing pure product were then freeze-dried on a lyophilizer.

### Conjugation of Half-Life Extension Moieties

Conjugation of peptides were performed on resin. Lys(ivDde) was used as the key amino acid. After assembly of the peptide on resin, selective deprotection of the ivDde group occurred using 3 x 5 min 2% hydrazine in DMF for 5 min. Activation and acylation of the linker using HBTU, DIEA 1-2 equivalents for 3 h, and Fmoc removal followed by a second acylation with the lipidic acid gave the conjugated peptide.

### EXAMPLE 2

### ACTIVITY OF PEPTIDE ANALOGUES

Peptide analogues were tested *in vitro* for induction of internalization of the human ferroportin protein. Following internalization, the peptides are degraded. The assay measures a decrease in fluorescence of the receptor.

The cDNA encoding the human ferroportin (SLC40A1) was cloned from a cDNA clone from Origene (NM_014585). The DNA encoding the ferroportin was amplified by PCR using primers also encoding terminal restriction sites for subcloning, but without the termination codon. The ferroportin receptor was subcloned into a mammalian GFP expression vector containing a neomycin (G418) resistance marker in such that the reading frame of the ferroportin was fused in frame with the GFP protein. The fidelity of the DNA encoding the protein was confirmed by DNA sequencing. HEK293 cells were used for transfection of the ferroportin-GFP receptor expression plasmid. The cells were grown according to standard protocol in growth medium and transfected with the plasmids using Lipofectamine (manufacturer's protocol, Invitrogen). The cells stably expressing ferroportin-GFP were selected using G418 in the growth medium (in that only cells that have taken up and incorporated the cDNA expression plasmid survive) and sorted several times on a Cytomation MoFlo ^{™} cell sorter to obtain the GFP-positive cells (488nm/530 nm). The cells were propagated and frozen in aliquots.

To determine activity of the hepcidin analogues (compounds) on the human ferroportin, the cells were incubated in 96 well plates in standard media, without phenol red. Compound was added to desired final concentration for at least 18 hours in the incubator. Following incubation, the remaining GFP-fluorescence was determined either by whole cell GFP fluorescence (Envision plate reader, 485 / 535 filter pair), or by Beckman Coulter Quanta ^{™} flow cytometer (express as Geometric mean of fluorescence intensity at 485nm/525nm). Compound was added to desired final concentration for at least 18 hours but no more than 24 hours in the incubator.

In certain experiments, reference compounds included native Hepcidin, Mini-Hepcidin, and R1-Mini-Hepcidin, which is an analog of mini-hepcidin. The "RI" in RI-Mini-Hepcidin refers to Retro Inverse. A retro inverse peptide is a peptide with a reversed sequence in all D amino acids. An example is that Hy-Glu-Thr-His-NH2 becomes Hy-DHis-DThr-DGlu-NH2. The EC₅₀ of these reference compounds for ferroportin degradation was determined according to the activity assay described above. These peptides served as control standards.

**Table 6. Reference compounds**

| **Name** | **Sequence** | **Potency EC50 (nM)** |
|---|---|---|
| Hepcidin | Hy-DTHFPIC(1)IFC(2)C(3)GC(2)C(4)HRSKC(3)GMC(4)C(1)KT- OH (SEQ ID NO: 56) | 34 |
| Mini-Hepcidin | Hy-DTHFPICIF-NH₂ (SEQ ID NO: 57) | 712 |
| RI-Mini Hepcidin | Hy-DPhe-DIle-DCys-DIle-DPro-DPhe-DHis-DThr-DAsp-NH₂ (SEQ ID NO: 58) | > 10 µM |

The potency EC₅₀ values (nM) determined for various peptide analogues of the present invention and other activity data are provided in granted patents, US 9,822,157 and US 10,030,061.

### EXAMPLE 3

### HEPCIDIN INHIBITION IN POLYCYTHEMIA VERA

Hepcidin analogues of the present invention are tested for their activity in Polycythemia vera as described by Casu et al., Blood. 2016; 128(2):265-276.

Hepcidin, a 25-amino-acid peptide, governs systemic iron homeostasis and is generated by the liver in response to plasma iron concentration and iron stores. Hepcidin inhibits the cellular iron exporter ferroportin (FPN-1), which is expressed on the surfaces of cells that are involved in iron absorption, recycling, and storage. Hepcidin modulates systemic iron restriction and exogenous administration of an exogenous hepcidin mimetic decreased Hgb concentrations and splenomegaly in a murine model for Polycythemia Vera (Casu et al, Blood. 2016;128(2):265-276).

When JAK2 is constitutively activated erythropoietin-independent red blood cell production is triggered leading to polycythemia. An approach to prevent the effect of mutated Jak2 is to induce iron restriction with hepcidin or hepcidin mimetic peptides. Low iron level inhibits erythropoietin signaling at a point that is downstream of Jak2 thus providing an override signal. When hepcidin mimetic peptides are administered to transgenic mice that express a human Jak2 gene carrying the polycythemia causing mutation the increased red cell production and hematocrit that is characteristic of polycythemia vera is reversed back to the normal range. (Casu et al, Blood. 2016; 128(2):265-276).

### EXAMPLE 4

### IN VIVO VALIDATION OF PEPTIDE ANALOGUES OF HEPCIDIN

Hepcidin analogues of the present invention were tested for *in vivo* activity, to determine their ability to decrease free Fe2+ in serum.

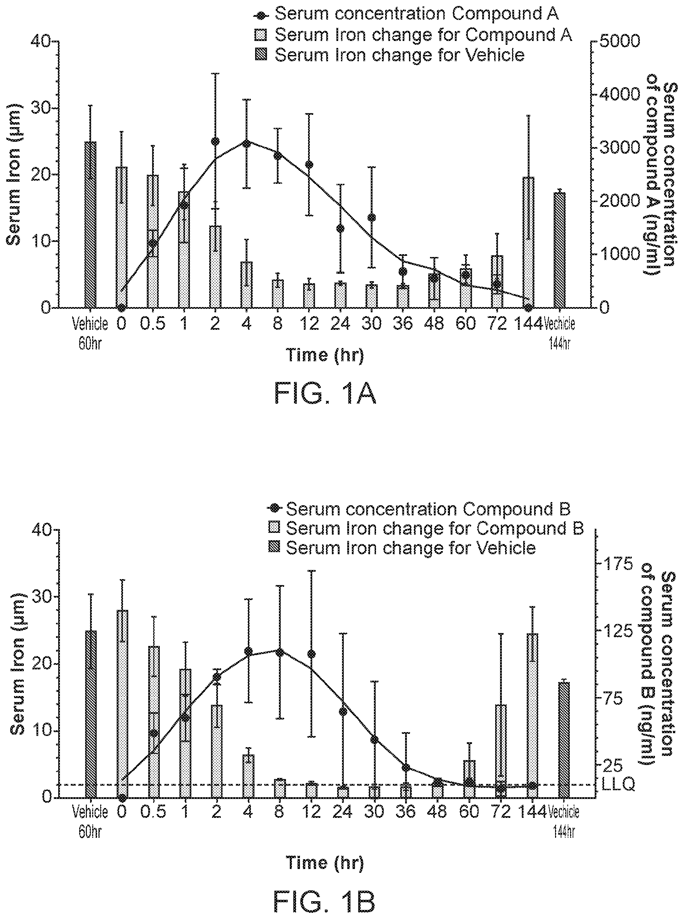

In a PK-PD experiment, hepcidin analog of the present invention Compound A (SEQ ID NO:45), or hepcidin analog Compound B (SEQ ID NO:55), or vehicle control were administered to Cynomolgus monkeys (n=3/group) at 2.44 mg/kg of Compound A or 2.93 mg/kg of Compound B subcutaneously. Serum samples were taken from groups of monkeys administered with the hepcidin analogs at 0.5 h, 1 h, 2 h, 4 h, 8h, 12 h, 24 h, 30 h, 36 h, 48 h, 60 h, 72 h, and 144 h post-administration of the dose. Iron content in plasma/serum was measured using a colorimetric assay on the Cobas c 111 according to instructions from the manufacturer of the assay (assay: IRON2: ACN 661). The data obtained from the Cobas Iron2 analysis is presented in Figure 1A for Compound A and Figure 1B for Compound B (as mean values with SD). Compound A elicited around 5-fold reduction in serum iron at 8hr post compound administration. Serum iron reduction for Compound B was maximal at about 12 hour post compound administration, and it was 12-fold lower than the pre-dose levels.

These studies demonstrate that hepcidin analogues of the present invention reduce serum iron levels for at least 60 hours when dosed in Cynomolgus monkeys. There is a concentration dependent effect on reduction of serum iron for both Compound A and Compound B. There is also an effect delay between serum concentration of the hepcidin analogue and its corresponding effect i.e. the nadir of the effect occured at a time delayed from the peak serum concentration of the compound. For Compound A, serum concentrations in the range of 200 to 3200 ng/mL were effective, with 1000 - 3200 ng/mL having maximum effect. For Compound B, serum concentrations of 25 - 125 ng/mL showed an effect, with 50 - 125 ng/mL having maximal effect.

### EXAMPLE 5

### EFFICACY OF PEPTIDES IN LIMITING ERYTHROPOIESIS

Hepcidin analogues of the present invention were tested for efficacy in limiting erythropoietic activity when administered in healthy Cynomolgus monkeys. In a repeat-dose study, 4 weekly doses of Compound A or vehicle control were administered subcutaneously (SC) to Cynomolgus monkeys, at three different doses of 0.6 mg/kg/dose, 2mg/kg/dose or 6mg/kg/dose (n=6/sex/high dose or control groups and n=3/sex/low or mid dose groups).

Compound A, at ≥ 3 mg/kg/dose, caused pharmacology-mediated anemia, specifically dose- and time-dependent decreases in Hematocrit (Hct) and Hemoglobin (Hgb) (Table 7 & Figure 2). At Day 29, Hgb levels for 1, 3, and 10 mg/kg Compound A treated males, were reduced by 0.1, 2.7, and 7.2 g/dL as compared to concurrent controls, respectively. Hct levels, in absolute terms, had decreased by 6% and 22% in the 3 and 10 mg/kg/dose male groups, respectively. Male animals yielded similar pharmacologic responses to females. Reticulocytosis occurred following prolonged red blood cell (RBC) reduction, with statistically significant increases observed on Day 29. Following cessation of dosing, RBC parameters returned to concurrent control values.

**Table 7: Hematocrit and hemoglobin changes, from concurrent controls, in NHPs administered Compound A SC once every week for a total of 4 doses followed by a 28-day recovery period**

| Dose (mg/kg/dose) | Day 7¹ | | Day 29¹ | | Day 56 (Recovery)¹ | |
|---|---|---|---|---|---|---|
| | M | F | M | F | M | F |
| % Hct | | | | | | |
| 0 | 36 | 35 | 40 | 37 | 41 | 38 |
| 1² | +2 | 0 | 0 | 0 | - | - |
| 3 | -1 | -2 | -6 | -5 | - | - |
| 10 | -1 | -2 | -22*** | -17*** | +4 | 0 |

| Hgb (g/dL) | | | | | | |
|---|---|---|---|---|---|---|
| 0 | 11.1 | 11.4 | 12.2 | 11.8 | 13.1 | 12.5 |
| 1 | +0.9 | -0.5 | -0.1 | -0.8 | - | - |
| 3 | -0.1 | -0.9 | -2.7** | -2.7** | - | - |

| Dose (mg/kg/dose) | Day 7¹ | | Day 29¹ | | Day 56 (Recovery)¹ | |
|---|---|---|---|---|---|---|
| | M | F | M | F | M | F |
| 10 | -0.2 | -1.4** | -7.2*** | -6.5*** | +1.2 | -0.4 |

| | | | | | | |
|---|---|---|---|---|---|---|
| ¹Samples were taken 6 days post the 1^{st} dose and 7 days post the 4th dose and Day 56 for the Recovery animals (34 days post the 4^{th} dose). ²Changes represent values from concurrent controls, data may probably be tighter and achieve greater statistical significance (lower group) if expressed as changes per individual animal, ie., changes from pre-dose levels. **p<0.01 and ***p<0.001 compared to concurrent control. | | | | | | |

Hematocrit levels and secondary hematologic indices, including mean corpuscular volume (MCV), mean corpuscular hemoglobin concentration (MCHC), and mean corpuscular Hgb per cell (MCH), reflecting the size and Hgb content of RBCs are shown in Figure 2. The RBCs generated following Compound A-induced iron-restricted erythropoiesis, are similar in size (MCV) to concurrent controls, but they exhibit a dose-dependent decrease in Hgb concentration per cell (MCHC and MCH) as compared to their concurrent controls. At recovery, the vehicle control and 10 mg/kg Compound A dosing groups were similar showing reversibility of the hematologic changes. The hematologic findings observed would be expected following exaggerated and sustained iron-restricted anemia in originally iron-replete animals.

### EXAMPLE 6

### EFFICACY OF PEPTIDES IN LIMITING ERYTHROPOIESIS

In another repeat-dose study, 13 weekly doses of Compound A or Vehicle control were administered subcutaneously to Cynomolgus monkeys, at three different doses, and followed by a 5-week recovery period.

Cynomolgus monkeys (6/sex/group) were administered subcutaneous doses of 0 (0.9% saline), 0.6, 2, or 6 mg/kg/dose of Compound A, QW for 3 months for a total of 13 doses (Days 1, 8, 15, 22, 29, 36, 43, 50, 57, 64, 71, 78, and 85). A cohort of animals (4/sex/group) were sacrificed on Day 92 (Main), and the remaining animals (2/sex/group) were sacrificed on Day 120 following a 35-day recovery period (Recovery). Hematology samples were obtained twice prior to start of treatment (Days -7 and -3), and on Days 27, 55 and 90 for Main and Recovery animals, corresponding to 5 days post the 4th, 8th and 13th doses, and Day 119 for Recovery animals.

Hematology changes were consistent with the expected pharmacology of a hepcidin mimetic administered to an iron replete NHP, and included dose-dependent decreases in RBC parameters (RBC counts, Hgb, and Hct) and increases in reticulocytes as well as alterations in RBC indices (decreased MCHC and MCH) and RBC morphology (e.g. microcytosis and hypochromia)(data not shown). Consistent with Compound A induced anemia, hematopoietic hypercellularity occurred in bone marrow (femur, sternum) and extramedullary hematopoiesis and hemosiderin deposition was observed in the liver and spleen (correlated with increased organ weight at 6 mg/kg/dose) at ≥ 2 mg/kg/dose (data not shown).

Hematologic changes consistent with the known erythropoietic pharmacological action of a hepcidin mimetic in inducing iron deficiency anemia occurred (Table 8). At Day 90, Hgb levels for 0.6. 2, and 6 mg/kg Compound A females were reduced by 1.3, 3.0, and 5.6 g/dL compared to concurrent controls, respectively. Male animals yielded similar pharmacologic responses to females. Reticulocytes were elevated across all dosing phase time points in a dose-dependent manner in response to anemia. Following cessation of dosing, RBC parameters returned to concurrent control values.

**Table 8: Hematocrit and hemoglobin changes from concurrent controls in NHPs administered Compound A by SC injection once every week for 13 weeks followed by a 35-day recovery period**

| Dose (mg/kg/dose) | Day 27¹ | | Day 55¹ | | Day 90¹ | | Day 119¹ (Recovery) | |
|---|---|---|---|---|---|---|---|---|
| | M | F | M | F | M | F | M | F |
| % Hct | | | | | | | | |
| 0 | 44 | 43 | 45 | 44 | 40 | 40 | 42 | 40 |
| 0.6² | -3* | -4 | -3** | -3 | -3 | -3 | +3 | +2 |
| 2 | -5*** | -7** | -7*** | -7*** | -4** | -7*** | +4 | +4 |
| 6 | -17*** | -16*** | -21*** | -13*** | -17*** | -14*** | +4 | +6 |

| Hgb (g/dL) | | | | | | | | |
|---|---|---|---|---|---|---|---|---|
| 0 | 13.5 | 13.0 | 13.8 | 13.6 | 12.5 | 12.2 | 13.1 | 12.2 |
| 0.6 | -1.0 | -0.9 | -1.0** | -1.4* | -1.2* | -1.3** | +0.7 | +0.9 |
| 2 | -1.3*** | -2.6*** | -3.0*** | -3.5*** | -2.4*** | -3.0*** | +1.1 | +1.3 |
| 6 | -5.9*** | -5.7*** | -7.2*** | -5.6*** | -6.2*** | -5.6*** | +0.9 | +1.6 |

| | | | | | | | | |
|---|---|---|---|---|---|---|---|---|
| ¹Samples were taken 5 days post the 4th, 8th and 13th doses, and Day 119 for Recovery animals. ²Changes represent values from concurrent controls, data may probably be tighter and achieve greater statistical significance (lower group) if expressed as changes per individual animal, i.e., changes from pre-dose levels. *p<0.05 compared to concurrent control. **p<0.01 compared to concurrent control. ***p<0.001 compared to concurrent control. | | | | | | | | |

In the same study, Compound A induced significant changes in secondary hematologic indices as shown in Figure 3. Dose-dependent decreases in MCHC and MCH are consistent with expected RBC rheological changes associated with iron-restricted erythropoiesis. Hematological effects were similar to concurrent controls 35 days after cessation of dosing.

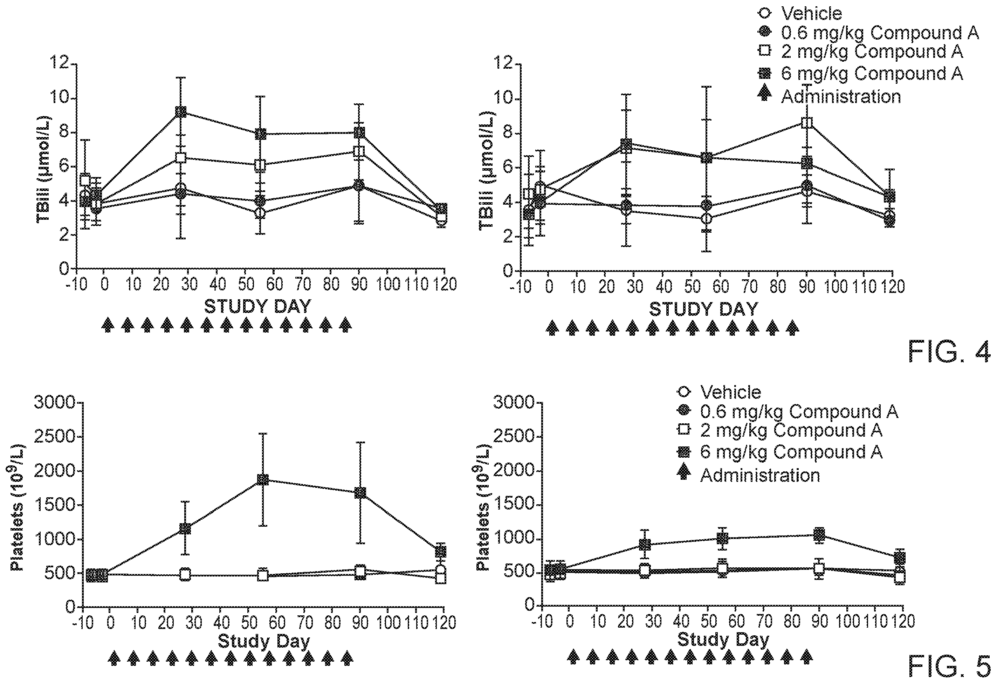

An increase in total bilirubin (tBili) at ≥ 2 mg/kg/day was also observed during the treatment period associated with destruction of RBCs associated with iron deficiency anemia (Figure 4) and considered associated with the preferential destruction of the youngest erythroid cells, the reticulocytes (Robinson & Koeppel, 1971). Consistent with expected induction of iron-deficiency anemia due to Compound A, significantly elevated levels of platelets were observed at the highest evaluated dose of 6 mg/kg/dose in both male and female animals (Figure 5). Following cessation of dosing at the Recovery timepoint, the bilirubin levels had returned to normal levels while the platelet levels were reversing to be within normal limits (compared to the concurrent controls).

### EXAMPLE 7

### Phase 2 Study of Compound A in Patients with Phlebotomy-Requiring Polycythemia Vera

Background: Polycythemia vera (PV) patients are normally treated with periodic therapeutic phlebotomy (with or without concurrent cytoreductive therapy) to maintain hematocrit <45%. Consequently, PV patients with high phlebotomy requirements are likely to have hematocrit >45% between appointments. On the other hand, the majority of PV patients are iron deficient at diagnosis, worsening after repeated phlebotomy. PV patients can be symptomatic from iron deficiency (with cognitive impairment and fatigue even in the absence of anemia), and iron supplementation typically results in increased phlebotomy rates. Recent studies demonstrate that available therapies improve PV-related symptoms in part by reversing iron deficiency. Thus, symptomatic phlebotomy-requiring PV patients present an unmet therapeutic need. We hypothesize that hepcidin mimetics promote the sequestration of iron in splenic macrophages, decreasing iron availability for malignant erythropoiesis to reduce phlebotomy requirements, while reversing iron deficiency-associated symptoms.

Compound A is a hepcidin mimetic agent in clinical studies for multiple blood disorders. In wild type mice, repeated subcutaneous injection of Compound A transiently decreased serum iron and caused a dose-related decrease in hematocrit. A Phase I study of Compound A as a single dose in 62 healthy subjects demonstrated a 65% reduction in serum iron concentration and 70% reduction in transferrin saturation from baseline, without significant adverse events.

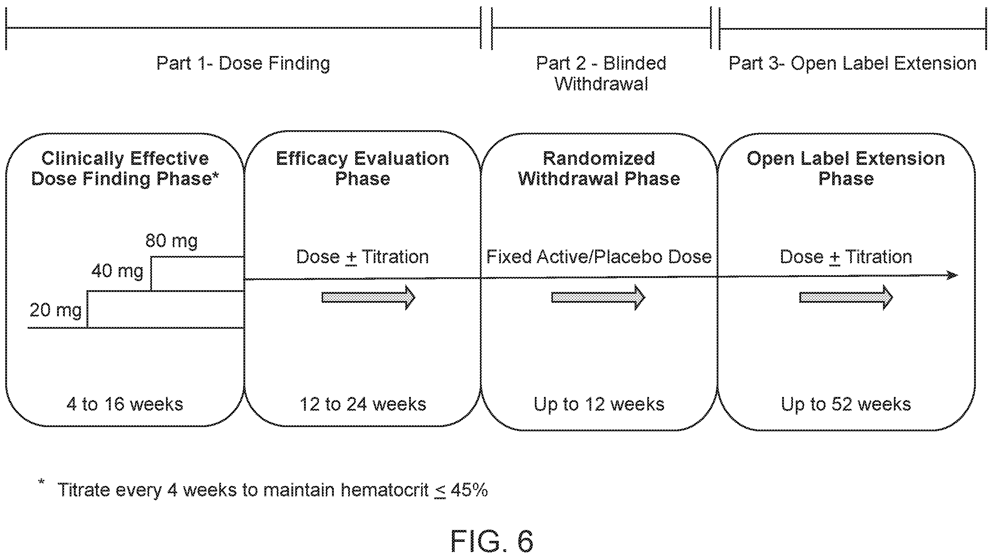

Aims: The primary objectives of this 3-part Phase 2 clinical trial (outlined in Figure 6) were to demonstrate efficacy (decrease in phlebotomy requirement) and safety of Compound A in phlebotomy-requiring PV patients. Secondary objectives were to determine the effect of Compound A on patient reported outcomes and markers of iron metabolism.

Methods: Eligibility criteria included PV diagnosis (by 2016 WHO criteria) and ≥3 phlebotomies to goal hematocrit <45% in the 6 months prior to enrollment with or without a stable dose of cytoreductive therapy. Eligible patients were enrolled in the 28-week dose finding part of the Phase 2 trial. Patients were given Compound A doses of 10, 20, 40, 60 and 80 mg administered subcutaneously weekly in individualized adjustment to maintain hematocrit <45% as outlined in Figure 7. Body iron status was quantified by monitoring serum ferritin, serum iron, transferrin saturation (TSAT), mean corpuscular volume (MCV) and mean corpuscular hemoglobin (MCH).

Results: Efficacy data was available for 13 subjects enrolled in the trial: 7/13 with low risk PV and 6/13 with high risk PV; mean age 57.4 years (range 31-74); with six receiving TP alone, 6 on concurrent hydroxyurea, and 1 on concurrent interferon; TP in the 24 weeks prior to enrollment = 3-9; and median time between TP = 42 days. Patient characteristics are provided in Table 9.

**Table 9: Subjects Enrolled in Compound A Study**

| **Subject** | **Age/ Sex** | **Risk Category** | **Screening HCT, WBC, PLT** | **Allele (%)** | **Prior Cytoreductive Therapy?** | **Concurrent Cytoreductive Therapy?** | **No. of PHL Prior to Compound A** | **Max. No. of Weeks Between PHL** | **No. of Weeks PHL Free After 1^{st} Compound A Dose** | **Compound A Exposure Range** |
|---|---|---|---|---|---|---|---|---|---|---|
| 3 | 64/M | H | 42.3%, 4.3 10^9/L, 234 10^9/L | 52 | Y (HU) | Y (HU 1500 mg) | 3 | 15 | 26 | 10, 20, 10 |
| 5 | 52/M | L | 45.8%, 12.8 10^9/L, 534 10^9/L | 77.7 | -- | N | 3 | 9 | 23 | 10, 20, 40, 80 |
| 1 | 58/F | L | 41.6%, 12.1 10^9/L, 993 10^9/L | 54.7 | -- | N | 5 | 11 | 37 (PHB@W31 i.e. after randomization) | 10, 20, 40, 80, 40, 60, 40 |
| 2 | 71 / M | H | 43.6%, 6.7 10^9/L, 536 10^9/L | 36.1 | -- | N | 4 | 10 | 37 | 10, 20, 40, 20, 40 |
| 4 | 43/M | L | 45.2%, 15.8 10^9/L, 796 10^9/L | 78.4 | HU stopped 2013 | N | 9 | 10 | 24 (PHB@W12) | 20, 40, 80, 60 |
| 9 | 31 / M | L | 43.9%, 5.6 10^9/L, 330 10^9/L | | -- | N | 6 | 11 | 6 | 20, 40 |
| 6 | 68 / F | H | 40.7%, 7.48 K/µL, 579 K/µL | 16.6 | Y (HU) | Y (HU 500 mg) | 3 | 8 | 14 | 20, 40, 80, 40 |
| 7 | 74 / F | H | 41%, 8.68 K/µL, 140 K/µL | 53.7 | Y (HU) | Y (HU 1500 mg) | 4 | 21 | 12 | 20, 40 |
| 10 | 61 / M | H | 42.1%, 5.32 K/µL, 637 K/µL | 34.5 | Y (IFN) | Y (Interferon 180 mcg qw) | 6 | 13 | 1 | 20, 40 |
| 12 | 50/M | L | 44.8%, 5.86 K/µL, 307 K/µL | | Y (IFN) | Y (Interferon) | 4 | 21 | 12 | 20 |
| 13 | 56/M | L | 42.4%, 19.82 K/µL, 328 K/µL | | -- | N | 6 | 13 | 1 | 20 |
| 11 | 52/F | L | 38.5%, 4.1 K/mm³, 351 K/ mm³ | 32.3 | Y (IFN) | Y (Interferon 45 meg) | 6 | 8 | 3 | 20 |
| 8 | 66 / F | H | 45.9%, 8.3 K/µL, 240 K/µL | 61.9 | HU stopped >2yrs ago | N | 5 | 7 | 10 | 20, 40, 20 |

| | | | | | | | | | | |
|---|---|---|---|---|---|---|---|---|---|---|
| Abbreviations: F = female, H = high, HCT = hematocrit, HU = hydroxyurea, L = low, M= male, N = no, PHB = phlebotomy(ies), PLT = platelets, WBC = white blood counts, Y = yes. | | | | | | | | | | |

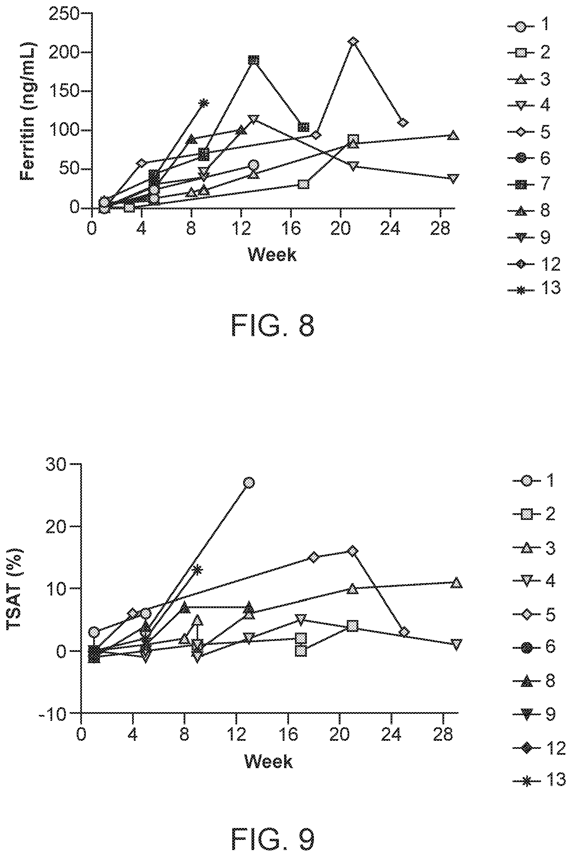

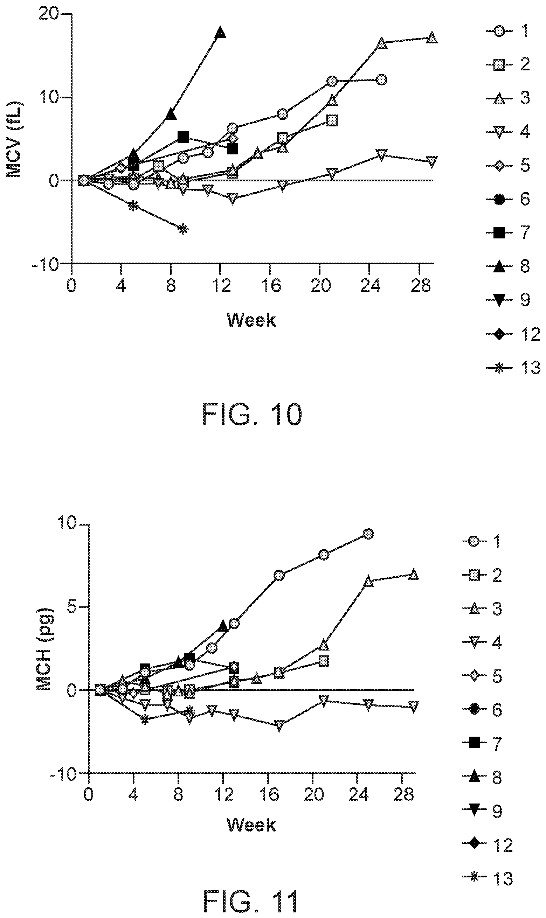

All subjects maintained hematocrit <45% after appropriate dose adjustment. Mean baseline values were serum ferritin = 14.2 ng/mL (5, 37); serum iron = 33.0 ug/dL (16.8, 107.8); and TSAT = 7.6% (4, 30). During treatment with Compound A, serum ferritin levels increased progressively toward normal (Figure 8) reflecting increase in iron stores. TSAT (Figure 9) and serum iron values varied transiently but remained below normal ranges, reflecting Compound A's pharmacodynamic effect of inhibiting iron release from intracellular stores. This was associated with increased MCV (Figure 10) and MCH (Figure 11) and decreased hematocrit and erythrocyte counts, together suggesting a normalization of iron distribution.

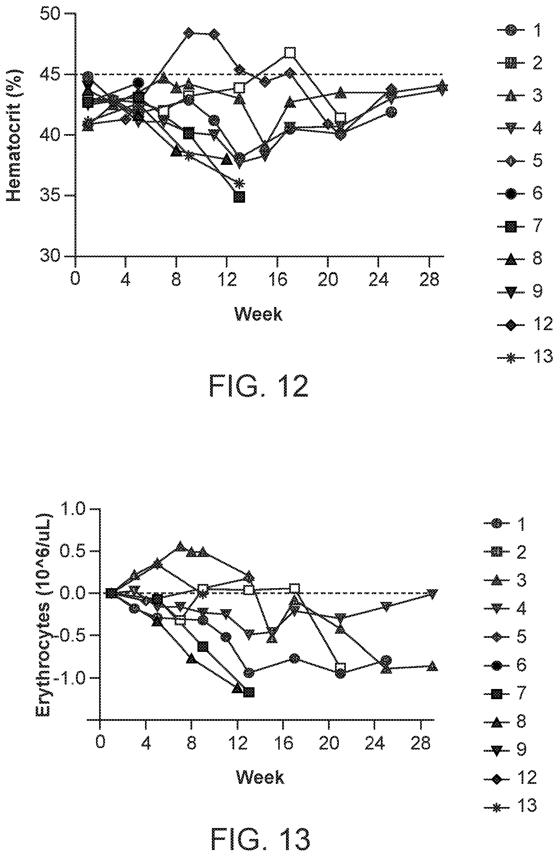

Eight of the subjects were treated for ≥3 months with Compound A (Figure 7). Three subjects were randomized. During the open label dose finding portion of the study, all subjects were phlebotomy-free, with the exception of one subject who was not treatment compliant and missed a planned treatment between weeks 4 and 9 and underwent a phlebotomy at about 13 weeks. Three subjects completed part 1 (28 weeks) with no TP as compared to 3-5 TP required in a similar period prior to study initiation. During the 28-week dose-finding period, the hematocrit was continuously controlled below 45% in all but two subjects (Figure 12). Two subjects had hematocrits transiently >45% but remained below 45% after phlebotomy in one and dose increase in both. Furthermore, erythrocyte numbers decreased (Figure 13) and MCV increased in all but two subjects. These findings suggest a redistribution of iron within erythropoiesis. Lastly, prior to treatment, mean iron-related parameters were consistent with systemic iron deficiency, while serum ferritin increased progressively toward normal range. Most frequent adverse events were injection site reaction (ISR) reported by three patients. Most of the reactions were grade 1-2 and were transient in nature and no patient discontinued the drug.

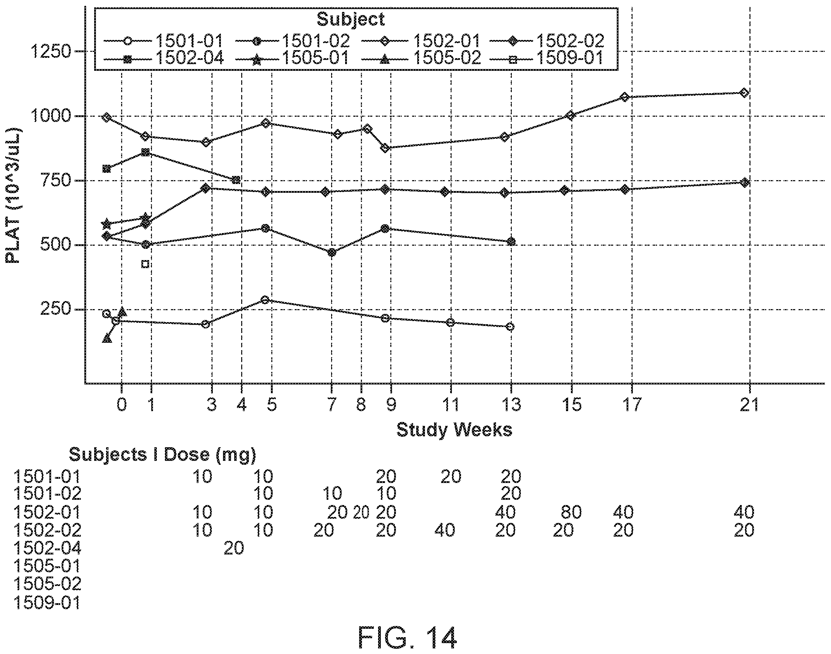

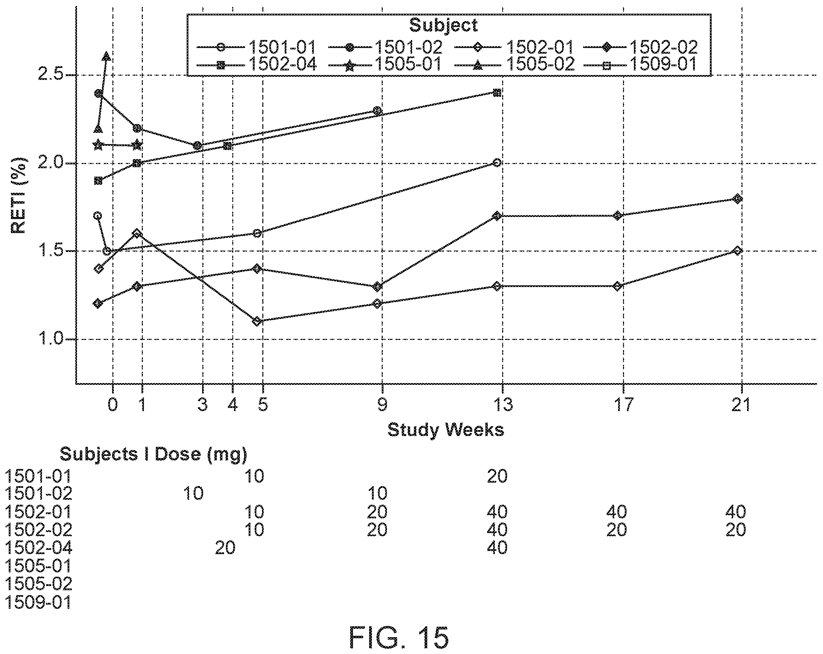

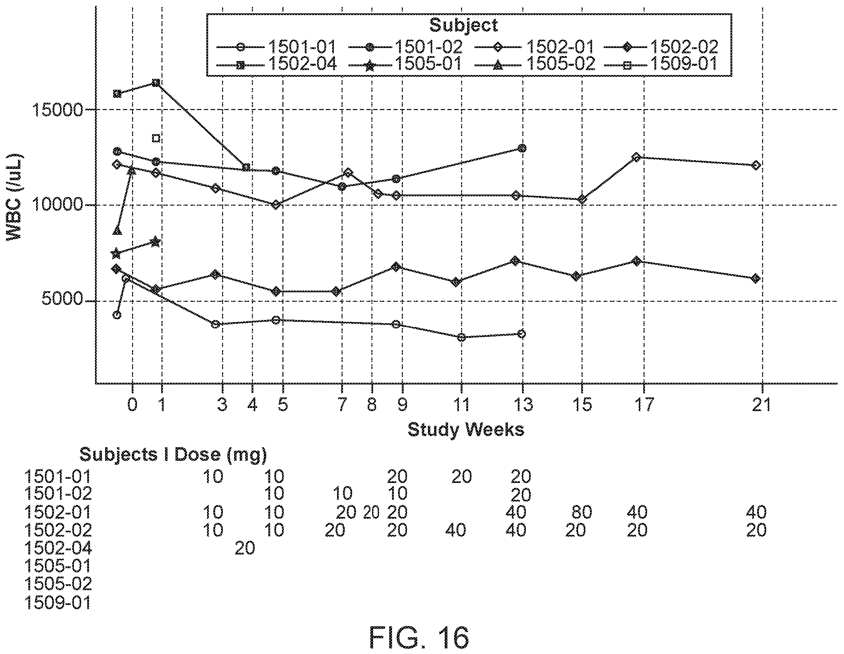

These studies demonstrate that Compound A was well tolerated. Treatment compliant subjects exhibited significantly decreased hematocrit and absolute levels below 45% and increased ferritin at the end of the evaluable treatment period relative to pre-enrollment, suggesting that symptoms of iron deficiency should be improving. The subjects' platelet counts remained generally steady over the course of treatment (Figure 14). The subjects' reticulocyte % showed an upward trend over the course of treatment (Figure 15), although a similar increase in mature red blood cells was not observed (data not shown). The subjects' leukocyte count remained generally steady over the course of treatment, suggesting that the treatment did not cause an inflammatory reaction (Figure 16). There appeared to be no PV disease progression as evidenced by no increases of platelets and white blood cells.

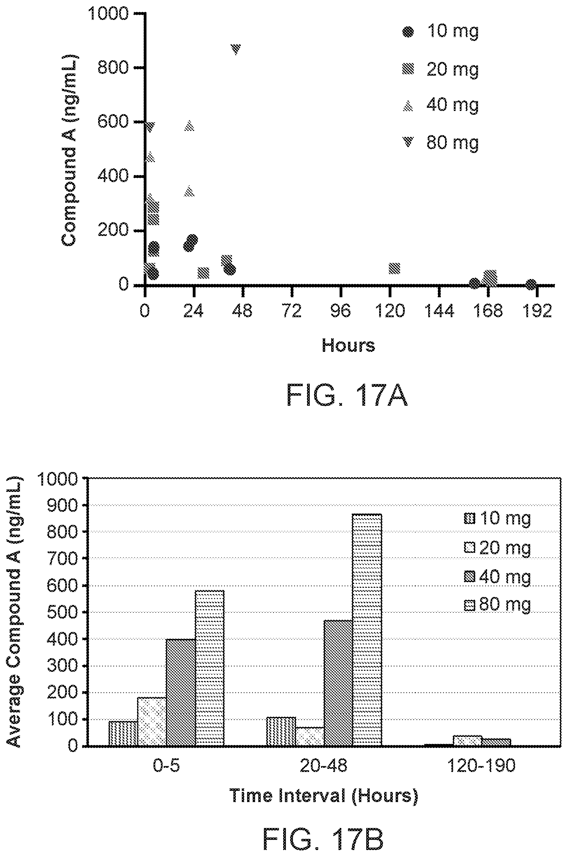

Plasma concentrations of Compound A were measured at various times after dosing of subcutaneous doses of 10 mg to 80 mg in PV patients. The concentrations increased in a dose-dependent manner and varied based on dose and time of sampling. Compound A concentrations ranged from below detectable (< 2 ng/mL) to 866 ng/mL (Figures 17A and 17B).

Conclusions: The current results support the use of hepcidin mimetics, such as Compound A, in the treatment of PV patients, including low-risk PV patients with high therapeutic phlebotomy requirements. It is hypothesized that Compound A and other hepcidin analogues promote the sequestration of iron in splenic macrophages, decreasing iron availability for malignant erythropoiesis to reduce phlebotomy requirements, while reversing iron deficiency-associated symptoms. These studies indicate that Compound A is an effective agent for the treatment of PV, reversing iron deficiency and eliminating the need for TP in PV patients. Elimination of TP requirements for 7 months in TP-dependent PV patients is significant and unexpected. The current results indicate that Compound A is an effective agent for the controlling hematocrit, reversing iron deficiency, and eliminating the therapeutic phlebotomies in both low and high-risk patients. These results also establish dosing regimens, route of administration, and methods of monitoring and adjusting dosages throughout treatment.

## Claims

1. A hepcidin analogue or a pharmaceutically acceptable salt or solvate thereof, for use in a method of treating polycythemia vera in a human subject in need thereof,
wherein the hepcidin analogue is: 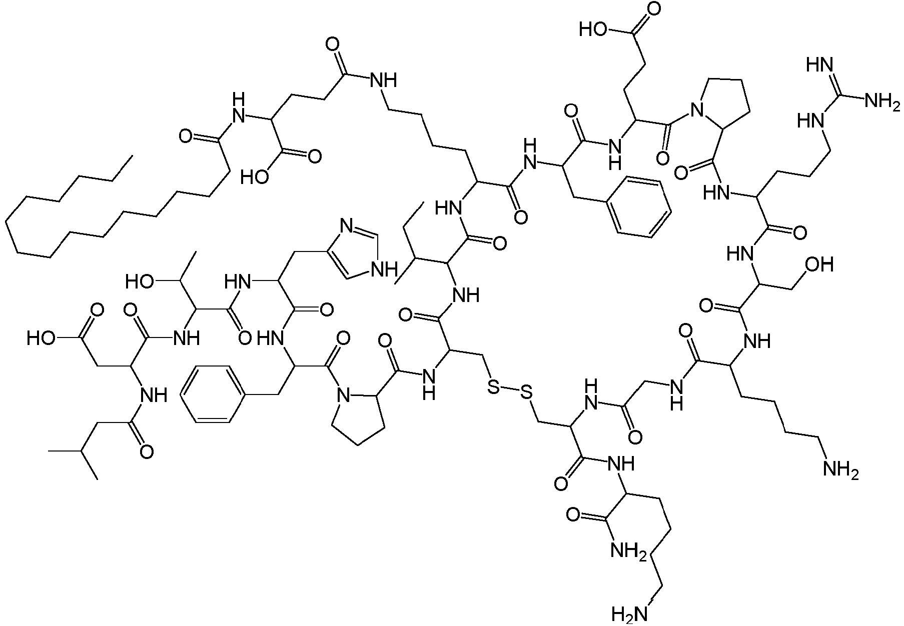 Isovaleric acid-DTHFPCI(K(isoGlu-Palm))FEPRSKGCK-NH₂ (SEQ ID NO:45),
wherein the amino acids are L-amino acids; and
wherein the hepcidin analogue or pharmaceutically acceptable salt thereof is administered via subcutaneous injection; the subject is administered from about 20 mg to about 100 mg of the hepcidin analogue or pharmaceutically acceptable salt thereof; and the hepcidin analogue or pharmaceutically acceptable salt thereof is provided to the subject twice a week, once a week, or once every two weeks.

2. The hepcidin analogue or pharmaceutically acceptable salt or solvate thereof for use of claim 1, wherein the hepcidin analogue is administered to the subject in a pharmaceutical composition comprising one or more pharmaceutically acceptable carriers, excipients, or diluents.

3. The hepcidin analogue or pharmaceutically acceptable salt or solvate thereof for use of any one of claims 1-2, wherein the hepcidin analogue or pharmaceutically acceptable salt thereof is provided to the subject at a dosage of about 20 mg to about 50 mg, about 20 mg to about 40 mg, about 80 mg, about 70 mg, about 60 mg, about 50 mg, about 40 mg, about 30 mg, about 25 mg, or about 20 mg.

4. The hepcidin analogue or pharmaceutically acceptable salt or solvate thereof for use of any one of claims 1-3, wherein the hepcidin analogue or pharmaceutically acceptable salt thereof is provided to the subject at a dosage of about 20 mg.

5. The hepcidin analogue or pharmaceutically acceptable salt or solvate thereof for use of any one of claims 1-3, wherein the hepcidin analogue or pharmaceutically acceptable salt thereof is provided to the subject at a dosage of about 40 mg.

6. The hepcidin analogue or pharmaceutically acceptable salt or solvate thereof for use of any one of claims 1-5, wherein the hepcidin analogue or pharmaceutically acceptable salt thereof is provided to the subject once a week.

7. The hepcidin analogue or pharmaceutically acceptable salt or solvate thereof for use of any one of claims 1-5, wherein the hepcidin analogue or pharmaceutically acceptable salt thereof is provided to the subject twice a week.

8. The hepcidin analogue or pharmaceutically acceptable salt or solvate thereof for use of any one of claims 1-3, wherein the hepcidin analogue is provided to the subject at a dosage of about 20 mg, about 25 mg, about 30 mg, or about 40 mg once a week

9. The hepcidin analogue or pharmaceutically acceptable salt or solvate thereof for use of any one of claims 1-3, wherein the hepcidin analogue is provided to the subject at a dosage of about 20 mg, about 25 mg, about 30 mg, or about 40 mg twice a week

10. The hepcidin analogue or pharmaceutically acceptable salt or solvate thereof for use of any one of claims 1-9, wherein the polycythemia vera is phlebotomy-requiring polycythemia vera.

11. The hepcidin analogue or pharmaceutically acceptable salt or solvate thereof for use of any one of claims 1-10, wherein the subject is diagnosed with polycythemia vera and has received at least three phlebotomies to goal hematocrit ≤45% in the 24 weeks prior to administration of the hepcidin analogue or pharmaceutically acceptable salt or solvate thereof to the subject.

12. The hepcidin analogue or pharmaceutically acceptable salt or solvate thereof for use of any one of claims 1-11, wherein the method further comprises determining the subject's hematocrit at one or more time points following administration of the hepcidin analogue or pharmaceutically acceptable salt or solvate thereof, and maintaining or adjusting the amount of the hepcidin analogue or pharmaceutically acceptable salt or solvate thereof administered to the subject, wherein the amount is increased if the subject's determined hematocrit is greater than 45%, wherein the amount is decreased if the subject's determined hematocrit is less than either 37.5% or 40%, and maintaining the amount if the subject's determined hematocrit is between 37.5% and 45% or between 40% and 44%.

13. The hepcidin analogue or pharmaceutically acceptable salt or solvate thereof for use of any one of claims 1-12, wherein the subject is treated by cytoreductive therapy, optionally wherein the cytoreductive therapy comprises hydroxyurea.

14. The hepcidin analogue or pharmaceutically acceptable salt or solvate thereof for use of any one of claims 1-13, wherein the method comprises multiple administrations of an effective amount of the hepcidin analogue or pharmaceutically acceptable salt thereof over a period of time, wherein the method further comprises determining the subject's hematocrit following one or more of the multiple administrations of the hepcidin analogue or pharmaceutically acceptable salt or solvate thereof, and maintaining or adjusting the amount of the hepcidin analogue or pharmaceutically acceptable salt or solvate thereof next administered to the subject, wherein the next administered amount is:
(i) increased if the subject's determined hematocrit is above an acceptable range based on the subject's sex and pregnancy status,
(ii) decreased if the subject's determined hematocrit is below the acceptable range, or
(iii) the same as the previously administered amount if the subject's determined hematocrit is within the acceptable range.

## Patentansprüche

1. Hepcidin-Analogon oder ein pharmazeutisch verträgliches Salz oder Solvat davon zur Verwendung in einem Verfahren zur Behandlung von Polycythaemia vera bei einem Menschen, der dessen bedarf,
wobei Hepcidin-Analogon Folgendes ist: 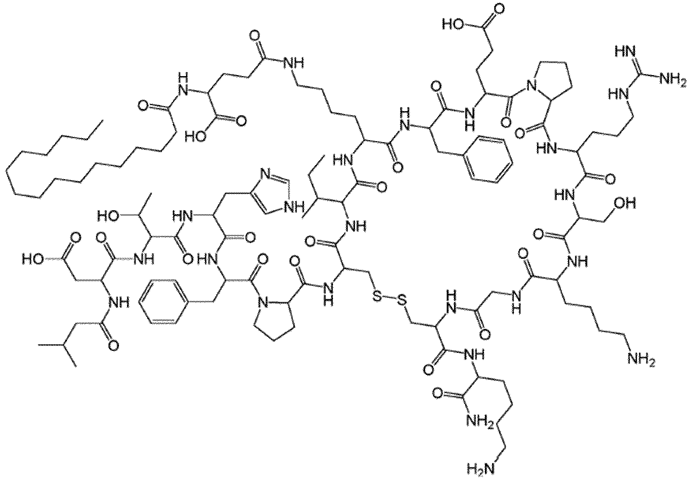 Isovaleriansäure-DTHFPCI(K(isoGlu-Palm))FEPRSKGCK-NH₂(SEQ ID NO:45),
wobei Aminosäuren L-Aminösäurén sind; und
wobei das Hepcidin-Analogon oder pharmazeutisch verträgliche Salz davon durch subkutane Injektion verabreicht wird; der Person etwa 20 mg bis etwa 100 mg des Hepcidin-Analogon oder pharmazeutisch verträglichen Salzes davon verabreicht wird; und das Hepcidin-Analogon oder pharmazeutisch verträgliche Salz davon der Person zweimal pro Woche, einmal pro Woche oder einmal alle zwei Wochen verabreicht wird.

2. Hepcidin-Analogon oder pharmazeutisch verträgliches Salz oder Solvat davon zur Verwendung nach Anspruch 1, wobei das Hepcidin-Analogon der Person in einer pharmazeutischen Zusammensetzung verabreicht wird, die einen oder mehrere pharmazeutisch verträgliche Träger, Hilfsstoffe oder Verdünnungsmittel umfasst.

3. Hepcidin-Analogon oder pharmazeutisch verträgliches Salz oder Solvat davon zur Verwendung nach einem der Ansprüche 1-2, wobei das Hepcidin-Analogon oder pharmazeutisch verträgliche Salz davon der Person in einer Dosierung von etwa 20 mg bis etwa 50 mg, etwa 20 mg bis etwa 40 mg, etwa 80 mg, etwa 70 mg, etwa 60 mg, etwa 50 mg, etwa 40 mg, etwa 30 mg, etwa 25 mg oder etwa 20 mg bereitgestellt wird.

4. Hepcidin-Analogon oder pharmazeutisch verträgliches Salz oder Solvat davon zur Verwendung nach einem der Ansprüche 1-3, wobei das Hepcidin-Analogon oder pharmazeutisch verträgliche Salz davon der Person in einer Dosierung von etwa 20 mg bereitgestellt wird.

5. Hepcidin-Analogon oder pharmazeutisch verträgliches Salz oder Solvat davon zur Verwendung nach einem der Ansprüche 1-3, wobei das Hepcidin-Analogon oder pharmazeutisch verträgliche Salz davon der Person in einer Dosierung von etwa 40 mg bereitgestellt wird.

6. Hepcidin-Analogon oder pharmazeutisch verträgliches Salz oder Solvat davon zur Verwendung nach einem der Ansprüche 1-5, wobei das Hepcidin-Analogon oder pharmazeutisch verträgliche Salz davon der Person einmal pro Woche bereitgestellt wird.

7. Hepcidin-Analogon oder pharmazeutisch verträgliches Salz oder Solvat davon zur Verwendung nach einem der Ansprüche 1-5, wobei das Hepcidin-Analogon oder pharmazeutisch verträgliche Salz davon der Person zweimal pro Woche bereitgestellt wird.

8. Hepcidin-Analogon oder pharmazeutisch verträgliches Salz oder Solvat davon zur Verwendung nach einem der Ansprüche 1-3, wobei das Hepcidin-Analogon oder pharmazeutisch verträgliche Salz davon der Person in einer Dosierung von etwa 20 mg, etwa 25 mg, etwa 30 mg oder etwa 40 mg einmal wöchentlich bereitgestellt wird.

9. Hepcidin-Analogon oder pharmazeutisch verträgliches Salz oder Solvat davon zur Verwendung nach einem der Ansprüche 1-3, wobei das Hepcidin-Analogon oder pharmazeutisch verträgliche Salz davon der Person in einer Dosierung von etwa 20 mg, etwa 25 mg, etwa 30 mg oder etwa 40 mg zweimal wöchentlich bereitgestellt wird.

10. Hepcidin-Analogon oder pharmazeutisch verträgliches Salz oder Solvat davon zur Verwendung nach einem der Ansprüche 1-9, wobei die Polyzythaemia vera eine Phlebotomie erfordernde Polyzythaemia vera ist.

11. Hepcidin-Analogon oder pharmazeutisch verträgliches Salz oder Solvat davon zur Verwendung nach einem der Ansprüche 1-10, wobei bei der Person Polycythaemia vera diagnostiziert ist und sie in den 24 Wochen vor der Verabreichung von dem Hepcidin-Analogon oder pharmazeutisch verträglichen Salz oder Solvat davon mindestens drei Phlebotomien erhalten hat, um einen Hämatokritwert von ≤ 45 % zu erreichen.

12. Hepcidin-Analogon oder pharmazeutisch verträgliches Salz oder Solvat davon zur Verwendung nach einem der Ansprüche 1-11, wobei das Verfahren ferner das Bestimmen des Hämatokritwerts der Person zu einem oder mehreren Zeitpunkten nach der Verabreichung von Hepcidin-Analogon oder pharmazeutisch verträglichem Salz oder Solvat davon umfasst und die Menge von dem Hepcidin-Analogon oder pharmazeutisch verträglichen Salz oder Solvat davon aufrechterhalten oder beibehalten wird, und Beibehalten oder Anpassen der Menge des der Person verabreichten Hepcidin-Analogon oder pharmazeutisch verträglichen Salzes oder Solvats davon, wobei die Menge erhöht wird, wenn der ermittelte Hämatokritwert der Person höher als 45 % ist, wobei die Menge verringert wird, wenn der ermittelte Hämatokritwert der Person niedriger als entweder 37,5 % oder 40 % ist und die Menge beibehalten wird, wenn der ermittelte Hämatokritwert der Person zwischen 37,5 % und 45 % oder zwischen 40 % und 44 % liegt.

13. Hepcidin-Analogon oder pharmazeutisch verträgliches Salz oder Solvat davon zur Verwendung nach einem der Ansprüche 1-12, wobei die Person durch eine zytoreduktive Therapie behandelt wird, wobei die zytoreduktive Therapie gegebenenfalls Hydroxyharnstoff umfasst.

14. Hepcidin-Analogon oder pharmazeutisch verträgliches Salz oder Solvat davon zur Verwendung nach einem der Ansprüche 1-13, wobei das Verfahren mehrere Verabreichungen einer wirksamen Menge von dem Hepcidin-Analogon oder pharmazeutisch verträglichen Salz davon über einen Zeitraum umfasst, wobei das Verfahren ferner das Bestimmen des Hämatokritwerts der Person nach einer oder mehreren der mehrfachen Verabreichungen von dem Hepcidin-Analogon oder pharmazeutisch verträglichen Salz oder Solvat davon umfasst und das Beinehalten oder Anpassen der Menge des Hepcidin-Analogon oder pharmazeutisch verträglichen Salzes oder Solvats davon, die der Person als nächstes verabreicht wird, umfasst, wobei die nächste verabreichte Menge Fogendes ist:
(i) erhöht, wenn der ermittelte Hämatokritwert der Person über einem akzeptablen Bereich liegt, der auf dem Geschlecht und dem Schwangerschaftsstatus der Person basiert,
(ii) verringert, wenn der ermittelte Hämatokritwert der Person unter dem akzeptablen Bereich liegt, oder
(iii) die gleiche Menge wie die zuvor verabreichte Menge, wenn der ermittelte Hämatokritwert der Person innerhalb des zulässigen Bereichs liegt.

## Revendications

1. Analogue d'hepcidine ou sel ou solvate pharmaceutiquement acceptable de celui-ci, pour une utilisation dans un procédé de traitement de la polycythémie vera chez un sujet humain en ayant besoin,
dans lequel l'analogue d'hepcidine est : 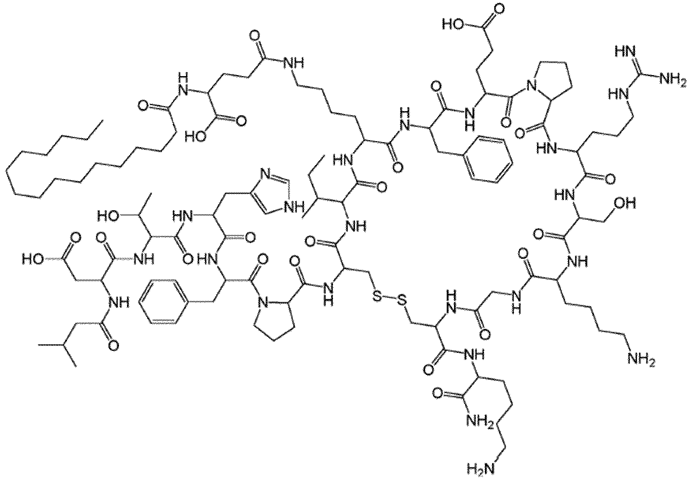
Acide isovalérique-DTHFPCI(K(isoGlu-Palm))FEPRSKGCK-NH₂(SEQ ID NO : 45),
dans lequel les acides aminés sont des acides aminés L ; et
dans lequel l'analogue d'hepcidine ou le sel pharmaceutiquement acceptable de celui-ci est administré par injection sous-cutanée ; le sujet reçoit environ 20 mg à environ 100 mg de l'analogue d'hepcidine ou du sel pharmaceutiquement acceptable de celui-ci ; et l'analogue d'hepcidine ou le sel pharmaceutiquement acceptable de celui-ci est fourni au sujet deux fois par semaine, une fois par semaine, ou une fois toutes les deux semaines.

2. Analogue d'hepcidine ou sel ou solvate pharmaceutiquement acceptable de celui-ci pour une utilisation selon la revendication 1, dans lequel l'analogue d'hepcidine est administré au sujet dans une composition pharmaceutique comprenant un ou plusieurs supports, excipients, ou diluants pharmaceutiquement acceptables.

3. Analogue d'hepcidine ou sel ou solvate pharmaceutiquement acceptable de celui-ci pour une utilisation selon l'une quelconque des revendications 1 et 2, dans lequel l'analogue d'hepcidine ou le sel pharmaceutiquement acceptable de celui-ci est fourni au sujet à une dose d'environ 20 mg à environ 50 mg, d'environ 20 mg à environ 40 mg, d'environ 80 mg, d'environ 70 mg, d'environ 60 mg, d'environ 50 mg, d'environ 40 mg, d'environ 30 mg, d'environ 25 mg, ou d'environ 20 mg.

4. Analogue d'hepcidine ou sel ou solvate pharmaceutiquement acceptable de celui-ci pour une utilisation selon l'une quelconque des revendications 1 à 3, dans lequel l'analogue d'hepcidine ou le sel pharmaceutiquement acceptable de celui-ci est fourni au sujet à une dose d'environ 20 mg.

5. Analogue d'hepcidine ou sel ou solvate pharmaceutiquement acceptable de celui-ci pour une utilisation selon l'une quelconque des revendications 1 à 3, dans lequel l'analogue d'hepcidine ou le sel pharmaceutiquement acceptable de celui-ci est fourni au sujet à une dose d'environ 40 mg.

6. Analogue d'hepcidine ou sel ou solvate pharmaceutiquement acceptable de celui-ci pour une utilisation selon l'une quelconque des revendications 1 à 5, dans lequel l'analogue d'hepcidine ou le sel pharmaceutiquement acceptable de celui-ci est fourni au sujet une fois par semaine.

7. Analogue d'hepcidine ou sel ou solvate pharmaceutiquement acceptable de celui-ci pour une utilisation selon l'une quelconque des revendications 1 à 5, dans lequel l'analogue d'hepcidine ou le sel pharmaceutiquement acceptable de celui-ci est fourni au sujet deux fois par semaine.

8. Analogue d'hepcidine ou sel ou solvate pharmaceutiquement acceptable de celui-ci pour une utilisation selon l'une quelconque des revendications 1 à 3, dans lequel l'analogue d'hepcidine est fourni au sujet à une dose d'environ 20 mg, d'environ 25 mg, d'environ 30 mg, ou d'environ 40 mg une fois par semaine.

9. Analogue d'hepcidine ou sel ou solvate pharmaceutiquement acceptable de celui-ci pour une utilisation selon l'une quelconque des revendications 1 à 3, dans lequel l'analogue d'hepcidine est fourni au sujet à une dose d'environ 20 mg, d'environ 25 mg, d'environ 30 mg, ou d'environ 40 mg deux fois par semaine.

10. Analogue d'hepcidine ou sel ou solvate pharmaceutiquement acceptable de celui-ci pour une utilisation selon l'une quelconque des revendications 1 à 9, dans lequel la polycythémie vera est une polycythémie vera nécessitant une phlébotomie.

11. Analogue d'hepcidine ou sel ou solvate pharmaceutiquement acceptable de celui-ci pour une utilisation selon l'une quelconque des revendications 1 à 10, dans lequel le sujet est diagnostiqué avec une polycythémie vera et a reçu au moins trois phlébotomies pour atteindre un hématocrite cible ≤ 45 % au cours des 24 semaines précédant l'administration de l'analogue d'hepcidine ou du sel ou solvate pharmaceutiquement acceptable de celui-ci au sujet.

12. Analogue d'hepcidine ou sel ou solvate pharmaceutiquement acceptable de celui-ci pour une utilisation selon l'une quelconque des revendications 1 à 11, dans lequel le procédé comprend également la détermination de l'hématocrite du sujet à un ou plusieurs moments suivant l'administration de l'analogue d'hepcidine ou du sel ou solvate pharmaceutiquement acceptable de celui-ci, et le maintien ou l'ajustement de la quantité de l'analogue d'hepcidine ou du sel ou solvate pharmaceutiquement acceptable de celui-ci administrée au sujet, dans lequel la quantité est augmentée si l'hématocrite déterminé du sujet est supérieur à 45 %, dans lequel la quantité est diminuée si l'hématocrite déterminé du sujet est inférieur à 37,5 % ou 40 %, et le maintien de la quantité si l'hématocrite déterminé du sujet est compris entre 37,5 % et 45 % ou entre 40 % et 44 %.

13. Analogue d'hepcidine ou sel ou solvate pharmaceutiquement acceptable de celui-ci pour une utilisation selon l'une quelconque des revendications 1 à 12, dans lequel le sujet est traité par thérapie cytoréductrice, éventuellement dans lequel la thérapie cytoréductrice comprend de l'hydroxyurée.

14. Analogue d'hepcidine ou sel ou solvate pharmaceutiquement acceptable de celui-ci pour une utilisation selon l'une quelconque des revendications 1 à 13, dans lequel le procédé comprend de multiples administrations d'une quantité efficace de l'analogue d'hepcidine ou du sel pharmaceutiquement acceptable de celui-ci sur une période de temps, dans lequel le procédé comprend également la détermination de l'hématocrite du sujet après l'une ou plusieurs des multiples administrations de l'analogue d'hepcidine ou du sel ou solvate pharmaceutiquement acceptable de celui-ci, et le maintien ou l'ajustement de la quantité d'analogue d'hepcidine ou du sel ou solvate pharmaceutiquement acceptable de celui-ci administrée ensuite au sujet, dans lequel la quantité administrée ensuite est :
(i) augmentée si l'hématocrite déterminé du sujet est supérieur à une plage acceptable sur la base du sexe du sujet et de son état de grossesse,
(ii) diminuée si l'hématocrite déterminé du sujet est inférieur à la plage acceptable, ou
(iii) la même que la quantité administrée précédemment si l'hématocrite déterminé du sujet se situe dans la plage acceptable.
